# EUROPEAN PATENT APPLICATION

(11) **EP 0 770 681 A2**
(43) Date of publication of application: **02.05.1997**
(21) Application number: 96202492.3
(22) Date of filing: 06.04.1993
(51) Int. Cl.: C12N 15/29, C07K 14/415, A61K 39/36, G01N 33/68

(54) **T cell epitopes of the major allergens from ambrosia artemisiifolia**

(30) Priority: 09.04.1992 US 866679
(62) Divisional of application: 93911584.6
(71) Applicant: IMMULOGIC PHARMACEUTICAL CORPORATION, Waltham, Massachusetts 02154 (US)
(72) Inventor: Kuo, Mei-Chang, Palo Alto, CA 94304 (US); Garman, Richard D., Arlington, MA 02174 (US); Greenstein, Julia L., West Newton, MA 02165 (US)
(74) Representative: Bizley, Richard Edward

(57) **Abstract**

The present invention provides isolated peptides of the major protein allergens of Ambrosia artemisiifolia, or short ragweed pollen. Peptides within the scope of the invention comprise at least one T cell epitope, or preferably at least two T cell epitopes of a protein allergen selected from the allergens Amb a I.1, Amb a I.2, Amb a I.3, Amb a I.4 and Amb a II. Modified peptides having similar or enhanced therapeutic properties as the corresponding, naturally-occurring allergen or portion thereof, but having reduced side effects are disclosed. The invention also provides nucleic acids having sequences encoding peptides of the invention. Methods of treatment or of diagnosis of sensitivity to ragweed pollen allergens in an individual and therapeutic compositions comprising one or more peptides of the invention are also provided.

## Description

### Background of the Invention

Ambrosia artemisiifolia or short ragweed pollen is the major cause of late summer hayfever in North America and Canada and considerable effort has been expended in trying to identify the major allergens produced by this species. Amb a I or Antigen E (AgE) has been reported to be the predominant allergen. (King, T.P., et al. Biochemistry, 3:458 (1964)). AgE has been characterized and reported to be a nonglycosylated protein of 38kD molecular mass (King, T.P., Adv. Immunol. 23:77 (1976); King, T.P., et al., Arch. Biochem. Biophys., 212:127 (1981)). An immunochemically related protein, Amb a II (AgK), has been reported to have similar properties (King, T.P., Adv. Immunol. 23:77 (1976); King, T.P., Biochemistry, 11:367 (1972)). Amb a I or AgE can be purified using conventional chromatographic or biochemical techniques. However, it has been reported that due to cleavage of the 38 kD single-chain precursors by the action of a trypsin-like pollen protease, purification often results in the isolation of two noncovalently associated chains of 26 and 12 kD molecular mass, designated α and β, respectively (King, T.P., et al., Arch. Biochem. Biophys., 212:127 (1981); King, T.P., et al., Immunochemistry, 11:83 (1974)). It has been reported that biochemically purified Amb a I has been used as an immunogen to produce murine monoclonal antibodies (mAb) as well as rabbit polyclonal antisera reactive with both the native (Olson, J.R., and D.G. Klapper, J. Immunol., 136:2109 (1986)) and denatured (Smith, et al., Molec. Immunol., 25:355 (1988)) protein.

Early studies using skin tests with Amb a I-depleted pollen extract led to the estimation that at least 90% of the allergenic activity in ragweed pollen can be attributed to Amb a I (King, T.P., et al., Biochemistry, 3:458 (1964); King, T.P., et al., Immunol., 23:77 (1976)). Competition experiments using murine mAb to inhibit the binding of Amb a I by human IgE in ELISA assays have confirmed that Amb a I binds a substantial proportion of human ragweed allergic IgE (Olson, J.R., et al., J. Immunol., 136:2109 (1986)). Recently, three cDNAs encoding proteins with properties of Amb a I were cloned and Amb a I was reported to be a family of homologous, but distinguishable sequences (Rafnar, T., et al., J. Biol. Chem., 266:1229 (1991)). Rafnar and co-workers reported that the individual cloned members of the Amb a I family, designated Amb a I.1, Amb a I.2, and Amb a I.3, share amino acid sequence homology exceeding 80%. The fourth family member designated Amb a I.D (Amb a I.4) is disclosed in U.S.S.N. 07/529,951, filed May 29, 1990. The deduced amino acid sequence of Amb a II has been disclosed and reported to share approximately 65% sequence identity with the Amb a I multigene family of allergens. (Rogers, B. L., et al., Journal of Immunology 147:2547-2552 (1992)).

### Summary of the Invention

The present invention provides isolated peptides of the major protein allergens of Ambrosia artemisiifolia including peptides derived from the family of related proteins, previously designated Amb a IA, Amb a IB, Amb a IC, and Amb a ID. These allergens have been renamed according to the IUIS approved nomenclature as Amb a I.1 (Amb a IA), Amb a I.2 (Amb a IB), Amb a I.3 (Amb a IC) and Amb a I.4 (Amb a ID). Peptides within the scope of the invention comprise at least one T cell epitope, preferably at least two T cell epitopes, of a protein allergen selected from the family of Amb a I allergens and Amb a II. The invention further provides peptides comprising at least two regions, each region comprising at least one T cell epitope of a ragweed pollen allergen. The regions are derived from the same or from different ragweed pollen allergens.

The invention also provides modified peptides having similar or enhanced therapeutic properties as the corresponding, naturally-occurring allergen or portion thereof, but having reduced side effects as well as modified peptides having improved properties such as increased solubility and stability. Peptides of the invention are capable of modifying, in a ragweed pollen-sensitive individual to whom they are administered, the allergic response of the individual to a ragweed pollen allergen. Methods of treatment or of diagnosis of sensitivity to a ragweed pollen allergen in an individual and therapeutic compositions comprising one or more peptides of the invention are also provided.

### Brief Description of the Drawings

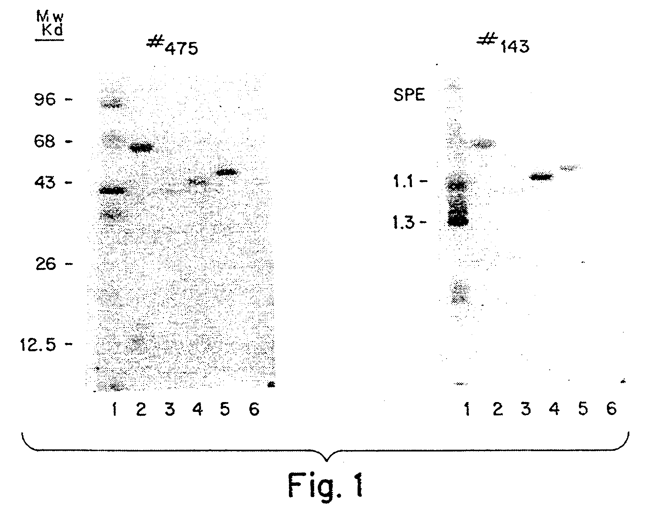

Fig. 1 shows Western blot analysis of IgE binding to recombinant Amb a I proteins.

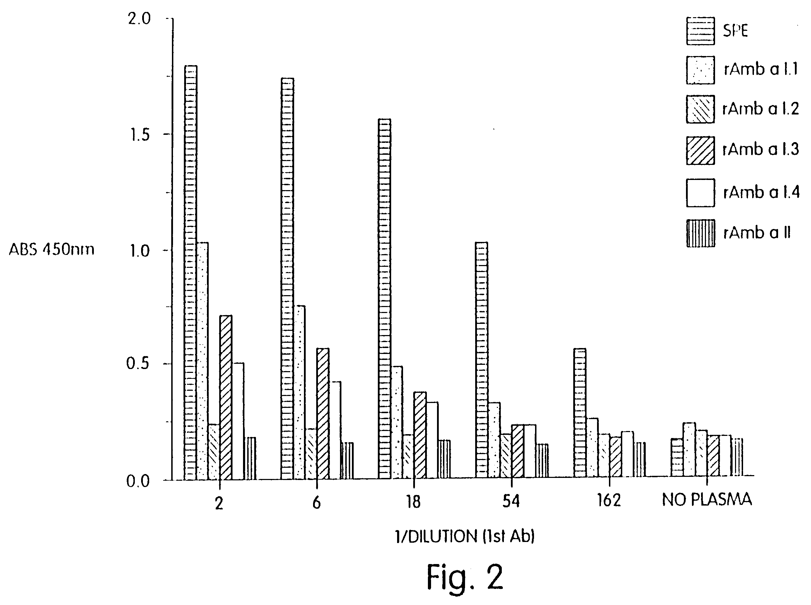

Fig. 2 is a graphic representation of a direct binding assay of IgE from a single ragweed allergic patient to recombinant Amb a I and Amb a II proteins.

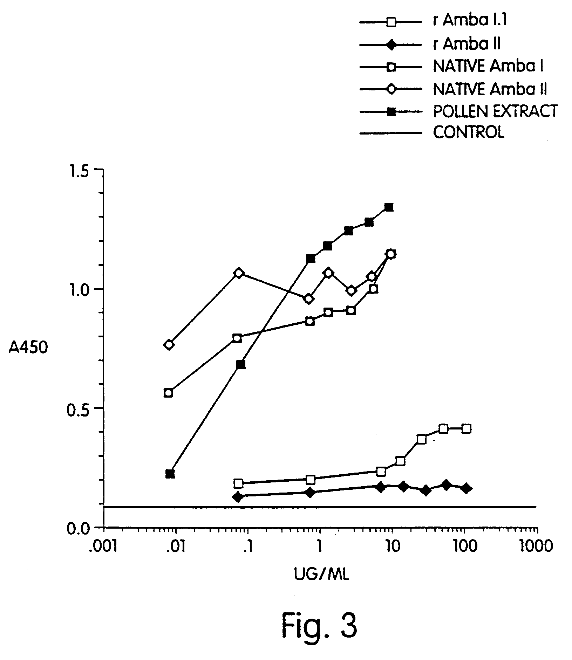

Fig. 3 is a graphic representation of the results of a direct binding assay of IgE from pooled human sera to native Amb a I, Amb a II, recombinant Amb a I.1, recombinant Amb a II and pollen extract.

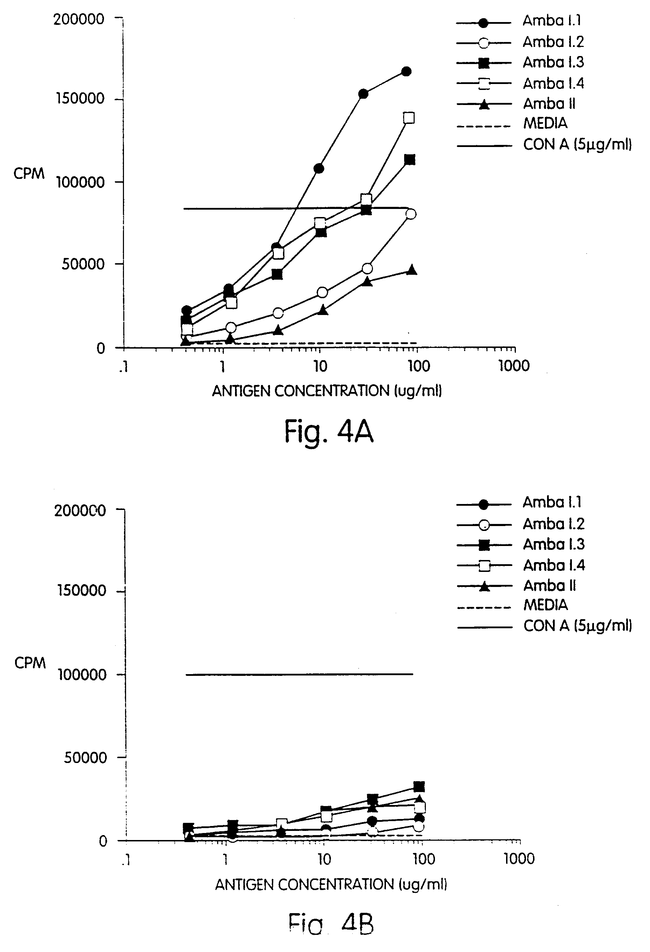

Fig. 4A and 4B are graphic representations depicting the responses of lymph node cells isolated from mice tolerized in vivo with either Amb a I.1 or PBS and CFA and challenged in vitro with various antigens.

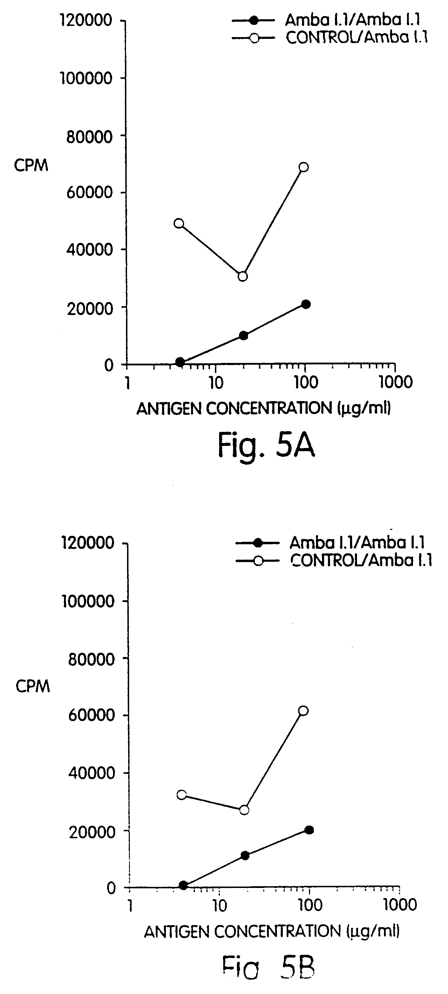

Fig. 5A-5F are graphic representations depicting the responses of lymph node cells isolated from mice tolerized in vivo with Amb a I.1 or pollen extract, challenged with Amb a I.1, and tested with various antigens.

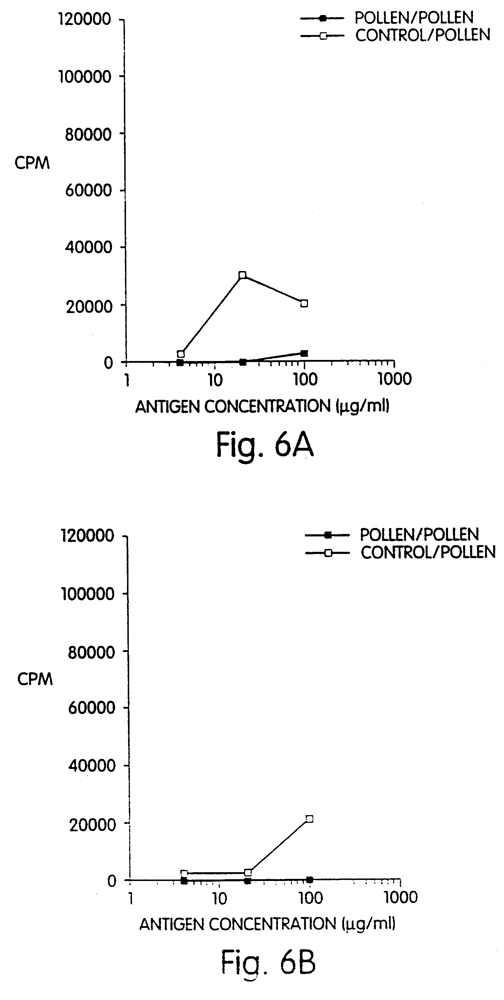

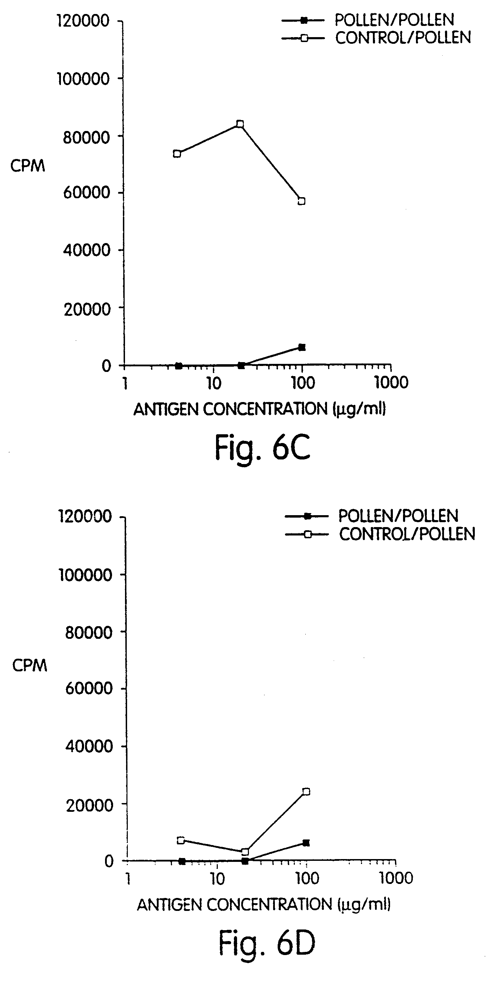

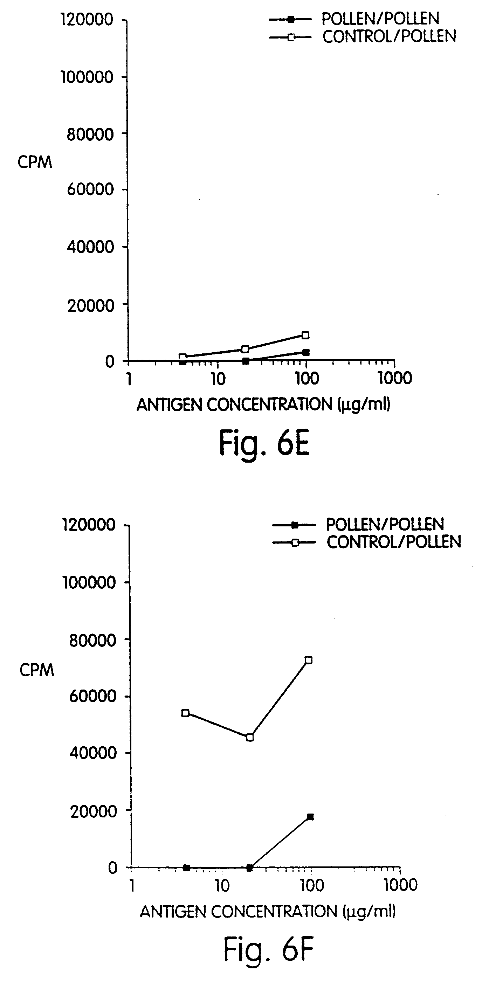

Fig. 6A-6F are graphic representations depicting the responses of lymph node cells isolated from mice tolerized with pollen extract challenged with pollen extract, and tested with various antigens.

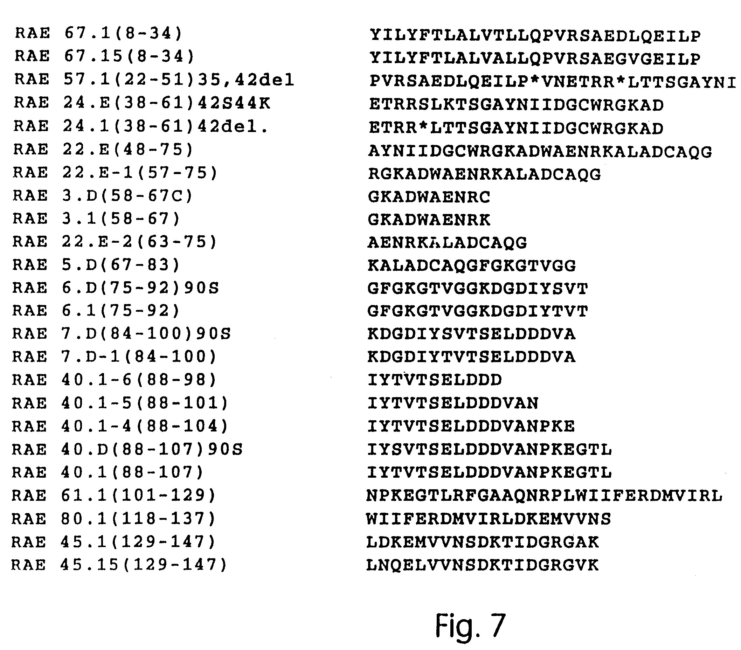

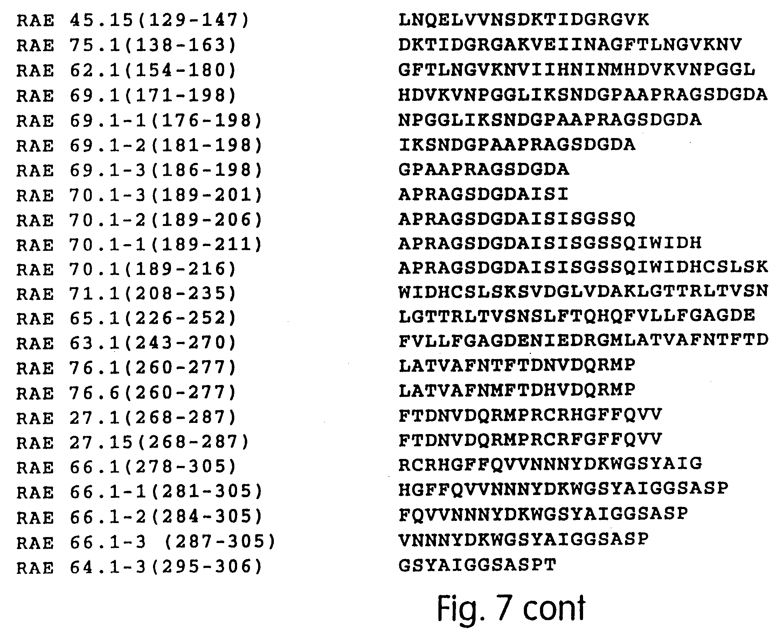

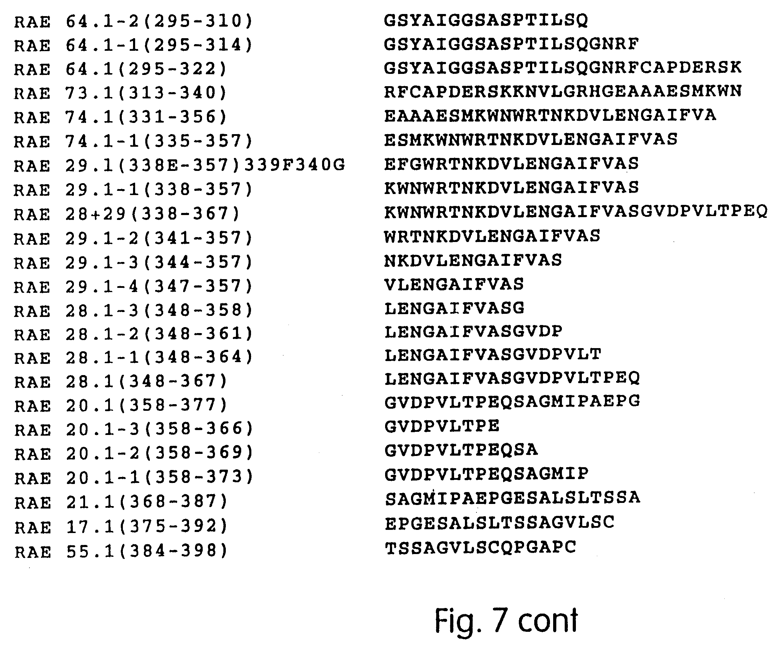

Fig. 7 shows various peptides of desired length derived from the Amb a I.1, Amb a I.2 and Amb a I.3 protein allergens.

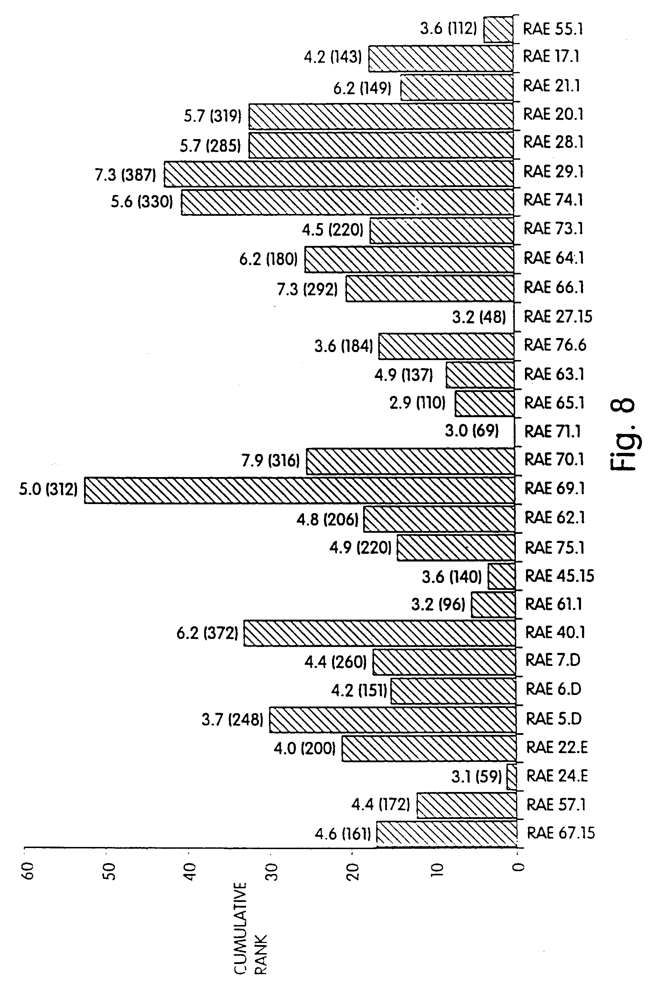

Fig. 8 is a graphic representation depicting the responses of T cell lines from 39 patients primed in vitro to recombinant Amb a I.1 protein and analyzed for response to various overlapping Amb a I.1 peptides and selected Amb a 1.2 and Amb a I.3 peptides by percent of positive responses within the individuals tested, the mean stimulation index of positive responses for the peptide and the ranked sum of peptide responses.

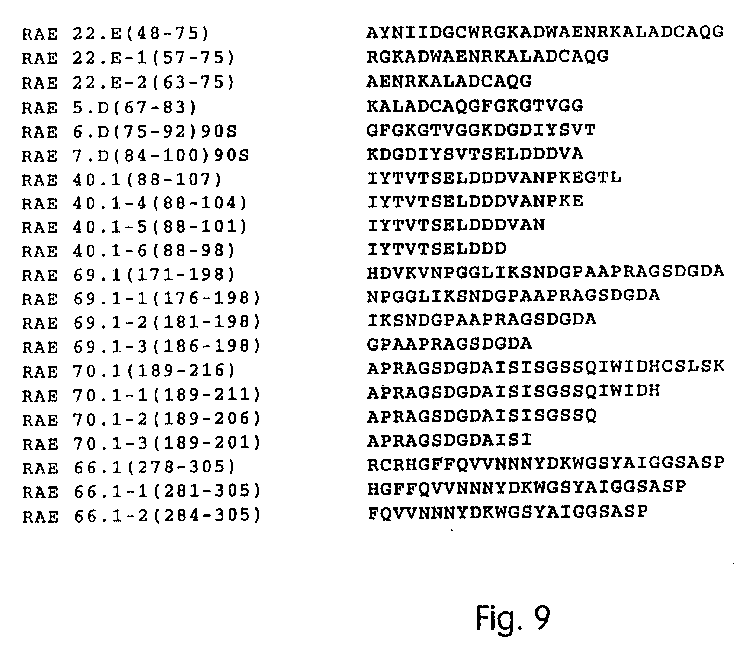

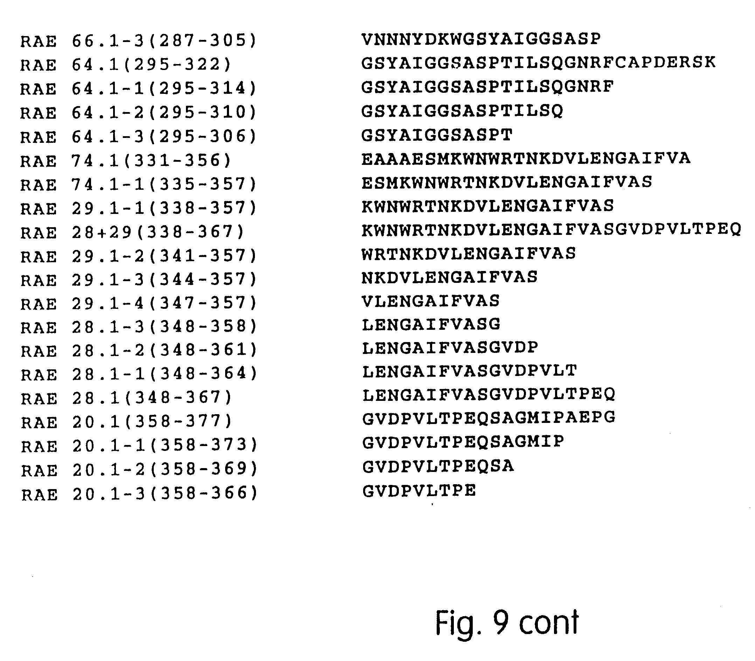

Fig. 9 shows selected peptides of desired lengths derived from the Amb a I.1 protein allergen.

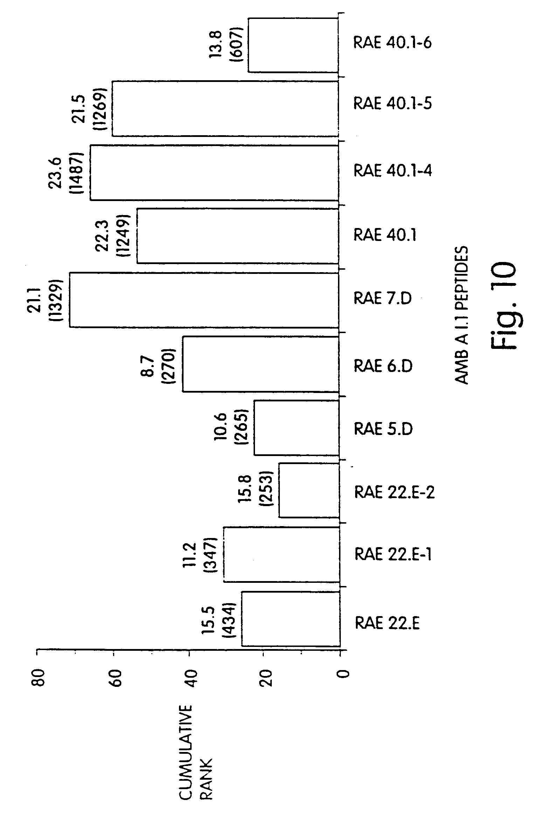

Fig. 10 is a graphic representation depicting the responses of T cell lines from 48 patients primed in vitro to recombinant Amb a I.1 protein and analyzed for response to selected peptides derived from Region 1 of the Amb a I.1 protein, by percent of positive responses within the individuals tested, the mean stimulation index of positive responses for the peptide and the ranked sum of peptide responses.

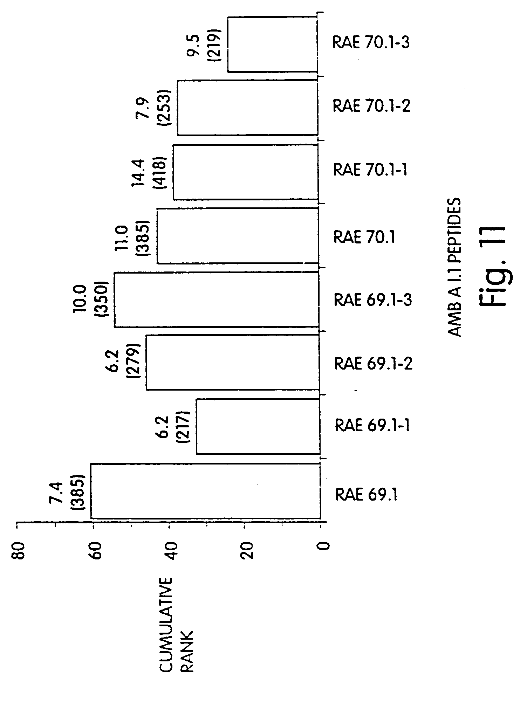

Fig. 11 is a graphic representation depicting the responses of T cell lines from 48 patients primed in vitro to recombinant Amb a I.1 protein and analyzed for response to selected peptides derived from Region 2 of the Amb a I.1 protein, by percent of positive responses within the individuals tested, the mean stimulation index of positive responses for the peptide and the ranked sum of peptide responses.

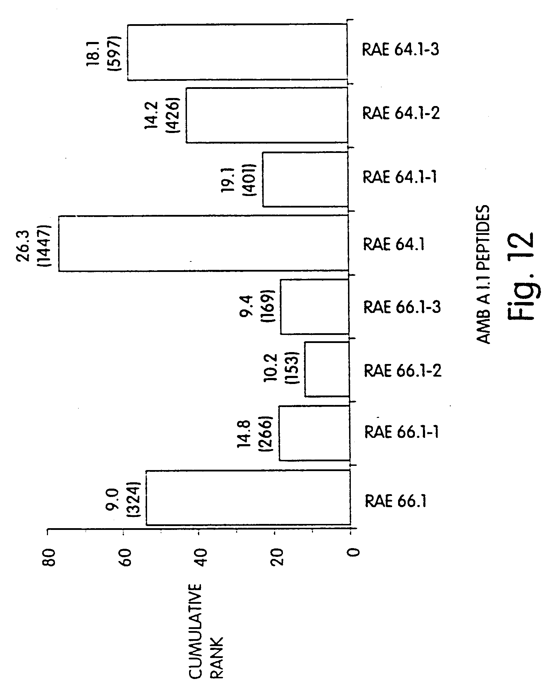

Fig. 12 is a graphic representation depicting the responses of T cell lines from 48 patients primed in vitro to recombinant Amb a I.1 protein and analyzed for response to selected peptides derived from Region 3 of the Amb a I.1 protein, by percent of positive responses within the individuals tested, the mean stimulation index of positive responses for the peptide and the ranked sum of peptide responses.

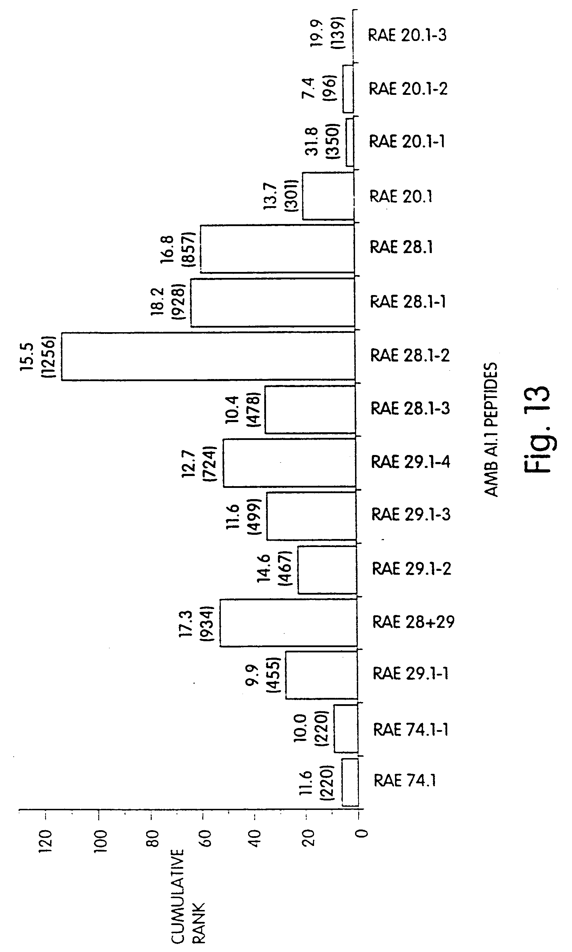

Fig. 13 is a graphic representation depicting the responses of T cell lines from 48 patients primed in vitro to recombinant Amb a I.1 protein and analyzed for response to selected peptides derived from Region 4 of the Amb a I.1 protein, by percent of positive responses within the individuals tested, the mean stimulation index of positive responses for the peptide and the ranked sum of peptide responses.

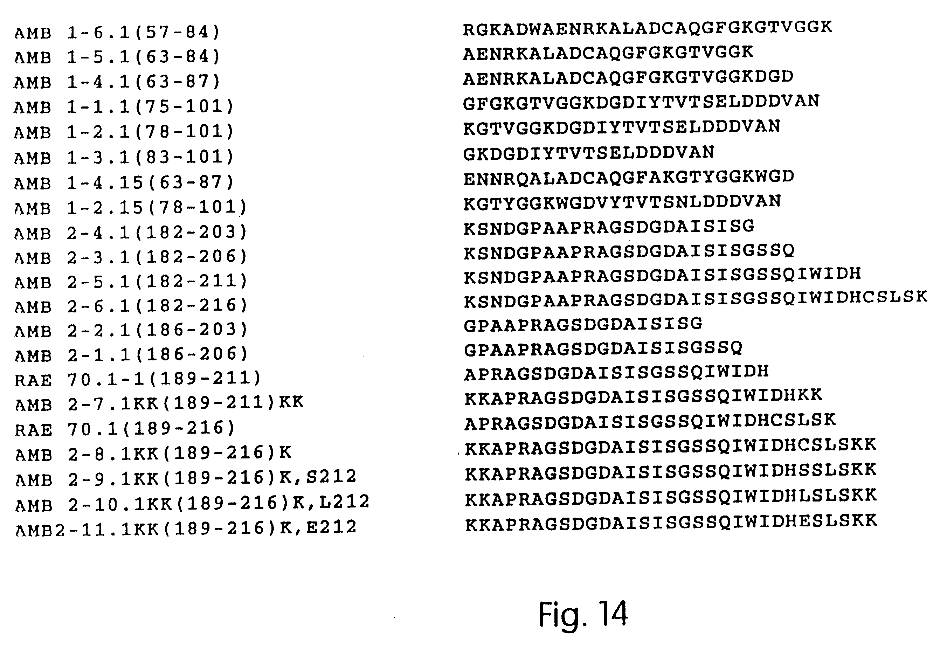

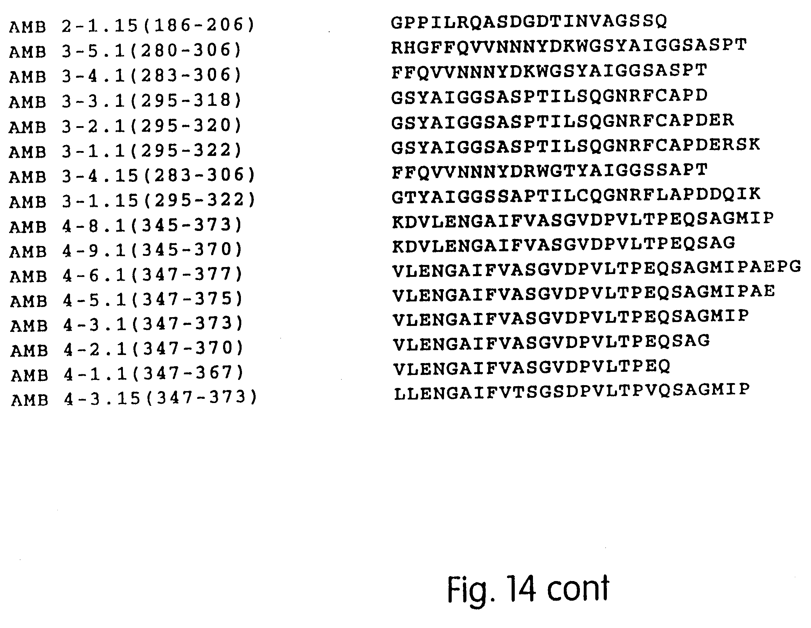

Fig. 14 shows selected peptides of desired lengths derived from the Amb a I.1 protein allergen and the Amb a I.3 protein allergen.

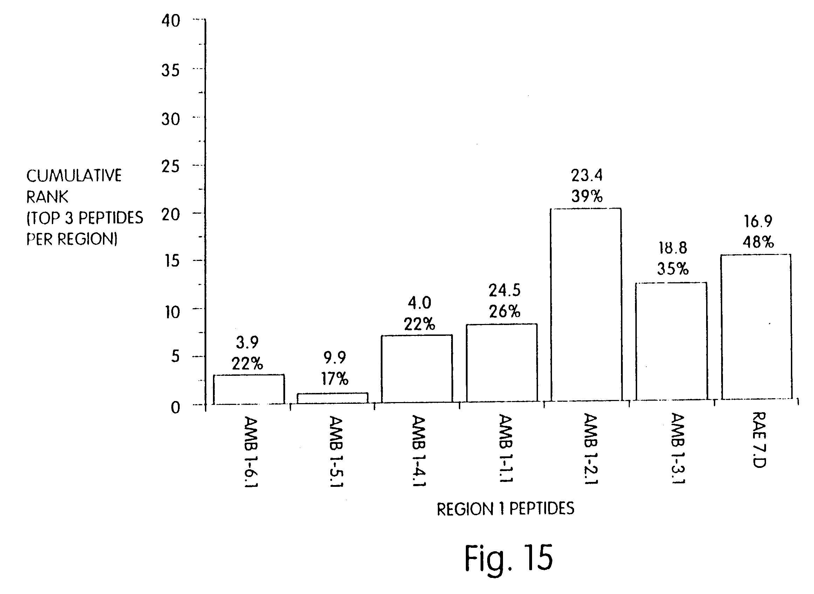

Fig. 15 is a graphic representation depicting the responses of T cell lines from 23 patients primed in vitro to recombinant Amb a I.1 protein and analyzed for response to selected peptides derived from Region 1 of the Amb a I.1 protein, by percent of positive responses within the individuals tested, the mean stimulation index of positive responses for the peptide and the ranked sum of peptide responses.

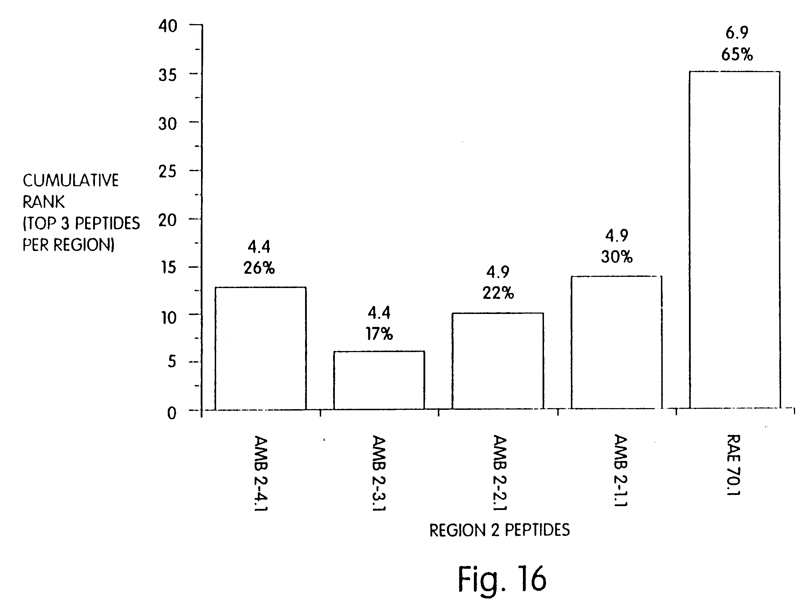

Fig. 16 is a graphic representation depicting the responses of T cell lines from 23 patients primed in vitro to recombinant Amb a I.1 protein and analyzed for response to selected peptides derived from Region 2 of the Amb a I.1 protein, by percent of positive responses within the individuals tested, the mean stimulation index of positive responses for the peptide and the ranked sum of peptide responses.

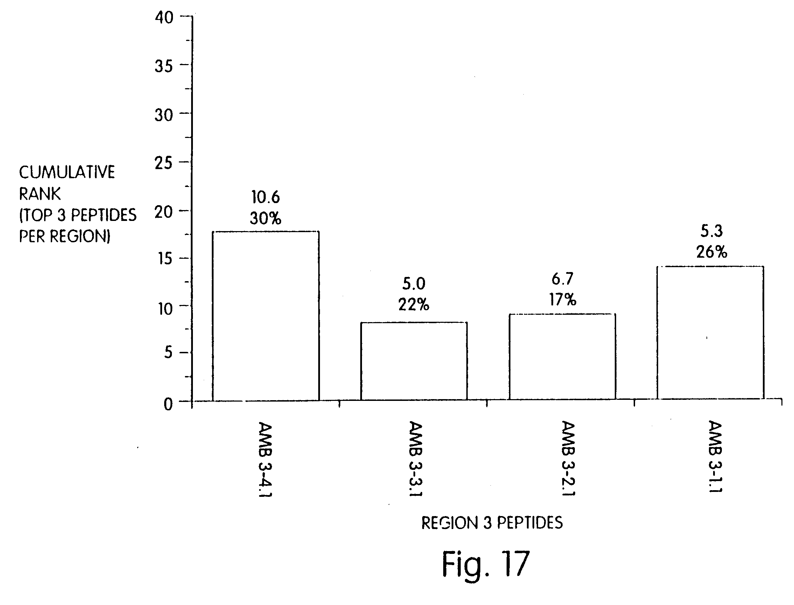

Fig. 17 is a graphic representation depicting the responses of T cell lines from 23 patients primed in vitro to recombinant Amb a I.1 protein and analyzed for response to selected peptides derived from Region 3 of the Amb a I.1 protein, by percent of positive responses within the individuals tested, the mean stimulation index of positive responses for the peptide and the ranked sum of peptide responses.

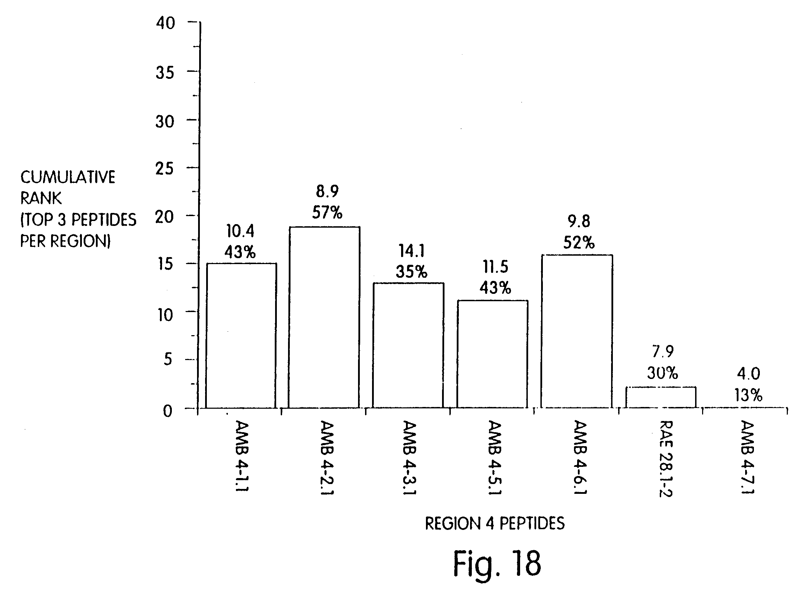

Fig. 18 is a graphic representation depicting the responses of T cell lines from 23 patients primed in vitro to recombinant Amb a I.1 protein and analyzed for response to selected peptides derived from Region 4 of the Amb a I.1 protein, by percent of positive responses within the individuals tested, the mean stimulation index of positive responses for the peptide and the ranked sum of peptide responses.

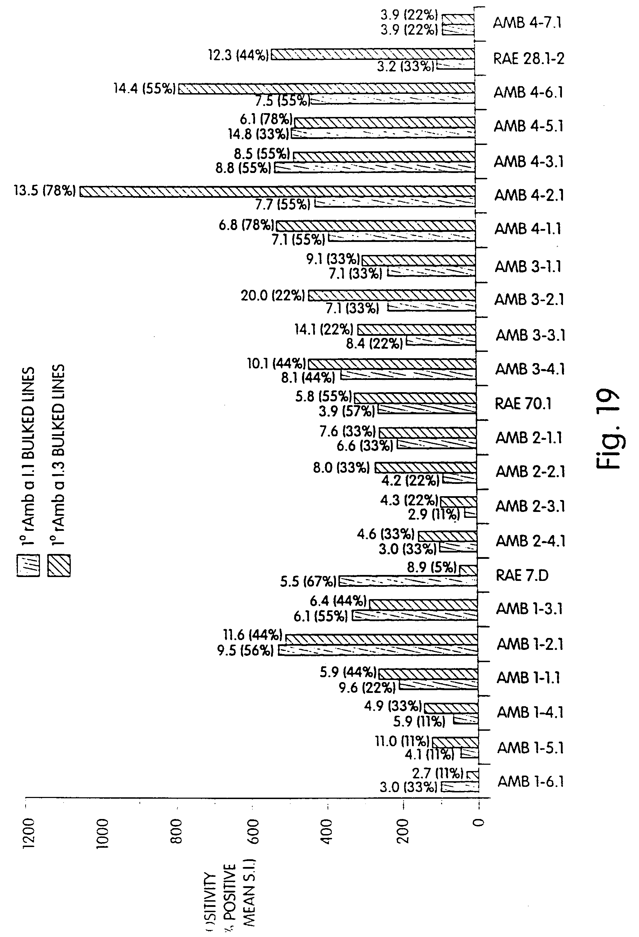

Fig. 19 is a graphic representation depicting the responses of T cell lines of 9 patients primed in vitro to recombinant Amb a I.1 protein or recombinant Amb a I.3 protein and analyzed for response to selected peptides derived from Amb a I.1, by percent of positive responses within the individuals tested and the mean stimulation index of positive responses for the peptide.

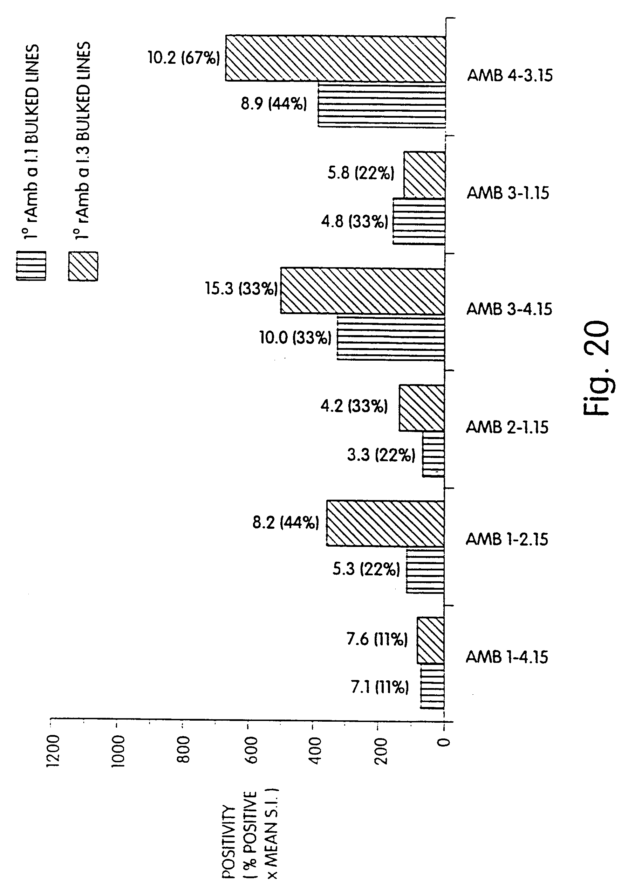

Fig. 20 is a graphic representation depicting the responses of T cell lines of 9 patients primed in vitro to recombinant Amb a I.1 protein or recombinant Amb a I.3 protein and analyzed for response to selected peptides derived from Amb a I.3, by percent of positive responses within the individuals tested and the mean stimulation index of positive responses for the peptide.

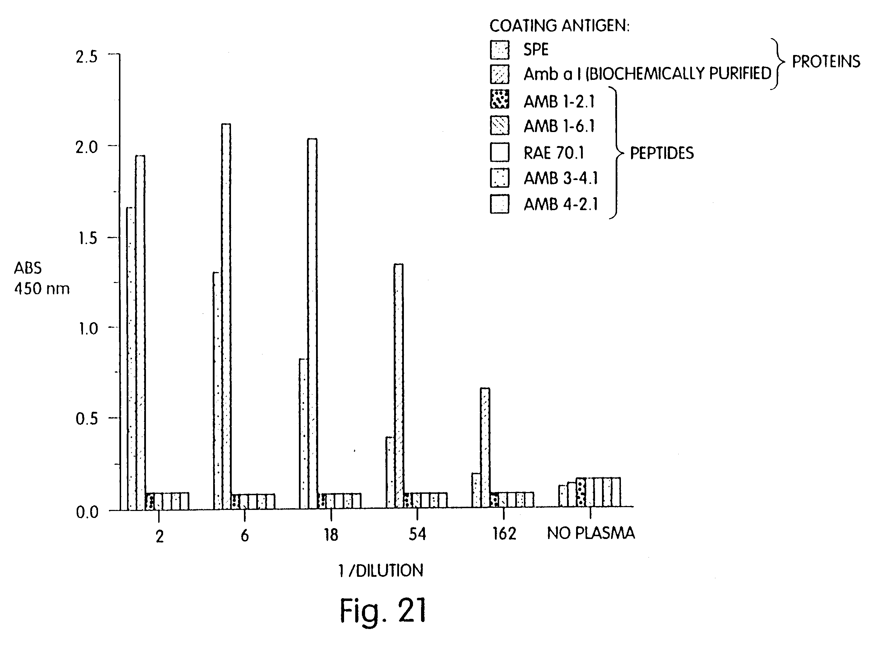

Fig. 21 is a graphic representation of a direct binding assay of IgE from a single ragweed allergic patient to peptides derived from Amb a I.

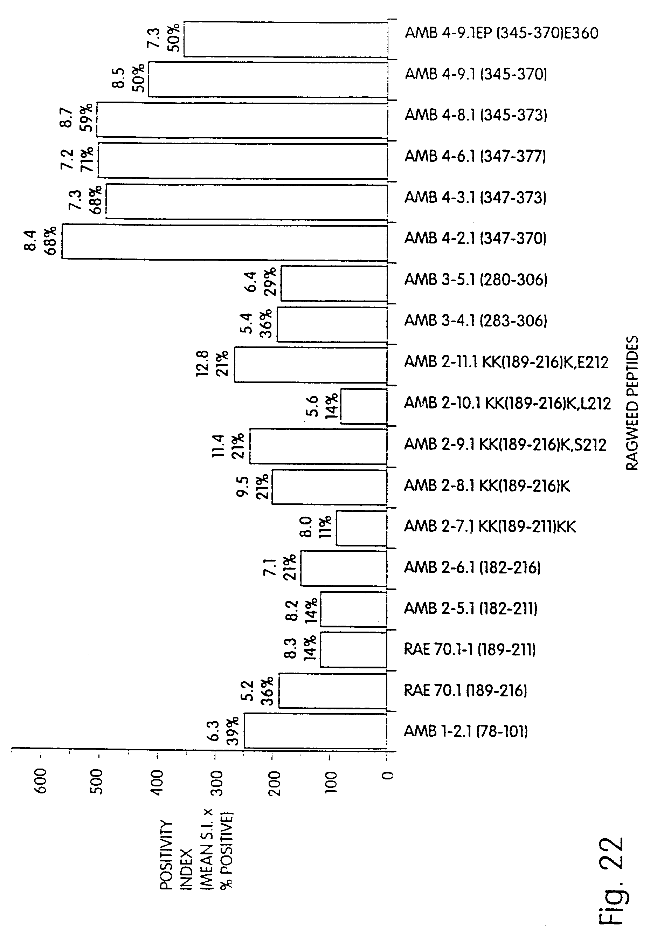

Fig. 22 is a graphic representation depicting the responses of T cell lines of 28 patients primed in vitro to recombinant Amb a I.1 protein and analyzed for response to selected peptides derived from Amb a I.1 by percent of positive responses within the individuals tested, the mean stimulation index of positive responses to the peptide and the ranked sum of peptide responses.

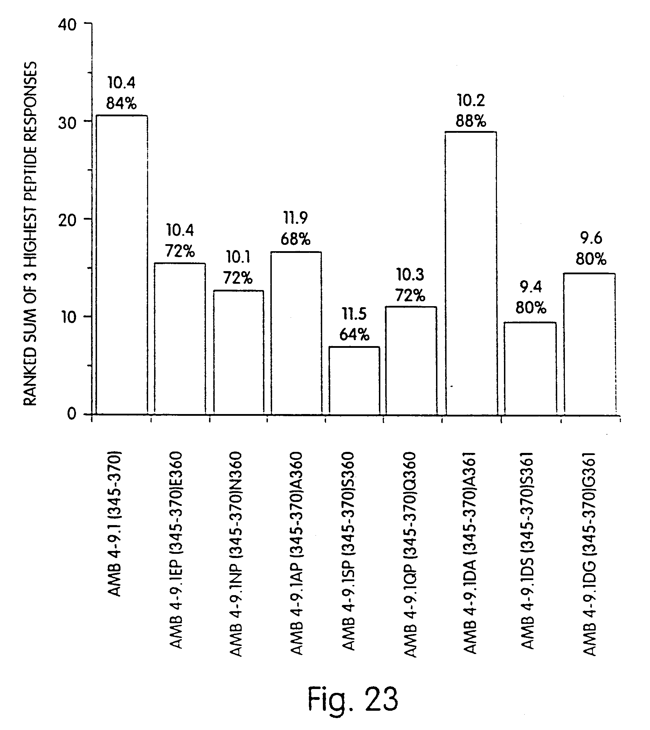

Fig. 23 is a graphic representation depicting the responses of T cell lines of 28 patients primed in vitro to recombinant Amb a I.1 protein and analyzed for response to selected peptides derived from Region 4 of Amb a I.1 by percent of positive responses within the individuals tested, the mean stimulation index of positive responses to the peptide and the ranked sum of peptide responses.

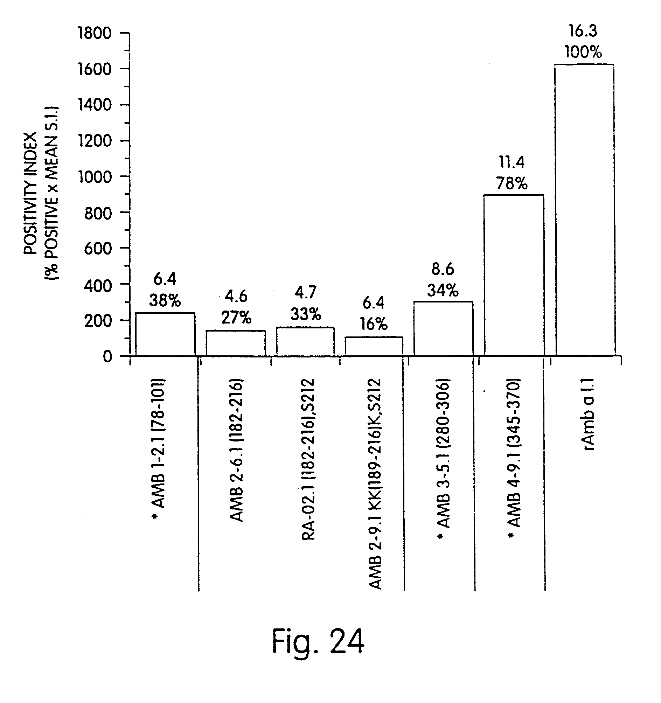

Fig. 24 is a graphic representation depicting the responses of T cell lines of 32 patients primed in vitro to recombinant Amb a I.1 protein and analyzed for response to six selected peptides derived from Amb a I.1 by percent of positive responses within the individuals tested, the mean stimulation index of positive responses to the peptide and the ranked sum of peptide responses.

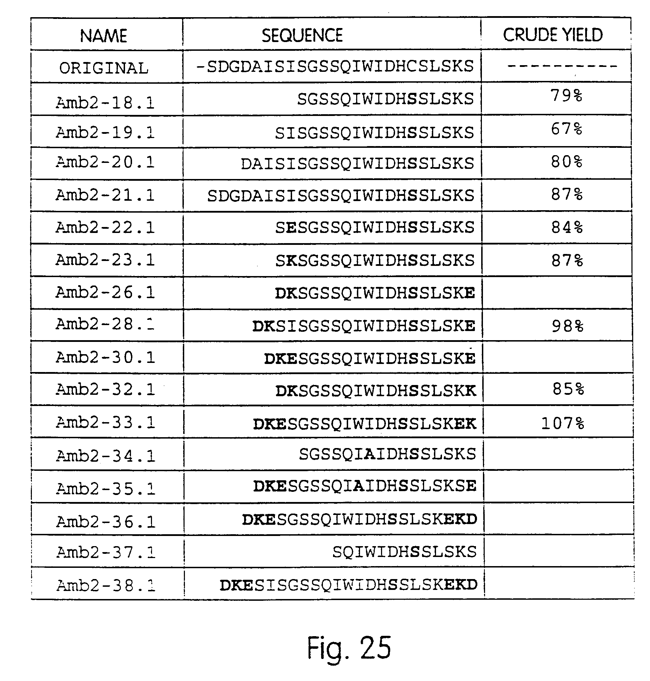

Fig. 25 shows various peptides derived from peptide RAE 70.1 which include modifications designed to increase the solubility of the peptide.

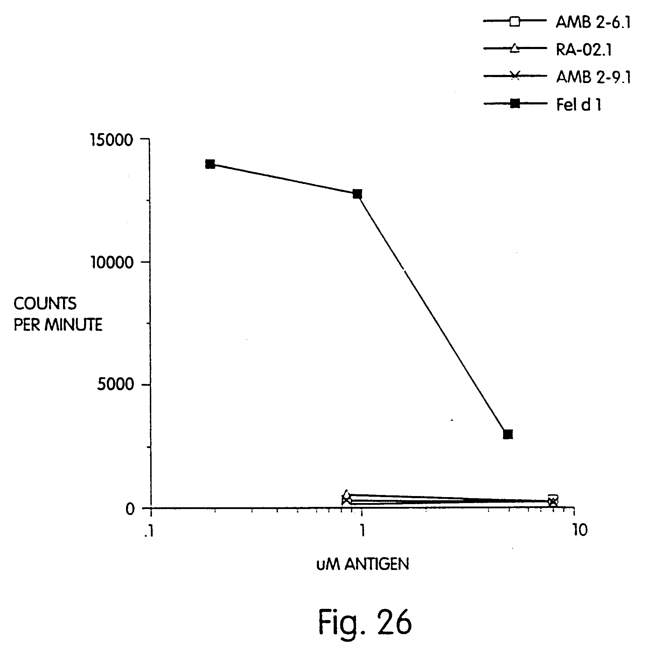

Fig. 26 is a graphic representation depicting the response of a T cell line from patient 956.2 primed in vitro to Fel d I and analyzed for response to various peptides derived from the Amb a I.1 protein.

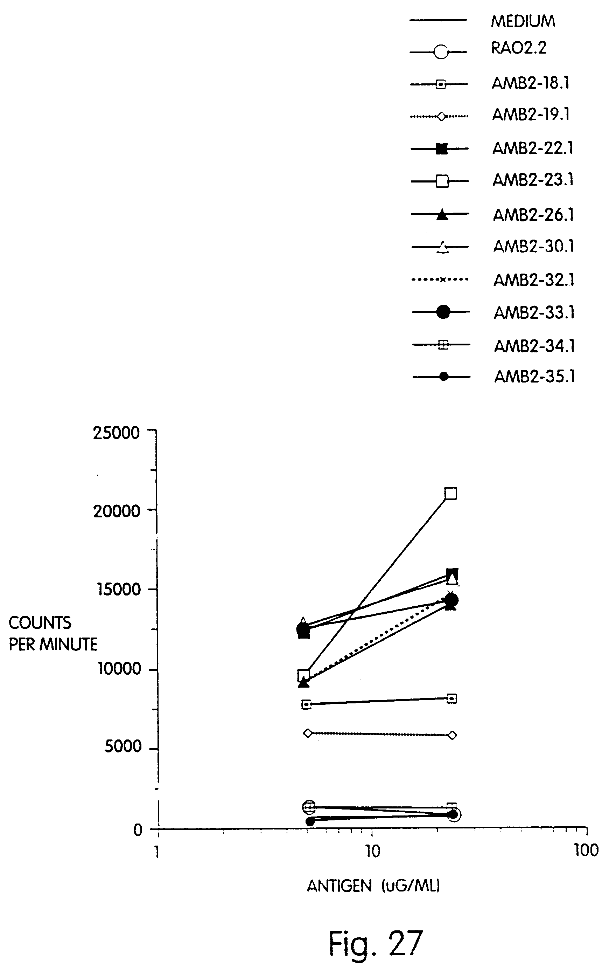

Fig. 27 is a graphic representation depicting the response of a T cell line from patient 119 primed in vitro with recombinant Amb a I.1 and analyzed for response to various modified peptides derived from Region 2 of the Amb a I.1 protein by tritiated thymidine incorporation.

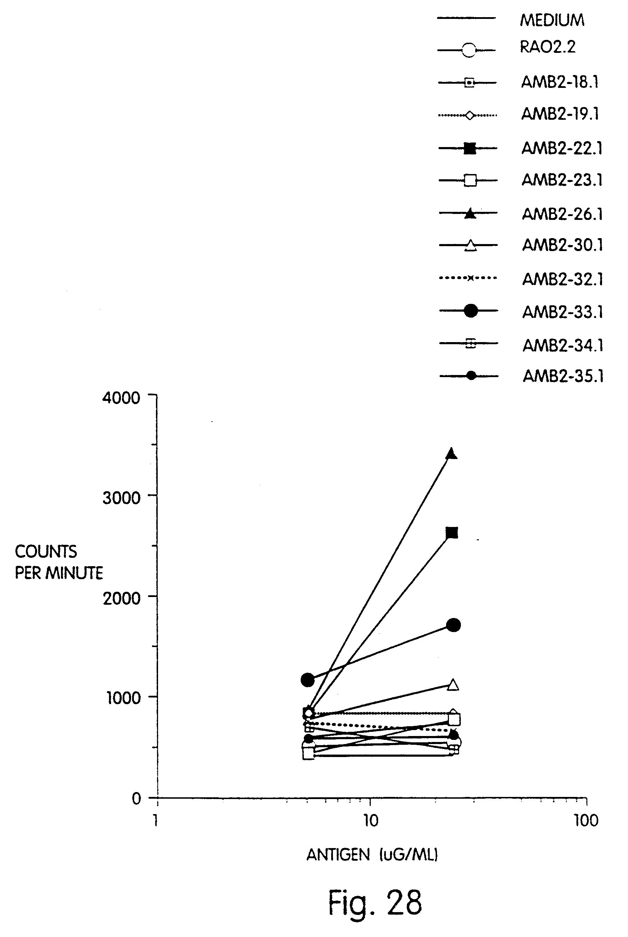

Fig. 28 is a graphic representation depicting the response of a T cell line from patient 1199 primed in vitro with recombinant Amb a I.1 and analyzed for response to various modified peptides derived from Region 2 of the Amb a I.1 protein by tritiated thymidine incorporation.

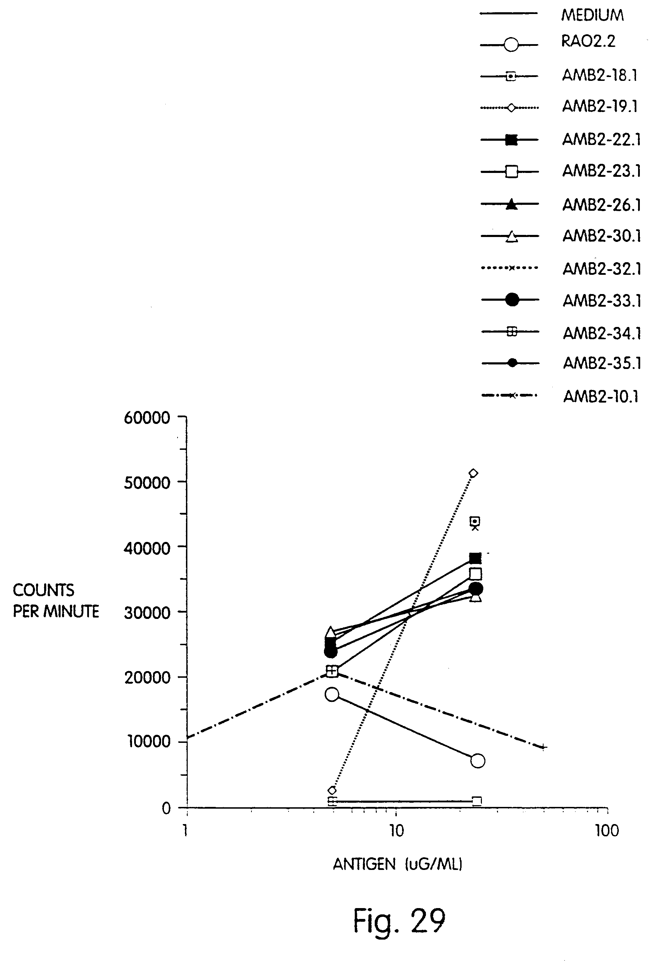

Fig. 29 is a graphic representation depicting the response of a T cell clone generated by limiting dilution from an Amb a I.1 specific T cell line stimulated with the AMB 2-10.1 peptide, primed in vitro w/recombinant Amb a I.1 and analyzed for response to various modified peptides derived from Region 2 of the Amb a I.1 protein by tritiated thymidine incorporation.

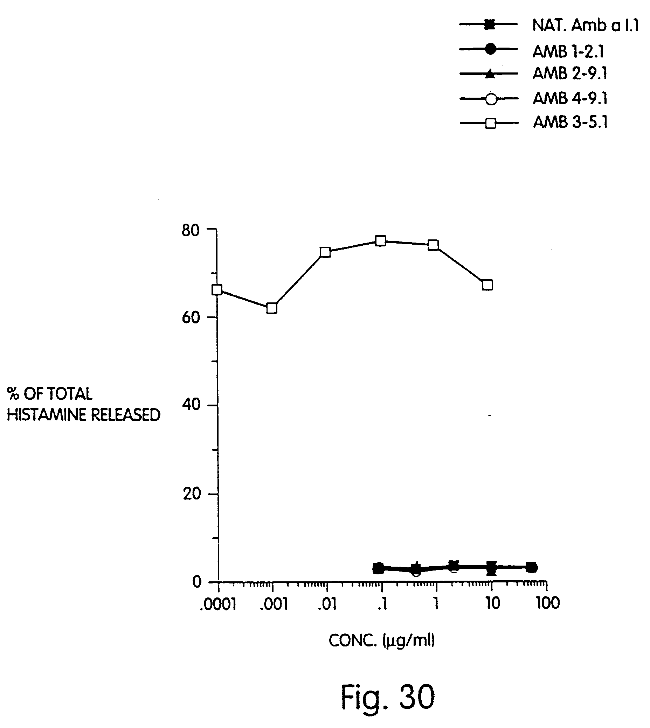

Fig. 30 is a graphic representation depicting the percent of total histamine release in blood samples from 8 ragweed-allergic patients in response to selected peptides derived from the Amb a I.1 protein.

### Detailed Description of the Invention

The present invention provides isolated peptides derived from the major protein allergens of Ambrosia artemisiifolia. As used herein, a peptide refers to an amino acid sequence having fewer amino acids than the entire amino acid sequence of a protein from which the peptide is derived. Peptides of the invention include peptides derived from Amb a I.1, Amb a I.2, Amb a I.3, Amb a I.4 and Amb a II which comprise at least one T cell epitope of the allergen.

Peptides comprising at least two regions, each region comprising at least one T cell epitope of a protein allergen of Ambrosia artemisiifolia are also within the scope of the invention. Each region of such peptides is derived from the same or from different ragweed pollen allergens. Isolated peptides or regions of isolated peptides, each comprising at least two T cell epitopes of a ragweed pollen allergen are particularly desirable for increased therapeutic effectiveness. Peptides which are immunologically related (e.g., by antibody or T cell cross-reactivity) to peptides of the present invention are also within the scope of the invention. Peptides immunologically related by antibody cross-reactivity are bound by antibodies specific for a peptide of a protein allergen of Ambrosia artemisiifolia. Peptides immunologically related by T cell cross-reactivity are capable of reacting with the same T cells as a peptide of the invention.

The present invention also pertains to a ragweed pollen allergen encoded by a nucleic acid sequence of clone IPCl/5. The full-length nucleic acid sequence of clone IPCl/5 has been determined and the encoded protein has been produced recombinantly in both the pSEM vector (as a fusion protein with β-galactosidase) and the pETlld vector. The recombinant protein was determined to bind approximately 10-20% of allergic serum IgE on a Western blot. The protein encoded by clone IPCl/5 was found to have a high degree of amino acid sequence homology with cysteine proteinase inhibitors in man and rice. The protein has 66.6% homology with the rice protein oryzacystatin-I. The nucleic acid sequence and deduced amino acid sequence of the allergen encoded by clone IPCl/5 is represented in SEQ ID NO. 11 and 12.

Isolated proteins and isolated peptides of the invention can be produced by recombinant DNA techniques in a host cell transformed with a nucleic acid having a sequence encoding such protein or peptide. The isolated proteins and isolated peptides of the invention can also be produced by chemical synthesis. In certain limited situations, isolated peptides can be produced by chemical cleavage of a protein allergen. When a protein or peptide is produced by recombinant techniques, host cells transformed with a nucleic acid having a sequence encoding the protein or peptide or the functional equivalent of the nucleic acid sequence are cultured in a medium suitable for the cells and protein or peptides can be purified from cell culture medium, host cells, or both using techniques known in the art for purifying proteins and peptides including ion-exchange chromatography, gel filtration chromatography, ultrafiltration, electrophoresis or immunopurification with antibodies specific for the protein or peptide, the protein allergen of Ambrosia artemisiifolia from which the peptide is derived, or a portion thereof. By isolated is meant that protein and peptides of the present invention are substantially free of cellular material or culture medium when produced by recombinant DNA techniques, or substantially free of chemical precursors or other chemicals when synthesized chemically. Recombinant ragweed pollen proteins including recombinant Amb a I.1, Amb a I.2, Amb a I.3, Amb a I.4, and Amb a II have been produced.

Suitable expression vectors for producing recombinant protein and recombinant peptides of the invention include pTRC, pGEX, pMAL, pRIT5, pETlld and pCA. The use of pTRC, pETlld and pGEX as expression vectors will result in expression of ragweed pollen protein as an unfused protein. The use of pMAL, pRIT5, pCA and pSEM as expression vectors will result in expression of ragweed pollen protein fused to maltose E binding protein (pMAL), protein A (pRIT5), truncated protein A (pCA), or β-galactosidase (pSEM). Suitable expression vectors are commercially available. When produced as a fusion protein, recombinant ragweed pollen protein can be recovered from the fusion protein through enzymatic or chemical (e.g., cyanogen bromide or dilute acid) cleavage and biochemical purification. For example, enzymatic cleavage sites for Factor X or thrombin can be introduced at the fusion junction between the carrier protein (e.g., Protein A) and the ragweed pollen protein. Suitable host cells for expression of recombinant ragweed pollen protein include bacteria, yeast and insect or mammalian cells. Appropriate vectors for expression in yeast include YepSec, pMFα and JRY88. These vectors are also commercially available.

To obtain isolated peptides of the present invention, a ragweed pollen allergen is divided into non-overlapping peptides of desired lengths or overlapping peptides of desired lengths as discussed in Example V which may be produced recombinantly, synthetically or in certain limited situations by chemical cleavage of the allergen. Peptides comprising at least one T cell epitope are capable of eliciting a T cell response, such as T cell proliferation or lymphokine secretion and/or are capable of inducing T cell anergy (i.e., tolerization). To determine peptides comprising at least one T cell epitope, isolated peptides are tested by, for example, T cell biology techniques to determine whether the peptides elicit a T cell response or induce T cell anergy. Those peptides found to elicit a T cell response or induce T cell anergy are defined as having T cell stimulating activity. As discussed in the Examples, human T cell stimulating activity can be tested by culturing T cells obtained from an individual sensitive to a ragweed pollen allergen, (i.e., an individual who has an IgE mediated immune response to a ragweed pollen allergen) with a peptide derived from the allergen and determining whether proliferation of T cells occurs in response to the peptide as measured, e.g., by cellular uptake of tritiated thymidine. As described in detail in the Examples, stimulation indices for responses by T cells to peptides can be calculated as the maximum CPM in response to a peptide divided by the medium control CPM. As used throughout this application, a peptide comprising at least one T cell epitope, when determined by T cell stimulation requires a stimulation index of at least 2.0. A peptide having a T cell stimulation index of 2.0 is considered useful as a therapeutic agent. Preferred peptides have a stimulation index of at least 2.5, more preferably at least 3.5, and most preferably at least 5.0.

In order to determine precise T cell epitopes by, for example, fine mapping techniques, a peptide having T cell stimulating activity and thus comprising at least one T cell epitope as determined by T cell biology techniques is modified by addition or deletion of amino acid residues at either the amino or carboxy terminus of the peptide and tested to determine a change in T cell reactivity to the modified peptide. If two or more peptides which share an area of overlap in the native protein sequence are found to have human T cell stimulating activity, as determined by T cell biology techniques, additional peptides can be produced comprising all or a portion of such peptides and these additional peptides can be tested by a similar procedure. Following this technique, peptides are selected and produced recombinantly or synthetically. Peptides are selected based on various factors, including the strength of the T cell response to the peptide (e.g., stimulation index), the frequency of the T cell response to the peptide in a population of individuals sensitive to ragweed pollen, and the potential cross-reactivity of the peptide with Amb a I family members and Amb a II. The physical and chemical properties of these selected peptides (e.g., solubility, stability) are examined to determine whether the peptides are suitable for use in therapeutic compositions or whether the peptides require modification as described herein. The ability of the selected peptides or selected modified peptides to stimulate human T cells (e.g., induce proliferation, lymphokine secretion) is determined.

In addition, preferred peptides of the invention do not bind immunoglobulin E (IgE) or bind IgE to a substantially lesser extent than the protein allergen from which the peptide is derived binds IgE. Recombinant ragweed pollen allergens including recombinant Amb a I.1, Amb a I.2, Amb a I.3, Amb a I.4, and Amb a II have been produced and shown to have reduced IgE binding activity as compared to the corresponding native protein allergen (See Fig. 3). The major complications of standard immunotherapy are IgE-mediated responses such as anaphylaxis. Immunoglobulin E is a mediator of anaphylactic reactions which result from the binding and cross-linking of antigen to IgE on mast cells or basophils and the release of mediators (e.g., histamine, serotonin, eosinophil chemotacic factors). Thus, anaphylaxis in a substantial percentage of a population of individuals sensitive to ragweed pollen allergen could be avoided by the use in immunotherapy of a recombinant protein, a peptide or peptides which do not bind IgE in a substantial percentage (e.g., at least about 75%) of a population of individuals sensitive to ragweed pollen allergen, or if the protein or peptide(s) binds IgE, such binding does not result in the release of mediators from mast cells or basophils. Additionally, the risk of anaphylaxis can be reduced by the use in immunotherapy of a recombinant protein, a peptide or peptides which have reduced IgE binding. Moreover, recombinant protein or peptides which have minimal IgE stimulating activity are desirable for therapeutic effectiveness. Minimal IgE stimulating activity refers to IgE production that is less than the amount of IgE production and/or IL-4 production stimulated by the native protein allergen (e.g., Amb a I.1).

A peptide or recombinant protein of the invention, when administered to a ragweed pollen-sensitive individual, is capable of modifying the allergic response of the individual to the allergen. Particularly, peptides of the invention comprising at least one T cell epitope of a ragweed pollen allergen or at least two regions derived from a ragweed pollen allergen each comprising at least one T cell epitope, when administered to a ragweed pollen-sensitive individual are capable of modifying the T cell response of the individual to the allergen. As used herein, modification of the allergic response of a ragweed pollen-sensitive individual to a ragweed pollen allergen can be defined as non-responsiveness or diminution in symptoms to a ragweed pollen allergen, as determined by standard clinical procedures (see e.g., Varney et al., British Medical Journal 302: 265-269 (1990)).

As a result of the work described herein, peptides derived from ragweed pollen allergens comprising at least one T cell epitope have been produced. T cell epitopes are believed to be involved in initiation and perpetuation of the immune response to ragweed pollen allergen(s) which are responsible for the clinical symptoms of ragweed pollen allergy. These T cell epitopes are thought to trigger early events at the level of the T helper cell by binding to an appropriate HLA molecule on the surface of an antigen presenting cell and stimulating the relevant T cell subpopulation. These events lead to T cell proliferation, lymphokine secretion, local inflammatory reactions, the recruitment of additional immune cells to the site, and activation of the B cell cascade leading to production of antibodies. One isotype of these antibodies, IgE, is fundamentally important in the development of allergic symptoms and its production is influenced early in the cascade of events, at the level of the T helper cell, by the nature of the lymphokines secreted. A T cell epitope is the basic element or smallest unit of recognition by a T cell receptor, where the epitope comprises amino acid residues essential to receptor recognition which may be contiguous and/or non-contiguous in the amino acid sequence of the protein. Amino acid sequences which mimic those of T cell epitopes and which modify the allergic response to protein allergens of Ambrosia artemisiifolia are within the scope of this invention.

Exposure of ragweed pollen allergic patients to peptides of the present invention may tolerize or anergize appropriate T cell subpopulations such that they become unresponsive to ragweed pollen allergen(s) and do not participate in mounting an immune response upon such exposure. In addition, administration of a peptide of the present invention may modify the lymphokine secretion profile as compared with exposure to the naturally-occurring ragweed pollen allergen or portion thereof (e.g., result in a decrease of IL-4 and/or an increase in IL-2). Furthermore, exposure to a peptide of the invention may influence T cell subpopulations which normally participate in the response to ragweed pollen allergen(s) such that these T cells are drawn away from the site(s) of normal exposure to the allergen (e.g., nasal mucosa, skin, and lung) towards the site(s) of therapeutic administration of the peptide. This redistribution of T cell subpopulations may ameliorate or reduce the ability of an individual's immune system to stimulate the immune response at the site of normal exposure to the ragweed pollen allergen(s), resulting in a diminution in allergic symptoms.

Isolated peptides of the invention comprise at least one T cell epitope of a protein allergen of Ambrosia artemisiifolia (i.e., the peptide comprises at least approximately seven amino acid residues of the protein allergen). For purposes of therapeutic effectiveness, therapeutic compositions of the invention preferably comprise at least two T cell epitopes of a ragweed pollen allergen. Accordingly, isolated peptides of the invention preferably comprise at least two T cell epitopes (i.e., the peptide comprises at least approximately eight amino acid residues, and preferably fifteen amino acid residues). Additionally, isolated peptides of the invention preferably comprise a sufficient percentage of the T cell epitopes of the entire protein allergen such that upon administration of the peptide to an individual sensitive to ragweed pollen, T cells of individual are tolerized to the protein allergen. Isolated peptides of the invention comprising up to approximately 45 amino acid residues in length, and most preferably up to approximately 30 amino acid residues in length are particularly desirable as increases in length may result in difficulty in peptide synthesis as well as retention of an undesirable property (e.g., immunoglobulin binding or enzymatic activity) due to maintenance of conformational similarity between the peptide and the protein allergen from which it is derived. All of the peptides shown in Fig. 8 were found to have human T cell stimulating activity.

Preferred peptides comprise all or a portion of the areas of major T cell reactivity within the Amb a I.1 protein allergen, i.e., Region 1, Region 2, Region 3 and Region 4. Each area is broadly defined as follows: Region 1 comprises amino acid residues 48-107; Region 2 comprises amino acid residues 171-216; Region 3 comprises amino acid residues 278-322; and Region 4 comprises amino acid residues 331-377. Preferred areas of major T cell reactivity within each Region comprise: amino acid residues 57-101; amino acid residues 182-216; amino acid residues 280-322; and amino acid residues 342-377. Similar areas of major T cell reactivity can be found within the other Amb a I family members (i.e., Amb a I.2, Amb a I.3 and Amb a I.4), and Amb a II. As shown in Example VIII, the Amb a I protein allergens and Amb a II demonstrate a high degree of T cell cross-reactivity. Given this cross-reactivity, shared areas of major T cell reactivity and shared T cell epitopes are likely to be found in conserved regions between Amb a I and the remaining Amb a I family members and Amb a II. For example, Amb a I.1 stimulated T cells have been shown to recognize both Amb a I.1 derived peptides and homologous Amb a I.3 derived peptides (See Example IX). Similarly, Amb a I.3 stimulated T cell recognize both Amb a I.1 and Amb a I.3 derived peptides.

Preferred ragweed pollen peptides comprise all or a portion of the following peptides: RAE 67.1 (SEQ ID NO:13); RAE 57.1 (SEQ ID NO:14); RAE 24.E (SEQ ID NO:15); RAE 24.1 (SEQ ID NO:16); RAE 22.E (SEQ ID NO:17); RAE 22.E-1 (SEQ ID NO:18); RAE 3.D (SEQ ID NO:19); RAE 3.1 (SEQ ID NO:20); RAE 22.E-2 (SEQ ID NO:21); RAE 5.D (SEQ ID NO:22); RAE 6.D (SEQ ID NO:23); RAE 6.1 (SEQ ID NO:24); RAE 7.D (SEQ ID NO:25); RAE 7.D-1 (SEQ ID NO:26); RAE 40.1-6 (SEQ ID NO:27); RAE 40.1-5 (SEQ ID NO:28); RAE 40.1-4 (SEQ ID NO:29); RAE 40.D (SEQ ID NO:30); RAE 40.1 (SEQ ID NO:31); RAE 61.1 (SEQ ID NO:32); RAE 80.1 (SEQ ID NO:33); RAE 45.1 (SEQ ID NO:34); RAE 75.1 (SEQ ID NO:35); RAE 62.1 (SEQ ID NO:36); RAE 69.1 (SEQ ID NO:37); RAE 69.1-1 (SEQ ID NO:38); RAE 69.1-2 (SEQ ID NO:39); RAE 69.1-3 (SEQ ID NO:40); RAE 70.1-3 (SEQ ID NO:41); RAE 70.1-2 (SEQ ID NO:42); RAE 70.1-1 (SEQ ID NO:43); RAE 70.1 (SEQ ID NO:44); RAE 71.1 (SEQ ID NO:45); RAE 65.1 (SEQ ID NO:46); RAE 63.1 (SEQ ID NO:47); RAE 76.1 (SEQ ID NO:48); RAE 27.1 (SEQ ID NO:49); RAE 66.1 (SEQ ID NO:50); RAE 66.1-1 (SEQ ID NO:51); RAE 66.1-2 (SEQ ID NO:52); RAE 66.1-3 (SEQ ID NO:53); RAE 64.1-3 (SEQ ID NO:54); RAE 64.1-2 (SEQ ID NO:55); RAE 64.1-1 (SEQ ID NO:56); RAE 64.1 (SEQ ID NO:57); RAE 73.1 (SEQ ID NO:58); RAE 74.1 (SEQ ID NO:59); RAE 74.1-1 (SEQ ID NO:60); RAE 29.1 (SEQ ID NO:61); RAE 29.1-1 (SEQ ID NO:62); RAE 28+29 (SEQ ID NO:63); RAE 29.1-2 (SEQ ID NO:64); RAE 29.1-3 (SEQ ID NO:65); RAE 29.1-4 (SEQ ID NO:66); RAE 28.1-3 (SEQ ID NO:67); RAE 28.1-2 (SEQ ID NO:68); RAE 28.1-1 (SEQ ID NO:69); RAE 28.1 (SEQ ID NO:70); RAE 20.1 (SEQ ID NO:71); RAE 20.1-3 (SEQ ID NO:72); RAE 20.1-2 (SEQ ID NO:73); RAE 20.1-1 (SEQ ID NO:74); RAE 21.1 (SEQ ID NO:75); RAE 17.1 (SEQ ID NO:76); RAE 55.1 (SEQ ID NO:77); RAE 76.6 (SEQ ID NO:78); RAE 67.15 (SEQ ID NO:79); RAE 45.15 (SEQ ID NO:80); RAE 27.15 (SEQ ID NO:81); AMB 1-1.1 (SEQ ID NO:85); AMB 1-2.1 (SEQ ID NO:86); AMB 1-3.1 (SEQ ID NO:87); AMB 1-4.1 (SEQ ID NO:84); AMB 1-5.1 (SEQ ID NO:83); AMB 1-6.1 (SEQ ID NO:82); AMB 1-4.15 (SEQ ID NO:88); AMB 1-2.15 (SEQ ID NO:89); AMB 2-4.1 (SEQ ID NO:90); AMB 2-3.1 (SEQ ID NO:91); AMB 2-5.1 (SEQ ID NO:92); AMB 2-6.1 (SEQ ID NO:93); AMB 2-2.1 (SEQ ID NO:94); AMB 2-1.1 (SEQ ID NO:95); AMB 2-7.1 (SEQ ID NO:96); AMB 2-8.1 (SEQ ID NO:97); AMB 2-9.1 (SEQ ID NO:98); AMB 2-10.1 (SEQ ID NO:99); AMB 2-11.1 (SEQ ID NO:100); AMB 2-1.15 (SEQ ID NO:101); AMB 3-4.1 (SEQ ID NO:103); AMB 3-5.1 (SEQ ID NO:102); AMB 3-3.1 (SEQ ID NO:104); AMB 3-2.1 (SEQ ID NO:105); AMB 3-1.1 (SEQ ID NO:106); AMB 3-4.15 (SEQ ID NO:107); AMB 3-1.15 (SEQ ID NO:108); AMB 4-8.1 (SEQ ID NO:109); AMB 4-9.1 (SEQ ID NO:110); AMB 4-6.1 (SEQ ID NO:111); AMB 4-5.1 (SEQ ID NO:112); AMB 4-3.1 (SEQ ID NO:113); AMB 4-2.1 (SEQ ID NO:114); AMB 4-1.1 (SEQ ID NO:115); AMB 4-3.15 (SEQ ID NO:116); Amb 2-18.1 (SEQ ID NO:126); Amb 2-19.1 (SEQ ID NO:127); Amb 2-20.1 (SEQ ID NO:128); Amb 2-21.1 (SEQ ID NO:129); Amb 2-22.1 (SEQ ID NO:130); Amb 2-23.1 (SEQ ID NO:131); Amb 2-26.1 (SEQ ID NO:132); Amb 28.1 (SEQ ID NO:133); Amb 2-30.1 (SEQ ID NO:134); Amb 2-32.1 (SEQ ID NO:135); Amb 2-33.1 (SEQ ID NO:136); Amb 2-34.1 (SEQ ID NO:137); Amb 2-35.1 (SEQ ID NO:138); Amb 2-36.1 (SEQ ID NO:139); Amb 2-37.1 (SEQ ID NO:140); Amb 2-38.1 (SEQ ID NO:141); AMB 4-9.1EP (SEQ ID NO:142); AMB 4-9.1NP (SEQ ID NO:143); AMB 4-9.1AP (SEQ ID NO:144); AMB 4-9.1SP (SEQ ID NO:145); AMB 4-9.1QP (SEQ ID NO:146); AMB 4-9.1DA (SEQ ID NO:147); amb 4-9.1DS (SEQ ID NO:148); AMB 4-9.1DG (SEQ ID NO:149); and RA-02.1 (SEQ ID NO:150), the amino acid sequences of such peptides being shown in Figs. 7, 14, 23, 24 and 25. Particularly preferred peptides comprise all or a portion of the following peptides: AMB 1-2.1 (SEQ ID NO:86); AMB 2-6.1 (SEQ ID NO:93); AMB 2-4.1 (SEQ ID NO:90); Amb 2-36.1 (SEQ ID NO:139); Amb 2-38.1 (SEQ ID NO:141); RA-02.1 (SEQ ID NO:150); AMB 2-9.1 (SEQ ID NO:98); AMB 3-5.1 (SEQ ID NO:102); and AMB 4-9.1 (SEQ ID NO:110).

Another embodiment of the present invention provides peptides comprising at least two regions, each region comprising at least one T cell epitope of a protein allergen of Ambrosia artemisiifolia (e.g., each region comprises at least approximately seven amino acid residues). These peptides comprising at least two regions can comprise as many amino acid residues as desired and preferably comprise at least about 7, more preferably at least about 15, even more preferably about 30 and most preferably at least about 40 amino acid residues of a ragweed pollen allergen. Each region of such peptide preferably comprises up to 45 amino acid residues in length, more preferably up to 40 residues in length and most preferably up to 30 amino acid residues in length as increases in length of a region may result in difficulty in peptide synthesis as well as retention of an undesirable property (e.g., immunoglobulin binding or enzymatic activity) due to maintenance of conformational similarity between the peptide and the protein allergen from which it is derived. If desired, the amino acid sequences of the regions can be produced and joined by a linker to increase sensitivity to processing by antigen-presenting cells. Such linker can be any non-epitope amino acid sequence or other appropriate linking or joining agent. To obtain preferred peptides comprising at least two regions, each comprising at least one T cell epitope, the regions are arranged in a configuration different from a naturally-occurring configuration of the regions in the allergen. For example, the regions containing T cell epitope(s) can be arranged in a noncontiguous configuration and can preferably be derived from the same protein allergen. Noncontiguous is defined as an arrangement of regions containing T cell epitope(s) which is different than that of an amino acid sequence present in the protein allergen from which the regions are derived. Furthermore, the noncontiguous regions containing T cell epitopes can be arranged in a nonsequential order (e.g., in an order different from the order of the amino acids of the native protein allergen from which the region containing T cell epitope(s) are derived in which amino acids are arranged from an amino terminus to a carboxy terminus). A peptide can comprise at least 15%, at least 30%, at least 50% or up to 100% of the T cell epitopes of a ragweed pollen allergen.

The individual peptide regions can be produced and tested to determine which regions bind immunoglobulin E specific for a ragweed pollen allergen and which of such regions would cause the release of mediators (e.g., histamine) from mast cells or basophils. Those peptide regions found to bind immunoglobulin E and cause the release of mediators from mast cells or basophils in greater than approximately 10-15% of the allergic sera tested are preferably not included in the peptide regions arranged to form peptides of the invention.

Preferred peptides of the invention comprise two or more regions derived from the same or from different ragweed pollen allergens (e.g., Amb a I.1, Amb a I.2, Amb a I.3, Amb a I.4 and Amb a II). For example, one region can be derived from Amb a I.1 and one region can be derived from Amb a I.2; one region can be derived from Amb a I.1 and one region can be derived from Amb a I.3; one region can be derived from Amb a I.1 and one region can be derived from Amb a I.4; one region can be derived from Amb a I.2 and one region can be derived from Amb a I.3; one region can be derived from Amb a I.2 and one region can be derived from Amb a I.4; one region can be derived from Amb a I.3 and one region can be derived from Amb a I.4; one region can be derived from Amb a I.1 and one region can be derived from Amb a II; one region can be derived from Amb a I.2 and one region can be derived from Amb a II; one region can be derived from Amb a I.3 and one region can be derived from Amb a II; and one region can be derived from Amb a I.4 and one region can be derived from Amb a II. Alternatively, the regions can be derived from the same protein allergen, e.g., Amb a I.1 and Amb a I.1, etc.

Regions of a peptide of the invention preferably comprise all or a portion of Region 1, Region 2, Region 3 and Region 4 of Amb a I.1, and the above discussed preferred areas of major T cell reactivity within each Region. If Region 1, 2, 3 or 4 is found to bind IgE and cause the release of mediators from mast cells or basophils, then it is preferred that more than one region of the peptide comprise such Region and that the various regions of the peptide do not bind IgE or cause release of mediators from mast cells or basophils. Examples of preferred regions include: AMB 1-1.1 (SEQ ID NO:85); AMB 1-2.1 (SEQ ID NO:86); AMB 1-3.1 (SEQ ID NO:87); AMB 1-4.1 (SEQ ID NO:84); AMB 1-5.1 (SEQ ID NO:83); AMB 1-6.1 (SEQ ID NO:82); AMB 1-4.15 (SEQ ID NO:88); AMB 1-2.15 (SEQ ID NO:89); AMB 2-4.1 (SEQ ID NO:90); AMB 2-3.1 (SEQ ID NO:91); AMB 2-5.1 (SEQ ID NO:92); AMB 2-6.1 (SEQ ID NO:93); AMB 2-2.1 (SEQ ID NO:94); AMB 2-1.1 (SEQ ID NO:95); AMB 2-7.1 (SEQ ID NO:96); AMB 2-8.1 (SEQ ID NO:97); AMB 2-9.1 (SEQ ID NO:98); AMB 2-10.1 (SEQ ID NO:99); AMB 2-11.1 (SEQ ID NO:100); AMB 2-1.15 (SEQ ID NO:101); AMB 3-4.1 (SEQ ID NO:103); AMB 3-5.1 (SEQ ID NO:102); AMB 3-3.1 (SEQ ID NO:104); AMB 3-2.1 (SEQ ID NO:105); AMB 3-1.1 (SEQ ID NO:106); AMB 3-4.15 (SEQ ID NO: 107); AMB 3-1.15 (SEQ ID NO:108); AMB 4-8.1 (SEQ ID NO:109); AMB 4-9.1 (SEQ ID NO:110); AMB 4-6.1 (SEQ ID NO:111); AMB 4-5.1 (SEQ ID NO:112); AMB 4-3.1 (SEQ ID NO:113); AMB 4-2.1 (SEQ ID NO:114); AMB 4-1.1 (SEQ ID NO:115); AMB 4-3.15 (SEQ ID NO:116); RA-02.1 (SEQ ID NO:150); Amb 2-36.1 (SEQ ID NO:139); and Amb 2-38.1 (SEQ ID NO:141), the amino acid sequences of such regions being shown in Fig. 14, Fig. 24 or Fig. 25, or portions of said regions comprising at least one T cell epitope.

Preferred peptides comprise various combinations of two or more regions, each region comprising all or a portion of Region 1, Region 2, Region 3 or Region 4 of Amb a I.1. Preferred peptides comprise various combinations of two or more regions, each region having an amino acid sequence as shown in Fig. 14, such combination of regions including the following: AMB 4-6.1 and RAE 70.1 (SEQ ID NO:111 and SEQ ID NO:44); AMB 4-6.1 and AMB 2-5.1 (SEQ ID NO:111 and SEQ ID NO:92); AMB 4-9.1 and AMB 2-5.1 (SEQ ID NO:110 and SEQ ID NO:92); AMB 4-9.1 and RAE 70.1 (SEQ. ID NO:110 and SEQ ID NO:44); AMB 4-6.1, AMB 2-5.1 and AMB 1-2.1 (SEQ ID NO:111, SEQ ID NO:92 and SEQ ID NO:86); AMB 4-9.1, AMB 2-5.1 and AMB 1-2.1 (SEQ ID NO:110, SEQ ID NO:92 and SEQ ID NO:86); AMB 4-6.1, RAE 70.1 and AMB 1-2.1 (SEQ ID NO:111, SEQ ID NO:44 and SEQ ID NO:86); AMB 4-9.1, RAE 70.1 and AMB 1-2.1 (SEQ ID NO:110, SEQ ID NO:44 and SEQ ID NO:86); AMB 4-6.1, RAE 70.1, AMB 1-2.1 and AMB 3-5.1 (SEQ ID NO:111, SEQ ID NO:44, SEQ ID NO:86 and SEQ ID NO:102); AMB 4-9.1, RAE 70.1, AMB 1-2.1 and AMB 3-5.1 (SEQ ID NO:110, SEQ ID NO:44, SEQ ID NO:86 and SEQ ID NO:102); AMB 4-6.1, AMB 2-5.1, AMB 1-2.1 and AMB 3-5.1 (SEQ ID NO:111, SEQ ID NO:92, SEQ ID NO:86 and SEQ ID NO:102); AMB 4-9.1, AMB 2-5.1, AMB 1-2.1 and AMB 3-5.1 (SEQ ID NO:110, SEQ ID NO:92, SEQ ID NO:86 and SEQ ID NO:102); AMB 4-6.1, RAE 70.1, AMB 1-2.1 and AMB 3-4.1 (SEQ ID NO:111, SEQ ID NO:44, SEQ ID NO:86 and SEQ ID NO:103); AMB 4-9.1, RAE 70.1, AMB 1-2.1 and AMB 3-4.1 (SEQ ID NO:110, SEQ ID NO:44, SEQ ID NO:86 and SEQ ID NO:103); AMB 4-6.1, AMB 2-5.1, AMB 1-2.1 and AMB 3-4.1 (SEQ ID NO:111, SEQ ID NO:92, SEQ ID NO:86 and SEQ ID NO:103); AMB 4-9.1, AMB 2-5.1, AMB 1-2.1 and AMB 3-4.1 (SEQ ID NO:110, SEQ ID NO:92, SEQ ID NO:86 and SEQ ID NO:103); AMB 2-1.15 and AMB 4-3.15 (SEQ ID NO:101, and SEQ ID NO:116); AMB 1-2.15, AMB 2-1.15 and AMB 4-3.15 (SEQ ID NO:89, SEQ ID NO:101, and SEQ ID NO:116); and AMB 1-2.15, AMB 2-1.15, AMB 4-3.15 and AMB 3-4.15 (SEQ ID NO:89, SEQ ID NO:101, SEQ ID NO:116 and SEQ ID NO:107).

Peptides of protein allergens of Ambrosia artemisiifolia within the scope of the invention can be used in methods of treating and preventing allergic reactions to ragweed pollen allergens. Thus, one aspect of the present invention provides therapeutic compositions comprising a peptide of Amb a I.1, Amb a I.2, Amb a I.3, Amb a I.4 or Amb a II including at least one T cell epitope, or preferably at least two T cell epitopes, and a pharmaceutically acceptable carrier or diluent. In another aspect, the therapeutic composition comprises a pharmaceutically acceptable carrier or diluent and a peptide comprising at least two regions, each region comprising at least one T cell epitope of a ragweed pollen allergen and is derived from the same or from different ragweed pollen allergens.

Administration of the therapeutic compositions of the present invention to desensitize an individual can be carried out using known techniques. For example, a peptide derived from a ragweed pollen allergen comprising at least one T cell epitope can be administered in combination with an appropriate diluent, a carrier, and/or an adjuvant. Preferably, peptides are administered in soluble form. Pharmaceutically acceptable diluents include saline and aqueous buffer solutions. Pharmaceutically acceptable carriers include polyethylene glycol (Wie et al., International Archives of Allergy and Applied Immunology 64: 84-99 (1981)) and liposomes (Strejan et al., Journal of Neuroimmunology 7: 27 (1984)). Pharmaceutically acceptable adjuvants include alum. For purposes of inducing T-cell anergy or tolerance in an individual, the therapeutic composition is preferably administered in non-immunogenic form, e.g., one that does not include adjuvant. Such compositions will generally be administered by injection (e.g., intravenous, subcutaneous, intramuscular), oral administration, (e.g., as in the form of a capsule), inhalation, transdermal application or rectal administration. Preferably, therapeutic compositions are administed subcutaneously.

The therapeutic compositions of the invention are administered to ragweed pollen-sensitive individuals at dosages and for lengths of time effective to reduce sensitivity (i.e., reduce the allergic response) or tolerize an individual to a ragweed pollen allergen. A therapeutically effective amount of one or more of the same or of different therapeutic composition can be administered simultaneously or sequentially to a ragweed pollen-sensitive individual. Effective amounts of the therapeutic compositions will vary according to factors such as the degree of sensitivity of the individual to ragweed pollen allergens, the age, sex, and weight of the individual, and the ability of the peptide to stimulate a T cell response in the individual.

In yet another aspect of the present invention, a composition is provided comprising at least two peptides (e.g., a physical mixture of at least two peptides), each comprising at least one T cell epitope of a protein allergen of Ambrosia artemisiifolia. Compositions comprising several peptides or combinations of separate peptides can include as many peptides as desired (e.g., 5, 6, 7...) for therapeutic efficacy. The peptides are derived from the same or from different ragweed pollen allergens. Such compositions can be administered in the form of a therapeutic composition with a pharmaceutically acceptable carrier or diluent. Preferably, peptides are administered in soluble form. A therapeutically effective amount of one or more of such compositions can be administered simultaneously or sequentially, preferably subcutaneously, to a ragweed pollen-sensitive individual to desensitize or tolerize the individual to ragweed pollen.

Preferred compositions and preferred combinations of peptides which can be administered simultaneously or sequentially (comprising peptides having amino acid sequences shown in Fig. 14) include the following combinations: AMB 4-6.1 and RAE 70.1 (SEQ ID NO:111 and SEQ ID NO:44); AMB 4-6.1 and AMB 2-5.1 (SEQ ID NO:111 and SEQ ID NO:92); AMB 4-9.1 and AMB 2-5.1 (SEQ ID NO:110 and SEQ ID NO:92); AMB 4-9.1 and RAE 70.1 (SEQ. ID NO:110 and SEQ ID NO:44); AMB 4-6.1, AMB 2-5.1 and AMB 1-2.1 (SEQ ID NO:111, SEQ ID NO:92 and SEQ ID NO:86); AMB 4-9.1, AMB 2-5.1 and AMB 1-2.1 (SEQ ID NO:110, SEQ ID NO:92 and SEQ ID NO:86); AMB 4-6.1, RAE 70.1 and AMB 1-2.1 (SEQ ID NO:111, SEQ ID NO:44 and SEQ ID NO:86); AMB 4-9.1, RAE 70.1 and AMB 1-2.1 (SEQ ID NO:110, SEQ ID NO:44 and SEQ ID NO:86); AMB 4-6.1, RAE 70.1, AMB 1-2.1 and AMB 3-5.1 (SEQ ID NO:111, SEQ ID NO:44, SEQ ID NO:86 and SEQ ID NO:102); AMB 4-9.1, RAE 70.1, AMB 1-2.1 and AMB 3-5.1 (SEQ ID NO:110, SEQ ID NO:44, SEQ ID NO:86 and SEQ ID NO:102); AMB 4-6.1, AMB 2-5.1, AMB 1-2.1 and AMB 3-5.1 (SEQ ID NO:111, SEQ ID NO:92, SEQ ID NO:86 and SEQ ID NO:102); AMB 4-9.1, AMB 2-5.1, AMB 1-2.1 and AMB 3-5.1 (SEQ ID NO:110, SEQ ID NO:92, SEQ ID NO:86 and SEQ ID NO:102); AMB 4-6.1, RAE 70.1, AMB 1-2.1 and AMB 3-4.1 (SEQ ID NO:111, SEQ ID NO:44, SEQ ID NO:86 and SEQ ID NO:103); AMB 4-9.1, RAE 70.1, AMB 1-2.1 and AMB 3-4.1 (SEQ ID NO:110, SEQ ID NO:44, SEQ ID NO:86 and SEQ ID NO:103); AMB 4-6.1, AMB 2-5.1, AMB 1-2.1 and AMB 3-4.1 (SEQ ID NO:111, SEQ ID NO:92, SEQ ID NO:86 and SEQ ID NO:103); AMB 4-9.1, AMB 2-5.1, AMB 1-2.1 and AMB 3-4.1 (SEQ ID NO:110, SEQ ID NO:92, SEQ ID NO:86 and SEQ ID NO:103); AMB 2-1.15 and AMB 4-3.15 (SEQ ID NO:101, and SEQ ID NO:116); AMB 1-2.15, AMB 2-1.15 and AMB 4-3.15 (SEQ ID NO:89, SEQ ID NO:101, and SEQ ID NO:116); and AMB 1-2.15, AMB 2-1.15, AMB 4-3.15 and AMB 3-4.15 (SEQ ID NO:89, SEQ ID NO:101, SEQ ID NO:116 and SEQ ID NO:107).

Particularly preferred compositions and preferred combinations of peptides for therapeutic administration include the following combinations: AMB 1-2.1 and AMB 4-9.1 (SEQ ID NO:86 and SEQ ID NO:110); AMB 1-2.1, Amb 2-38.1 and AMB 4-9.1 (SEQ ID NO:86, SEQ ID NO:141 and SEQ ID NO:110); AMB 1-2.1, Amb 2-38.1, AMB 4-9.1 and AMB 2-4.1 (SEQ ID NO:86, SEQ ID NO:141, SEQ ID NO:110 and SEQ ID NO:90); AMB 1-2.1, Amb 2-38.1, AMB 4-9.1, AMB 2-4.1 and AMB 3-5.1 (SEQ ID NO:86, SEQ ID NO:141, SEQ ID NO:110, SEQ ID NO:90, and SEQ ID NO:102); AMB 1-2.1, Amb 2-36.1 and AMB 4-9.1 (SEQ ID NO:86, SEQ ID NO:139 and SEQ ID NO:110); AMB 1-2.1, Amb 2-36.1, AMB 4-9.1 and AMB 2-4.1 (SEQ ID NO:86, SEQ ID NO:139, SEQ ID NO:110 and SEQ ID NO:90); and AMB 1-2.1, Amb 2-36.1, AMB 4-9.1, AMB 2-4.1 and AMB 3-5.1 (SEQ ID NO:86, SEQ ID NO:139, SEQ ID NO:110, SEQ ID NO:90, and SEQ ID NO:102).

The present invention also provides methods of detecting sensitivity in individuals to ragweed pollen allergens comprising combining a blood sample obtained from the individual with a peptide of the present invention, under conditions appropriate for binding of blood components with the peptide and determining the extent to which such binding occurs. The extent to which binding occurs is determined by assessing T cell function, T cell proliferation or a combination thereof.

It is also possible to modify the structure of a peptide of the invention for such purposes as increasing solubility, enhancing therapeutic or preventive efficacy, or stability (e.g., shelf life ex vivo, and resistance to proteolytic degradation in vivo.) A modified peptide can be produced in which the amino acid sequence has been altered, such as by amino acid substitution, deletion, or addition, to modify immunogenicity and/or reduce allergenicity, or to which a component has been added for the same purpose.

For example, a peptide can be modified so that it maintains the ability to induce T cell anergy and bind MHC proteins without the ability to induce a strong proliferative response or possibly, any proliferative response when administered in immunogenic form. In this instance, critical binding residues for the T cell receptor can be determined using known techniques (e.g., substitution of each residue such as, for example, with alanine and determination of the presence or absence of T cell reactivity). Those residues shown to be essential to interact with the T cell receptor can be modified by replacing the essential amino acid with another, preferably similar amino acid residue (a conservative substitution) whose presence is shown to enhance, diminish, but not eliminate, or not affect T cell reactivity. In addition, those amino acid residues which are not essential for T cell receptor interaction can be modified by being replaced by another amino acid whose incorporation may enhance, diminish or not affect T cell reactivity, but not eliminate binding to relevant MHC. Preferred amino acid substitutions for non-essential amino acids include, but are not limited to substitutions with alanine, gluatamic acid or a methyl amino acid.

Another example of a modification of peptides is substitution of cysteine residues preferably with serine, threonine, leucine or glutamic acid to minimize dimerization via disulfide linkages. As described in Example XI, a peptide from Region 2, RAE 70.1 (SEQ ID NO:44) was modified to minimize dimerization by substituting serine for the cysteine residue at amino acid position 212. In addition, as shown in Figure 23, peptide AMB 4-9.1 which contains an acid-sensitive aspartic acid-proline bond at amino acid residues 360-361, was modified to increase the stability of this peptide. For example, in peptide AMB 4-9.1DA, the proline at position 361 was substituted with alanine to remove the acid-sensitive bond.

In order to enhance stability and/or reactivity, peptides can also be modified to incorporate one or more polymorphisms in the amino acid sequence of a protein allergen resulting from natural allelic variation. Additionally, D-amino acids, non-natural amino acids or non-amino acid analogues can be substituted or added to produce a modified peptide within the scope of this invention. Furthermore, peptides can be modified using the polyethylene glycol (PEG) method of A. Sehon and co-workers (Wie et al., supra) to produce a peptide conjugated with PEG. In addition, PEG can be added during chemical synthesis of a peptide of the invention. Modifications of peptides or portions thereof can also include reduction/alkylation (Tarr in: Methods of Protein Microcharacterization, J.E. Silver ed. Humana Press, Clifton, NJ, pp. 155-194 (1986)); acylation (Tarr, supra); esterification (Tarr, supra); chemical coupling to an appropriate carrier (Mishell and Shiigi, eds, Selected Methods in Cellular Immunology, WH Freeman, San Francisco, CA (1980); U.S. Patent 4,939,239); or mild formalin treatment (Marsh International Archives of Allergy and Applied Immunology 41: 199-215 (1971)).

In another embodiment, peptides within an allergen group (e.g., Amb a I or Amb a II) can be modified to enhance T cell reactivity. Given the cross-reactivity within the Amb a I family and Amb a II, a peptide of one group allergen which may be less reactive than a peptide of another group allergen corresponding in amino acid position can have one or more amino acids substituted with one or more amino acids from the corresponding peptide. Additionally, peptides can be modified to incorporate a polymorphism in the amino acid sequence of a protein allergen resulting from natural allelic variation. Modification of peptides to include one or more of these polymorphisms may result in enhanced stability and/or reactivity.

To facilitate purification and potentially increase solubility of peptides of the invention, it is possible to add reporter group(s) to the peptide backbone. For example, poly-histidine can be added to a peptide to purify the peptide on immobilized metal ion affinity chromatography (Hochuli, E. et al., Bio/Technology, 6:1321-1325 (1988)). In addition, specific endoprotease cleavage sites can be introduced, if desired, between a reporter group and amino acid sequences of a peptide to facilitate isolation of peptides free of irrelevant sequences. In order to successfully desensitize an individual to a protein antigen, it may be necessary to increase the solubility of a peptide by adding functional groups to the peptide or by not including hydrophobic T cell epitopes or regions containing hydrophobic epitopes in the peptides. For example, in Region 3 of the Amb a I.1 protein, a selected peptide AMB 3-4.1 was modified to increase its solubility by the addition of three naturally occurring sequential residues found in the Amb a I.1 protein, "RHG", to the 5' end of the peptide. These residues are not necessary for T cell recognition and are also found in peptide AMB 3-5.1. Similarly, as described in Example XI, peptide RAE 70.1 was divided into two fragments and modified to increase solubility. As shown in Figure 25, a fragment of RAE 70.1 ("ORIGINAL" in Figure 25, amino acid residues 194-216, SEQ ID NO:125) was modified by substitution or addition of amino acids to increase the hydrophilicity and decrease the pI of the peptide to thereby increase the solubility. For example, in peptide Amb 2-22.1, isoleucine was substituted with glutamic acid to decrease the pI and avoid precipitation of the peptide from solution at a physiological pH. Similar substitutions and additions are shown in Figure 25.

To potentially aid proper antigen processing of T cell epitopes within a peptide, canonical protease sensitive sites can be recombinantly or synthetically engineered between regions, each comprising at least one T cell epitope. For example, charged amino acid pairs, such as KK or RR, can be introduced within a peptide or at the ends of a peptide during recombinant or synthetic construction of the peptide. (See Fig. 14, AMB 2-8.1, AMB 2-9.1, AMB 2-10.1, AMB 2-7.1 and AMB 2-11.1) The resulting peptide can be rendered sensitive to cathepsin and/or other trypsin-like enzymes cleavage to generate portions of the peptide containing one or more T cell epitopes. In addition, such charged amino acid residues can result in an increase in solubility of a peptide.

Site-directed mutagenesis of DNA encoding a peptide of the invention can be used to modify the structure of the peptide. Such methods may involve PCR with degenerate oligonucleotides (Ho et al., Gene, 77:51-59 (1989)) or total synthesis of mutated genes (Hostomsky, Z., et al., Biochem. Biophys. Res. Comm., 161:1056-1063 (1989)). To enhance bacterial expression, the aforementioned methods can be used in conjunction with other procedures to change the eucaryotic codons in DNA constructs encoding peptides of the invention to ones preferentially used in E. coli, yeast, mammalian cells or other eucaryotic cells.

The present invention a!so provides nucleic acids having sequences encoding proteins and peptides of the invention. Nucleic acid sequences used in any embodiment of this invention can be cDNA as described herein, or alternatively, can be any oligodeoxynucleotide sequence having all or a portion of a sequence represented herein, or their functional equivalents. Such oligodeoxynucleotide sequences can be produced chemically or mechanically, using known techniques. A functional equivalent of an oligonucleotide sequence is one which is 1) a sequence capable of hybridizing to a complementary oligonucleotide to which the sequence (or corresponding sequence portions) of SEQ ID NO: 1, 3, 5, 7, 9 and 11 or fragments thereof hybridizes, or 2) the sequence (or corresponding sequence portion) complementary SEQ ID NO: 1, 3, 5, 7, 9 and 11 and/or 3) a sequence which encodes a product (e.g., a polypeptide or peptide) having the same functional characteristics of the product encoded by the sequence (or corresponding sequence portion) of SEQ ID NO: 1, 3, 5, 7, 9 and 11. Whether a functional equivalent must meet one or more criteria will depend on its use (e.g., if it is to be used only as an oligoprobe, it need meet only the first or second criteria and if it is to be used to produce a peptide of the present invention, it need only meet the third criterion).

As described in the Examples which follow, Amb a I and Amb a II proteins have been recombinantly expressed in E. coli, purified and shown to have reduced binding to human allergic ragweed pollen IgE on Western blots. Overlapping peptides derived from the Amb a I.1 protein and various peptides derived from Amb a I.3 and Amb a I.2 were synthesized and used to identify regions of T cell reactivity within the protein. These regions of T cell reactivity were further defined by modifying selected Amb a I.1 peptides and determining T cell reactivity to these peptides.

This invention is further illustrated by the following non-limiting examples.

### Example I Native Ragweed Pollen Allergen Purification

What follows is a description of the work done to biochemically purify the allergens of Ambrosia artemisiifolia in their native form as primary antigens for human T-cell epitope mapping.

50 g of defatted short ragweed pollen (Greer Labs) was extracted in 500 ml .05 M Tris pH 7.95 containing protease inhibitors. The extract was then depigmented by batch absorption with Whatman DE-52 DEAE cellulose (150 g dry weight) in the presence of 0.2 M NaCl at 4°C. Unbound material was dialysed against .025 M Tris pH 7.95 with protease inhibitors. The depigmented sample was next applied to an 80 ml DEAE cellulose column (Whatman DE-52) equilibrated in .025 M Tris pH 7.95 containing protease inhibitors. Acidic proteins were eluted with .025 M Tris, 0.2 M NaCl pH 7.95 at 4°C with inhibitors.

In order to biochemically purify Amb a I, the acidic DEAE elution sample was fractionated by ammonium sulfate precipitation into 0-45% and 45-59% saturation samples (4°C). The Amb a I-enriched 45-59% pellet was applied at 0.5 ml/min (4°C) to a 500 ml Sephacryl S200 (Pharmacia) column in .05 M ammonium bicarbonate containing inhibitors. Purified Amb a I was recovered in the 38 kD region and dialysed against .04 M Tris pH 8.0. This sample was next applied to an 8 ml Mono Q HR 10/10 (Pharmacia) column in .04 M Tris pH 8.0 at 25°C. Elution was performed with .04 M Tris pH 8.0 containing .08M NaCl and the major peaks were analyzed as discussed below for confirmation and purity of Amb a I.

To biochemically purify Amb a II, the acidic DEAE elution sample was separated into an Amb a II-enriched fraction by ammonium sulfate precipitation at 0-45% saturation (4°C). The pellet was applied at 0.5 ml/min (4°C) to a 200 ml Sephadex G75 (Pharmacia) column in .05 M ammonium bicarbonate containing inhibitors. Purified Amb a II was recovered in the 38 kD region and dialysed against .04 M Tris pH 8.0 at 25°C to separate contaminating Amb a I from Amb a II. Elution was performed with .04 M Tris pH 8.0 containing .08M NaCl.

The major peaks were analyzed by IEF SDS-PAGE using a mixture of 4.5-5.3 and 3.5-10.0 ampholytes (Pharmacia) in a 7% acrylamide gel. Protein sequencing was also performed to confirm Amb a II.

### Example II Recombinant Ragweed Pollen Allergen Expression

What follows is a description of the work done to produce the allergens of Ambrosia artemisiifolia as recombinant proteins in E. coli.

Described and provided in USSN 07/529,951, filed May 29, 1990, (incorporated herein by reference) are full length cDNAs encoding Amb a I.1, Amb a I.2, Amb a I.3, Amb a I.4 and Amb a II. cDNA inserts encoding these five protein allergens were constructed in the vector pTrc99A (Amann, E., et al., Gene, 69:301 (1988)) which was kindly provided by Dr. Egon Amann (Behringwerke AG, Marburg, FRG). The nucleotide sequences and deduced amino acid sequences of the Amb a I family members are shown in the sequence listing as SEQ ID NO:1 and 2 (Amb a I.1), SEQ ID NO:3 and 4 (Amb a I.2), SEQ ID NO:5 and 6 (Amb a I.3), SEQ ID NO:7 and 8 (Amb a I.4), and SEQ ID NO:9 and 10 (Amb a II). The cDNAs encoding each allergen were cloned in frame with a polylinker encoding six sequential histidines, (CAC)₆, that had been inserted into the 5' end of the pTrc99A vector as a NcoI/EcoRI synthetic adapter (Maniatis T., et al. Molecular Cloning: A Laboratory Manual, Cold Spring Harbor Laboratory, Cold Spring Harbor, NY (1982). These cDNA inserts were then cloned in-frame into the appropriate pTrc99. To further enhance expression, a retroregular stem-loop sequence was placed at the 3' end in the untranslated region (Skoglund, C. M., et al. Gene 88:1 (1990)). The H₆ leader sequence allowed purification using QIAGEN NTA-Agarose (Diagen GmbH, Dusseldorf, FRG), a Ni²⁺ chelating support (Hochuli, E. et al., Biotechnology, 6:1321 (1988)). The vectors were transformed into the XL-1 Blue host bacteria. Expression of individual recombinant proteins was induced when cultures reached OD₆₀₀=0.6 to 0.7 by the addition of isopropyl-D-thiogalactopyranoside (IPTG) to 1 mM concentration to the culture medium. After 2 hours of further growth at 37°, the cells were pelleted. Recombinant proteins were then obtained by either of the following procedures. In one embodiment, the cells were resuspended in lysozyme containing phosphate buffer (0.4 mg/ml) and incubated for 30 minutes on ice. The cell suspension was frozen and quick thawed followed by sonication (Bond, J. G. et al., J. Immunol., 146:3380 (1991)). Insoluble recombinant protein was recovered by a low speed centrifugation and solubulized in 10 ml (per 1 liter growth) of buffer containing 8 M urea, 50 mM Tris, pH 8.0, 2 µg/ml leupeptin, 2 µg/ml pepstatin and 1 µg/ml soybean trypsin inhibitor. The urea solubilized preparation was subjected to a low speed centrifugation and the recombinant proteins in the supernatant isolated by metal ion chromatography (Hochui supra).

In another embodiment, the pelleted bacteria were resuspended in 6 M guanidine HCl, 100 mM 2-mercaptoethanol, 100 mM NaPO₄, 10 mM Tris pH 8.0. This suspension was subjected to centrifugation at 15,000 X g, and the supernatant removed, adjusted to pH 8.0 with 10 N NaOH, and applied to an NTA agarose column that had been equilibrated in 6 M guanidine HCl, 100 mM NaPO₄, 10 mM Tris pH 8.0. The column was washed in 6 M guanidine HCl, 100 mM NaPO₄, 10 mM Tris pH 8.0 until the OD₂₈₀ of the effluent reached background. The column buffer was then switched to 8 M urea, 100 mM NaPO₄, 10 mM Tris pH 8.0. After equilibration, a more stringent wash was performed in 8 M urea, 100 mM NaOAc, 10 mM Tris pH 6.3 until the OD₂₈₀ of the effluent reached background. Recombinant protein (as an H₆ fusion) was then eluted in 8 M urea, 100 mM NaOAc, 10 mM Tris pH 4.5 and collected in aliquots whose OD₂₈₀ profile was monitored. The protein peak was dialyzed 3 times into 500 volumes of PBS for human T-cells analysis. Yield ranged from 1-3 µg of recombinant protein per litre with purity of approximately 55% (as determined by densitometric scanning).

### Example III IgE Analysis With Purified Recombinant Ragweed Pollen Allergens

Both Western blotting and ELISA techniques were used to analyze the binding of IgE to purified recombinant Amb a I and Amb a II proteins. Additionally, ELISA was used for analysis of IgE reactivity to peptides derived from the Amb a I.1 protein.

### A. Western Blot Analysis

The antigens were loaded on the gels as follows: lane 1 SPE (Soluble Pollen Extract), 15 µg/lane; lane 2 rAmb a I.1 (recombinant Amb a I.1), 3 µg/lane; lane 3 rAmb a I.2, 4 µg/lane; lane 4 rAmb a I.3, 3 µg/lane; lane 5 rAmb a I.4, 3 µg/lane; and lane 6 rAmb a II.1, 4 µg/lane.

The gel electrophoresis and Western blot transfer procedures used were essentially as described elsewhere (Towbin, H., et al., Proc. Natl. Acad. Sci., USA, 76:4350 (1979)). Briefly, SDS-PAGE was performed in 10% acrylamide gels under reducing conditions (10mM dithiothreitol, at constant current). The transfer to nitrocellulose (0.1 µM, Schleicher & Schuell, Keene, NH) was performed in a Hoeffer apparatus according to the protocol of Towbin et al., supra. After transfer, the blots were rinsed in blot solution (25 mM Tris-HCl, pH 7.5, 0.17 M NaCl, and 0.05% Tween 20) and stained for 1 hour with 0.1% India ink. All subsequent incubations with antibodies and washes were performed in blot solution at room temperature. The first antibody incubations were performed overnight then rinsed and incubated with the appropriate biotinylated second antibody (Kirkegaard Perry Laboratories, Gaithersburg, MD). The final incubation was performed using ¹²⁵-I streptavidin (1 µCi/25 ml blot solution) for 1 hour followed by removal of unbound labeled material and autoradiography at -80^{o}C with an intensifying screen.

Fig. 1 shows the western blot IgE binding pattern of two ragweed allergic patient plasma samples (#475 and #143). These patterns are representative of the typical IgE reactivity to these proteins on a Western blot. Both patients show binding to the Amb a I.1 and Amb a I.3 gene products in the soluble pollen extract (lane 1, Fig. 1). The results demonstrate clear IgE binding reactivity in these patients to Amb a I.1, Amb a I.3, Amb a I.4. In contrast, Amb a I.2 and Amb a II.1 showed markedly reduced IgE binding.

### B. ELISA

Patient #143 IgE was also tested for binding to purified recombinant Amb a I and Amb a II proteins by ELISA. Fig. 2 shows the results of this analysis performed according to the following method.

Corning assay plates (#25882-96) were coated with each antigen listed in Fig. 2 at the following concentrations: Soluble Pollen Extract (SPE) 15 µg/ml; rAmb a I.1, 5 µg/ml; rAmb a I.2, 20 µg/ml; rAmb a I.3, 5 µg/ml; rAmb a I.4, 15 µg/ml; and rAmb a II, 20 µg/ml. 50 µls/well of the above antigens were added and coating was carried out overnight at 4° C. The coating antigens were removed and the wells were blocked with 0.5% gelatin in PBS, 200µl/well for 2 hours at room temperature. Patient #143 plasma was serially diluted with PBS-Tween 20 (PBS with 0.05% nonionic detergent Tween-20 Sigma, St. Louis MO) and 100µl/well was added and incubated overnight at 4°C (plasma dilutions are tested in duplicate). The second antibody (biotinylated goat anti-Human IgE, 1:1000, Kirkegaard & Perry Laboratories Inc. Gaithersburg, MD), was added at 100 µl/well for one hour at room temperature. This solution was removed and streptavidin-HRPO, 1:10000, (Southern Biotechnology Associates, Inc., Birmingham, AL) was then added at 100µl/well for one hour at room temperature (all wells are washed three times with PBS-Tween between each incubation step). TMB Membrane Peroxidates Substrate system (Kirkegaard & Perry Laboratories) was freshly mixed, and added at 100µl/well. The color was allowed to develop for 2-5 minutes. The reaction was stopped by the addition of 100µl/well of 1 M phosphoric acid. Plates were read on a Microplate EL 310 Autoreader (Biotek Instruments, Winooski, VT) with a 450nm filter. The absorbance levels of duplicate wells was averaged. The graphed results (log of the dilution vs absorbance) of the ELISA assays are shown in Fig. 2. The order of coating antigens listed vertically in these figures corresponds in order from left to right to the coating antigens listed for each histogram.

The results of the ELISA assay, Fig. 2, demonstrate a similar reactivity pattern as seen in the Western blot analysis (Fig. 1). The observation of much stronger binding to SPE is due to the fact that this antigen preparation is non-denatured. However, the recombinant Amb a proteins, because of their non-native bacterial origin behave as denatured antigens. The hierarchy of IgE binding in this patient to rAmb a proteins by ELISA shows that Amb a I.1 binds IgE more strongly than Amb a I.3 and Amb a I.4. The results also show that Amb a I.2 and Amb a II demonstrate markedly reduced binding to IgE.

As shown in Fig. 3, another ELISA assay was performed using a pool of human allergic sera. This assay demonstrates that biochemically purified (native) Amb a I and Amb a II proteins bind significantly more IgE than the respective recombinant proteins (rAmb a I.1 and rAmb a II). (The control in this experiment was plates coated without antigen and PBS).

### Example IV Tolerization of Mice with Recombinant Amb a I Proteins

Balb/c mice (H-2 d) were immunized in the hind foot pads and at the base of the tail with an emulsion of Complete Freunds Adjuvant (CFA) and 50µg/mouse Amb a I.1. Seven days later, the popliteal lymph nodes, superficial inguinal lymph nodes, and the periaortic lymph nodes were harvested and cultured (1 x 10⁵ lymph node cells + 2 x 10⁵ irradiated spleen feeders) with various challenge antigens in vitro. The in vitro antigens consisted of various doses of Amb a I.1, Amb a I.2, Amb a I.3, Amb a I.4, Amb a II, and a media control. Cultures were incubated for 3 days at 37°C in a CO₂ incubator. On day 3, each culture was pulsed with 1µCi ³H thymidine and on day 4 the cultures were harvested and proliferation was monitored by incorporation of ³H into DNA.

As seen in Fig. 4A, the mice that were immunized with 50µg Amb a I.1 + CFA have a good response to Amb a I.1, Amb a I.3, and Amb a I.4. The response to Amb a I.2 and Amb a II is also good but less than other antigens. It appears as though Amb a I.1 is very immunogenic in Balb/c mice and results in a response to each Amb a family member. When mice were immunized with PBS + CFA, there was no significant response to any of the Amb a family members (Fig. 4B).

An experiment was conducted to determine whether mice could be tolerized with Amb a I.1. The mice were tolerized with Amb a I.1 and pollen extract and then challenged with Amb a I.1 or pollen extract. The outline of this experiment #5 is shown below:
- Day 1:: Group (1): 6 Balb/c mice tolerized with 300µg Amb a I.1 + Incomplete Freunds Adjuvant (IFA) intraperitoneally (i.p.)
Group (2): 6 Balb/c mice tolerized with 300µg Pollen Extract + IFA i.p.
Group (3): 6 Balb/c mice exposed to PBS + IFA i.p.
- Day 15: Challenge 3 mice from each group with 50µg Amb a I.1 + CFA subcutaneously (s.c.)
Challenge the remaining 3 mice from each group with 50µg Pollen extract + CFA s.c.
- Day 23: Harvest Lymph Nodes (Analyze each mouse separately)
Test Lymph Node Response on Amb a I.1, I.2, I.3, I.4, II.1, Pollen Extract, Concanavalin A (Con A), and media.

The animals were sacrificed by cervical dislocation on day 23 and the popliteal lymph nodes, superficial inguinal lymph nodes, and the periaortic lymph nodes were removed and placed in rinsing buffer (cold RPMI 1640 containing 1% FCS). The nodes were rinsed with rinsing buffer and forced through a fine stainless steel mesh, using a glass pestal to suspend them in rinsing buffer. The suspended cells were rinsed two times by centrifugation at 1000 rpm for 10 minutes and resuspended in rinsing buffer. An aliquot from each sample was taken in order to do a cell count. The cells (4 x 10⁶/ml) were incubated in culture media (RPMI 1640 media containing 10% FCS, 2mM L-glutamine, 50U/ml penicillin, 50 µg/ml streptomycin and 5 x 10⁻⁵ M 2-mercaptoethanol) and test antigens at various concentrations. The triplicate 0.1 ml cultures (U-bottom 96 well plates (Costar)) were incubated at 37^{o}C and at 5% CO₂. After 24 hours, 50µl of media from each well was placed in separate flat bottom 96 well plates (Costar) and frozen overnight at -20°C to eliminate carryover of live cells. The supernatants were tested after thawing for their ability to support the growth of CTLL3, an IL-2 dependent T cell clone. CTLL3 in log phase growth were rinsed 3 times by centrifugation at 1000 rpm for 10 minutes. CTLL3's were added to the warmed culture supernatants (5 x 10³ cells/well) and the IL-2 assay was incubated at 37°C and 5% CO₂. After 24 hours, 1 µCi/well ³H thymidine was added in 50 µl/well and the CTLL3 cells were incubated for an additional 4-6 hours. Following the pulse with ³H, the plates were harvested on a Tom Tek 96 well cell harvester. The ³H incorporation in each well was counted by a Betaplate Model 1205 scintillation counter. Background counts were not subtracted.

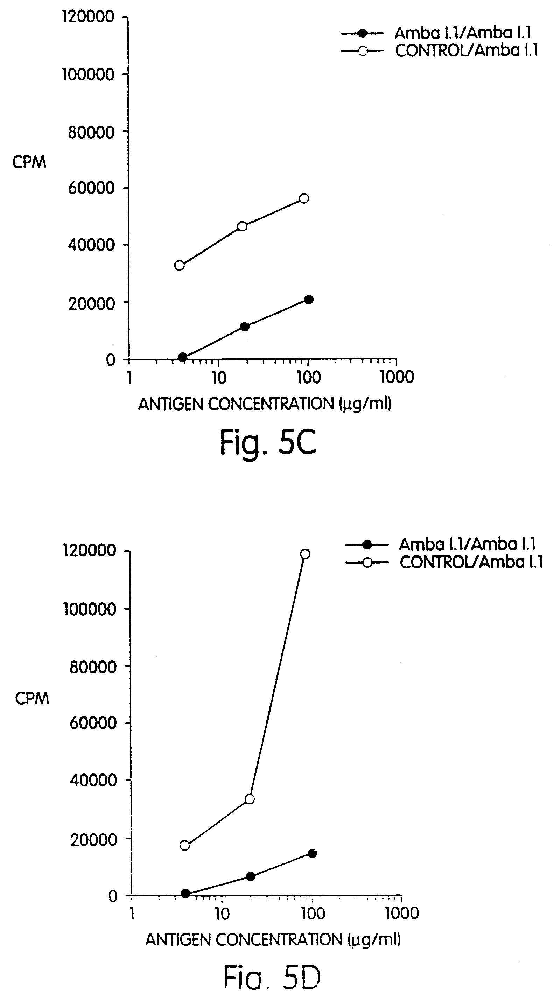

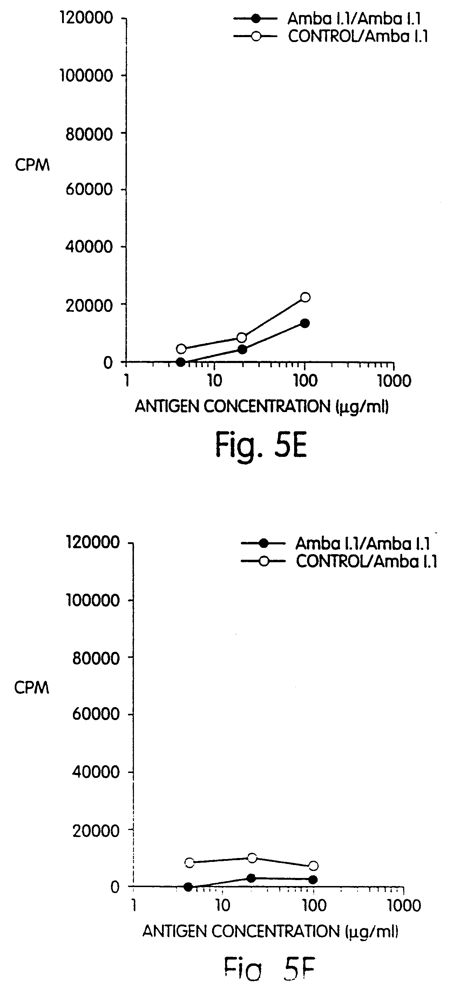

Fig. 5 shows the lymph node responses to Amb a I.1, Amb a I.2, Amb a I.3, Amb a I.4, and Amb a II in the mice that were tolerized with Amb a I.1 or Phosphate Buffered Saline (PBS) and challenged with Amb a I.1. Fig. 5A, shows that the mice that were tolerized with Amb a I.1 have a lower response to Amb a I.1 than the mice that were tolerized with PBS. The same appears to be true in the response to Amb a I.2, Amb a 1.3, and Amb a I.4 (Figs. 5B, 5C, 5D). In Figs. 5E and 5F, it appears as though there is no significant difference in the response to Amb a II or pollen extract between the tolerized group and the non-tolerized group. Figs. 6A-6F show the lymph node responses to the various proteins in the mice that were exposed to pollen extract or PBS and challenged with pollen extract. Table 1 summarizes the results of these experiments.

**TABLE 1**

| | Amba I.1 | Amba I.2 | Amba I.3 | Amba I.4 | Amba II.1 | Pollen |
|---|---|---|---|---|---|---|
| Tolerize/Challenge | | | | | | |
| Control/Amba I.1 | + | + | + | + | - | - |
| Amba I.1/Amba I.1 | - | - | - | - | - | - |
| Control/Pollen | + | + | +/- | +/- | - | + |
| Pollen/Pollen | - | - | - | - | - | - |
| (-) indicates a diminished response as compared to control | | | | | | |

### Example V Synthesis of Overlapping Peptides

Amb a I.1 overlapping peptides as shown in Fig. 7 were synthesized using standard Fmoc/tBoc synthetic chemistry and purified by dialysis or Reverse Phase HPLC. In addition, various peptides derived from Amb a I.2 and Amb a I.3 were synthesized. The amino acid residues of the synthesized peptides are in brackets by the peptide name and the amino acid sequence (in single letter code) is next to the peptide name. The peptide names are consistent throughout the Figures. In the design of the overlapping peptides, the relationship of the Amb a I family members at the level of T cell cross-reactivity as determined in Example VIII was considered. As shown in Table IV, the Amb a I protein allergens share a high degree of cross-reactivity. In addition, Amb a I.1 and Amb a II were found to have 55.2% cross-reactivity. Given the high degree of cross-reactivity it was expected that "shared" T cell epitopes exist among Groups I and II. Thus, the amino acid sequences of the Amb a I family members and Amb a II were examined to identify conserved and variable regions. It was expected that conserved regions within the Amb a I family members and Amb a II would contain "shared" T cell epitopes.

### Example VI T Cell Responses to Ragweed Peptides

Peripheral blood mononuclear cells (PBMC) were purified by Sepracell-MN or lymphocyte separation medium (LSM) centrifugation of 60 ml of heparinized blood from ragweed-allergic patients who exhibited clinical symptoms of seasonal rhinitis and were skin prick test positive for ragweed. T cell lines were established from these cells by stimulation of 1-2 x 10⁶ PBMC/ml in RPMI-1640 containing 5% human AB serum (complete medium) with recombinant Amb a I.1 at 20-30 µg/ml for 5-6 days at 37°C in a humidified CO₂ incubator. Viable cells were purified by LSM centrifugation and cultured in complete medium supplemented with 5 units recombinant human IL-2/ml and 5 units recombinant human IL-4/ml for up to three weeks until the cells no longer responded to lymphokines and were considered "rested". The ability of the T cells to proliferate to Amb a I.1, Amb a I.2 and Amb a I.3 sequence-derived synthetic peptides was then assessed.

For assay, 2 x 10⁴ rested cells were restimulated in the presence of 2 x 10⁴ autologous Epstein-Barr virus (EBV)-transformed B cells (gamma-irradiated with 25,000 RADS) or 5 x 10⁴ autologous PBMC (3,500 RADS) with various concentrations of Amb a I.1 synthetic peptides in a volume of 200 µl complete medium in duplicate or triplicate wells in 96-well round bottom plates for 3 days. Each well then received 1 µCi tritiated thymidine for 16-20 hours. The counts incorporated were collected onto glass fiber filter mats and processed for liquid scintillation counting. Table II shows the results of a representative assay. The maximum response in a titration of each peptide is expressed as the S.I. or stimulation index. The S.I. is the CPM incorporated by cells in response to peptide divided by the CPM incorporated by cells in medium only. An S.I. value greater than the background level is considered "positive" and indicates that the peptide contains a T cell epitope. However, only individual S.I. values above 2.0 (a response two-fold or greater over background) were used in calculating mean stimulation indices for each peptide for the group of patients tested. The results shown in Table II demonstrate that this patient (#466) responds very well to peptides RAE 7.D, RAE 69.1, RAE 64.1, and RAE 29.1. This indicates that these peptides contain Amb a I.1 T cell epitopes recognized by T cells from this particular allergic patient.

**TABLE II**

| Antigen | Concentration (µg/ml) | CPM | S.I. |
|---|---|---|---|
| Medium | -- | 1677 | -- |
| Amb a I.1 | 2 | 12113 | |
| | 10 | 25047 | |
| | 50 | 45710 | 27.3 |
| RAE 22.E | 5 | 3198 | |
| | 50 | 3581 | 2.1 |
| RAE 7.D | 5 | 3948 | |
| | 50 | 8572 | 5.1 |
| RAE 45.15 | 5 | 1160 | |
| | 50 | 1370 | 0.8 |
| RAE 69.1 | 5 | 6800 | |
| | 50 | 11464 | 6.8 |
| RAE 70.1 | 5 | 4116 | 2.5 |
| | 50 | 3888 | |
| RAE 65.1 | 5 | 1236 | |
| | 50 | 1784 | 1.1 |
| RAE 64.1 | 5 | 12828 | 7.7 |
| | 50 | 2259 | |
| RAE 29.1 | 5 | 6998 | |
| | 50 | 13078 | 7.8 |

The above procedure was followed with a number of other patients. Individual patient results were used in calculating the mean S.I. for each peptide if the patient responded to the Amb a I.1 protein at an S.I. of 2.0 or greater and the patient responded to at least one peptide derived from Amb a I.1 at an S.I. of 2.0 or greater. A summary of positive experiments from 39 patients is shown in Fig. 8. The bar represents the cumulative rank of the peptide response in the 39 patients. To determine the cumulative rank, the 5 peptides with the highest S.I. in each patient were determined and assigned a numerical rank in descending order with 5 representing the strongest response. The ranks for each peptide were then summed in the 39 patients to determine the cumulative rank for the peptide. The number above each bar is the mean S.I. of the positive responses (S.I. of 2.0 or greater) from the group of patients to that peptide. In parentheses above each bar is the positivity index (P.I). The P.I. for each peptide is determined by multiplying the mean S.I. by the percent of patients who responded to that peptide. The P.I. therefore represents both the strength of the response (S.I.) and the frequency of a response to a peptide in the group of patients tested. For example, peptide RAE 69.1 had the highest cumulative rank so it was the best peptide response in the overall population of 39 even through it did not have the highest mean S.I. Similarly, RAE 70.1 had the highest mean S.I but not the best cumulative rank or P.I. Thus, the response to RAE 70.1 was strong when it occurred but it did not occur as frequently in the population as the response to other peptides. The peptide with the highest P.I., RAE 29.1, also had a strong S.I. and the second highest cumulative rank. The response to this peptide was therefore generally strong and relatively frequent in this population.

### Example VII T Cell Epitope Fine Map Studies with Amb a I.1

Based on the above analysis 4 major areas of T cell reactivity within Amb a I.1 were identified. (Regions 1, 2, 3 and 4). All 39 patients responded to Amb a I.1 and a peptide from at least one of these regions: Region 1, amino acids residues 48-107; Region 2, amino acid residues 171-216; Region 3, amino acid residues 278-322; and Region 4, amino acid residues 331-377. Based in part on the T cell reactivity shown in Fig. 8, Amb a I.1 peptides were selected and modified by addition or deletion of amino acid residues at either the 5' or 3' end of the peptide. This set of peptides is shown in Fig. 9. T cell studies similar to those of Example VI were performed using these selected peptides to more precisely define the major areas of T cell reactivity within Regions 1-4 of the Amb a I.1 protein. For example, PBMC from a single ragweed-allergic patient were isolated as described in Example VI and were stimulated with 20 µg/ml of recombinant Amb a I.1 as described above. The results of proliferation assays with this one patient to selected peptides using irradiated (24,000 RADS) autologous EBV B cells as antigen presenting cells is shown in Table III. The data indicates that T cells from this patient respond well to RAE 7.D, RAE 70.1, RAE 40.1-4, and RAE 28.1-2.

**TABLE III**

| Antigen | Concentration (µg/ml) | CPM | S.I. |
|---|---|---|---|
| Medium | -- | 2762 | -- |
| PHA | 1 | 97864 | 35.4 |
| Amb a I.1 | 2 | 28025 | |
| | 10 | 56172 | |
| | 50 | 86598 | 31.4 |
| RAE 22.E | 5 | 3318 | 1.2 |
| | 50 | 784 | |
| RAE 7.D | 5 | 53292 | |
| | 50 | 60943 | 22.1 |
| RAE 40.1-4 | 5 | 4504 | |
| | 50 | 13549 | 4.9 |
| RAE 69.1 | 5 | 5213 | |
| | 50 | 5981 | 2.2 |
| RAE 70.1 | 5 | 14223 | 5.1 |
| | 50 | 4729 | |
| RAE 64.1 | 5 | 3418 | 1.2 |
| | 50 | 2538 | |
| RAE 28.1-2 | 5 | 7542 | |
| | 50 | 24208 | 8.8 |

The above procedure was followed with a number of other patients and yielded 47 positive experiments. A summary of the results for Region 1 peptides of the Amb a I.1 protein is shown in Fig. 10, Region 2 peptides in Fig. 11, Region 3 peptides in Fig. 12, and Region 4 peptides in Fig. 13. As described in Example VI, the bar shows the cumulative rank. In those experiments the 3 best peptide responses were ranked. The number above each bar represents the mean S.I. for the peptide and the number in parentheses the P.I. for the peptide. In these experiments an individual S.I. of 2.5 or greater was used in calculating the mean S.I.

Fig. 10 indicates that the major area of T cell reactivity within Region 1 of the Amb a I.1 protein is represented by peptides RAE 6.D, RAE 7.D, RAE 40.1, RAE 40.1-4, and RAE 40.1-5. Thus, a preferred area of major T cell reactivity within Region 1 comprises amino acid residues 57-101. Fig. 11 indicates that there is a broad area of weak T cell reactivity in Region 2 of the Amb a I.1 protein relative to Region 1. A preferred area of major T cell reactivity within Region 2 thus comprises amino acid residues 182-216. Fig. 12 shows that the most frequent and dominant response within Region 3 of the Amb a I.3 protein is to peptide RAE 64.1. However, another area of T cell reactivity is represented by peptide RAE 66.1. A preferred area of major T cell reactivity within Region 3 comprises amino acid residues 280-322. Fig. 13 indicates chat the major area of T cell reactivity in Region 4 is represented by peptides RAE 28.1-2, RAE 28.1-1, and RAE 28.1. From this analysis, a preferred area of major T cell reactivity within Region 4 comprises amino acid residues 342-377.

To further validate and define peptides derived from the Amb a I.1 protein comprising T cell epitopes, selected peptides from Regions 1-4 were further modified by addition or deletion of amino acid residues as described above. These selected modified peptides are shown in Fig. 14. To determine the T cell reactivity of these peptides, PBMC were stimulated with recombinant Amb a I.1 and assayed with the selected peptides as described in Example VI, except in some cases autologous PBMC (irradiated 3,500 RADS) were used as antigen presenting cells. The assay was followed with a number of patients and resulted in 23 positive experiments. In these assays an individual S.I. of 2.0 was used in calculating the mean stimulation index. In Figs. 15-18, the bar represents the cumulative rank of each peptide. The best 3 peptide responses for each patient were ranked as described above. The mean S.I. for each peptide and percent positive are found above the bar.

### Example VIII Amb a I.1 Specific T Cell Cross-Reactivity To Other Amb a I Family Members and Amb a II

To determine the relationship of the Amb a I family members (i.e., Amb a I.1, I.2, I.3, and I.4) at the level of T cell reactivity, PBMC were stimulated with recombinant Amb a I.1 (r Amb a I.1) as described in Example VI. For assay, 2 x 10⁴ rested cells were restimulated in the presence of an equal number of autologous EBV-transformed B cells (irradiated with 25,000 RADS) with various concentrations (0-100 µg/ml) of recombinant Amb a I.1, I.2, I.3, I.4, Amb a II, or ragweed pollen extract in a volume of 200 ml complete medium in duplicate or triplicate wells in 96-well round bottom plates for 3 days. Each well then received 1 µCi tritiated thymidine for 16-20 hours. The counts incorporated were assessed and analyzed as described in Example VI. Table IV shows the S.I., P.I., percent of cultures positive for the assay antigen, and number of cultures analyzed. For these assays an individual S.I. greater or equal to 2.0 was used in calculating the mean S.I. Amb a I.1 stimulated cells respond less well and less frequently to the least homologous family members I.3 and I.2. However, this level of cross-reactivity, 68.8% and 60% respectively, is still considered high. The lowest level of Amb a I.1 stimulated T cell cross-reactivity is noted to the least homologous allergen, Amb a II. In addition, Amb a I.1 reactive T cells are found at a high percentage from ragweed pollen extract stimulated cultures, again demonstrating the importance of the Amb a I.1 allergen.

**TABLE IV**

| Assay Antigen | S.I. | Recombinant Amb a I.1 Specific Lines | | |
|---|---|---|---|---|
| | | % positive | n | P.I. |
| Pollen Extract | 13.5 | 87.8% | 41 | 1185 |
| r Amb a I.1 | 27.5 | 95.1% | 41 | 2616 |
| r Amb a I.2 | 5.0 | 60% | 5 | 300 |
| r Amb a I.3 | 9.3 | 68.8% | 16 | 639 |
| r Amb a I.4 | 8.5 | 100% | 11 | 850 |
| r Amb a II | 12.8 | 55.2% | 29 | 70 |

### Example IX T Cell Cross-Reactivity of Amb a I.1 or Amb a I.3 Stimulated Cells to Amb a I.1 or Amb a I.3 Peptides

Since a high degree of T cell cross-reactivity could be demonstrated between Amb a I.1 and the most highly sequence divergent family member Amb a I.3, the ability of Amb a I.1 stimulated cells to recognize both Amb a I.1 peptides and homologous Amb a I.3 peptides was assessed. In a similar fashion, the ability of Amb a I.3 stimulated cells to recognize both Amb a I.3 peptides and homologous Amb a I.1 peptides was determined. The peptides used in this study are lasted below. The sequences for the Amb a I.1 homologous Amb a I.3 peptides were based on an alignment of the Amb a I.3 sequence to the Amb a I.1 sequence.

### Amb a I.1 peptides

- AMB 1-2.1: KGTVGGKDGDIYTVTSELDDDVAN
- AMB 1-4.1: AENRKALADCAQGFGKGTVGGKDGD
- AMB 2-1.1: GPAAPRAGSDGDAISISGSSQ
- AMB 3-1.1: GSYAIGGSASPTILSQGNRFCAPDERSK
- AMB 3-4.1: FFQVVNNNYDKWGSYAIGGSASPT
- AMB 4-3.1: VLENGAIFVASGVDPVLTPEQSAGMIP

### Amb a I.3 peptides

- AMB 1-2.15: KGTYGGKWGDVYTVSNLDDDVAN
- AMB 1-4.15: ENNRQALADCAQGFAKGTYGGKWGD
- AMB 2-1.15: GPPILRQASDGDTINVAGSSQ
- AMB 3-1.15: GTYAIGGSSAPTILCQGNRFLAPDDQIK
- AMB 3-4.15: FFQVVNNNYDRWGTYAIGGSSAPT
- AMB 4-3.15: LLENGAIFVTSGSDPVLTPVQSAGMIP

PBMC from ragweed allergic patients were stimulated with 20 µg/ml recombinant Amb a I.1 protein as described in Example VI with the addition of cultures of PBMC which were stimulated with 20 µg/ml recombinant Amb a I.3 protein. Assays were performed as described in Example VI except the homologous Amb a I.3 peptides were also tested at various doses. In these experiments an individual S.I of 2.0 or greater was used in calculating the mean S.I. Fig. 19 shows the results from a set of 9 matched patients stimulated with either recombinant Amb a I.1 protein or recombinant Amb a I.3 protein and tested on the set of Amb a I.1 peptides described above. Each bar represents the P.I. The dark bar represents cells stimulated with recombinant Amb a I.1 protein and tested with Amb a I.1 peptides, whereas, the stippled bar represents cells stimulated with recombinant Amb a I.3 protein and tested with Amb a I.1 peptides. With two exceptions, the results directly parallel each other, indicating that cells stimulated with recombinant Amb a I.3 proteins recognize Amb a I.1 derived peptides comprising at least one T cell epitope. One exception is that cells primed with Amb a I.3 protein recognize the RAE 7.D peptide poorly compared to recombinant Amb a I.1 stimulated cells. In addition, cells stimulated with recombinant Amb a I.3 protein gave stronger responses overall to Amb a I.1 derived peptides from Region 4 than the recombinant Amb a I.1 stimulated cells, particularly peptide RAE 28.1-2.

Cells were also stimulated with either recombinant Amb a I.1 or recombinant Amb a I.3 and tested for reactivity with Amb a I.3 derived peptides (Fig. 20). The results indicate that cells stimulated with recombinant Amb a I.1 protein recognize the Amb a I.3 derived peptides in a similar pattern to that of cells stimulated with recombinant Amb a I.3 protein. In Fig. 20 the bars represent the P.I. and the number above the bar the mean S.I. in the nine patients. The ".15" designation following the peptide name indicates that the peptide is derived from the Amb a I.3 protein. The data suggests a high degree of cross-reactivity at the T cell level between the Amb a I.1 protein and the Amb a I.3 protein.

### Example X IgE Analysis with Peptides Derived From Amb a I.1

To analyze IgE reactivity of peptides derived from the Amb a I.1 protein, a direct binding ELISA was performed according to the procedure described in Example III. The source of IgE for this analysis was a pool of ragweed allergic patient plasma from 38 ragweed skin test positive patients. The ELISA protocol was the same as Example III except that the antigen coating with peptides and proteins was a concentration of 10 µg/ml at 100 µls/well. Fig. 21 shows the graphed results of this assay demonstrating strong reactivity to both SPE and biochemically purified Amb a I.1. By this assay there is no detectable binding to any of the peptides. A set of these assays was also run with rabbit and mouse antisera to demonstrate that the coating of the peptides onto these plates was successful.

### Example XI Further T Cell Epitope Fine Map Studies With Amb a I.1

To further define the regions of Amb a I.1 specific T cell reactivity, selected peptides from Figure 14 were analyzed. Amb a I.1 specific T cells lines were derived from 28 ragweed allergic patients as described in Example VI. These lines were assessed for their ability to proliferate in response to the peptide in the presence of autologous EBV transformed antigen presenting cells by the uptake of tritiated thymidine as described previously. Several lysine substituted peptides derived from the peptide RAE 70.1 sequence were designed to increase the solubility of the peptide from Region 2. These peptides shown in Figure 22 were tested against non-substituted controls to determine if the modification resulted in a change in T cell reactivity. In addition, truncations of peptide AMB 4-6.1 (amino acid residues 347-377) were tested to further refine the relative T cell reactivity in that region.

Figure 22 shows the responses of the 28 T cell lines to these peptides as analyzed by the positivity index, the mean stimulation index and the percent of positive responses. The positivity index, as defined previously, is the mean stimulation index multiplied by the percent of patients responding to a peptide. Responses to peptides were considered positive if they were greater or equal to 2 fold over background. Figure 22 demonstrates that certain lysine substituted peptides in the 182-216 sequence resulted in greater T cell responses, indicating that not all substitutions are recognized equally by a given T cell line. In addition, the T cell responses to the lysine modified peptides having a substitution of cysteine at position 212 with serine reflect the responses of T cell lines tested with peptides without the lysine substitutions. Thus, the decrease in response to AMB 2-10.1, with a substitution of leucine for cysteine at 212, reflects the decrease in the percentage of patients responding to this peptide relative to peptides AMB 2-9.1 and AMB 2-11.1 which contain serine and glutamic acid substitutions, respectively, at position 212.

In addition, the truncation peptides derived from peptide AMB 4-6.1 showed indistinguishable responses based on this analysis. Thus, the shortest sequence, 345-370 was chosen for further analysis. Substitutions at positions 360 and 361 were made to increase the stability of the AMB 4-9.1 peptide which contained an acid-sensitive aspartic acid-proline bond. As shown in Figure 23, three peptides were synthesized in which the proline at position 361 was substituted with either alanine, serine or glycine (AMB 4-9.1DA (SEQ ID NO:147), AMB 4-9.1DS (SEQ ID NO:148), and AMB 4-9.1DG (SEQ ID NO:149), respectively). Similarly, five peptides were synthesized in which the aspartic acid at position 360 was substituted with the following: glutamic acid (AMB 4-9.1EP, SEQ ID NO:142); asparagine (AMB 4-9.1NP, SEQ ID NO:143); alanine (AMB 4-9.1AP, SEQ ID NO:144); serine (AMB 4-9.1SP, SEQ ID NO:145); and glutamine (AMB 4-9.1QP, SEQ ID NO:146). This group of modified peptides were tested to determine whether a change in T cell reactivity occurred. 28 Amb a I.1 specific T cells lines were analyzed by the relative strength of the response to the modified peptides in a given T cell line compared to the overall response to all peptides from that line. Figure 23 shows the ranked sum of the strongest three peptide responses in the 28 T cell lines. The ranked sum was determined as described previously with the strongest response to a peptide given a value of 3, the second strongest a 2, and the third strongest a value of 1. The values from all 28 lines were then added to obtain the ranked sum. Figure 23 indicates that the modified peptide AMB 4-9.1DA elicits T cell responses similar to those of the native sequence AMB 4-9.1. In contrast, other substitutions, while eliciting strong responses do not rank as highly in this analysis.

Based in part on the above described analyses, five peptides were selected for further study (AMB 1-2.1, AMB 2-6.1, AMB 2-9.1, AMB 3-5.1 and AMB 4-9.1). An additional peptide, RA-02.1 (SEQ ID NO:150) was also synthesized and tested to confirm that the serine substitution at position 212 was recognized by Amb a I.1 specific T cell lines. The T cell reactivity of these peptides in 32 different Amb a I.1 specific T cell lines was determined as described previously. Figure 24 demonstrates the relative positivity indices, percent of positive responses and mean stimulation indices of these six peptides compared to the overall response to Amb a I.1. This figure shows that peptide AMB 4-9.1 elicits the strongest response in a high percentage of patients.

In contrast to previous data, the response to the 182-216 peptide (AMB 2-6.1) and substituted variants within this sequence (RA-02.1) gave weaker stimulation indices. This could reflect a potential toxic effect of the peptides in the T cell assays. To investigate this possibility, an assay was conducted to determine the ability of the peptides to inhibit T cell proliferation. Inhibition was defined as proliferation of a T cell line in response to the peptide which was less than half the proliferation of media control T cell lines plus autologous EBV transformed antigen presenting cells. As seen in Table V, the percent of patients in which inhibition of T cell proliferation is seen is far greater in cultures where the AMB 2-6.1, RA-02.1, and AMB 2-9.1 peptides are present as compared to AMB 1-2.1 or AMB 4-9.1. This data suggests that AMB 2-6.1, RA-02.1 and AMB 2-9.1 may be inhibitory due to toxicity in in vitro T cell line culture. This is further supported by the finding that these peptides also inhibit a T cell line from patient 956.2 which is specific for an irrelevant antigen, native Fel d I, as shown in Figure 26.

**TABLE V**

| ASSESSMENT OF PEPTIDE TOXICITY IN Amb a I.1 PRIMED SECONDARY T CELL CULTURE | | | | |
|---|---|---|---|---|
| peptide | #/(%)patients | low dose only | high dose only | both |
| AMB 1-2.1 | 3/46 (7) | 2/3 | 1/3 | 0 |
| AMB 2-6.1 | 14/44 (32) | 0 | 11/14 | 3/14 |
| RA-02.1 | 19/45 (41) | 0 | 15/19 | 4/19 |
| AMB 2-9.1 | 23/38 (61) | 0 | 14/23 | 9/23 |
| AMB 4-9.1 | 1/46 (2) | 0 | 0 | 1/1 |
| AMB 3-5.1 | 19/46(41) | 0 | 17/19 | 2/19 |
| Legend: Toxicity defined as proliferation, as assessed by incorporation of tritiated thymidine, which is less than half the proliferation of media control of an Amb a I.1 stimulated T cell line plus APC. Low dose = 5 µg/ml peptide in secondary assay, high dose = 50 µg/ml peptide. | | | | |

A peptide from Region 2, RAE 70.1 (SEQ ID NO:44) was modified to minimize dimerization via disulfide linkages by substituting serine for cysteine at position 212. In addtion, peptide RAE 70.1 was divided into two fragments and modified to increase solubility and to further define the residues necessary for T cell reactivity. As shown in Figure 25, a fragment of RAE 70.1 (amino acid residues 194-216) was modified by substitution or addition of amino acids which would increase the hydrophilicity and decrease the pI of the peptide to thereby increase solubility.

Peptides Amb 2-18.1 (SEQ ID NO:126), Amb2-19.1 (SEQ ID NO:127), Amb2-20.1 (SEQ ID NO:128) and Amb2-21.1 (SEQ ID NO:129) correspond to different lengths of the original peptide which were synthesized (including the serine substitution at position 212) and analyzed to determine solubility. It was found that peptide Amb2-19.1 (residues 200-217) was most soluble. Thus, substitutions and/or amino acid additions to this peptide were made in order to further increase the solubility while maintaining T cell reactivity. For example, in peptides Amb2-22.1 (SEQ ID NO:130) and Amb2-23.1 (SEQ ID NO:131) isoleucine at position 201 was substituted with glutamic acid (Amb2-22.1) or lysine (Amb2-23.1) to lower the pI of the resulting peptide and avoid precipitation of the peptide at physiological pH. Similarly, the following peptides were also synthesized with various substitutions or additions designed to decrease the pI and increase the hydrophilicity of the peptide, each is shown in Figure 25: Amb2-26.1 (SEQ ID NO:132); Amb2-28.1 (SEQ ID NO:133); Amb2-30.1 (SEQ ID NO:134); Amb2-32.1 (SEQ ID NO:135); Amb2-33.1 (SEQ ID NO:136); Amb2-34.1 (SEQ ID NO:137); Amb2-35.1 (SEQ ID NO:138); Amb2-36.1 (SEQ ID NO:139); Amb2-37.1 (SEQ ID NO:140); and Amb2-38.1 (SEQ ID NO:141).

Figures 27 and 28 show representative examples of proliferation of two individual Amb a I.1 specific T cell lines to peptides selected from those shown in Figure 25. Assays were performed as described previously. Both Figure 27 and Figure 28 indicate a hierarchy of responses to these peptides. Patient 119 shown in Figure 27, has a hierarchy of response from strongest to weakest as follows: AMB2-23.1>AMB2-22.1>AMB2-30.1>AMB2-26.1>AMB2-33.1>AMB2 -32.1>AMB2-18.1>AMB2-19.1. Peptides AMB2-34.1, AMB2-35.1, and AMB 2-9.1 did not elicit significant T cell proliferation in this patient. Patient 1199 shown in Figure 28 demonstrated strong responses to AMB 2-26.1>AMB 2-22.1>AMB 2-33.1. T cells from this patient showed less than 2 fold over background responses to all other peptides. These responses are not significantly different from the media control of the T cell line plus autologous EBV antigen presenting cells.

To more closely examine the T cell epitopes within the above set of peptides to which Amb a I.1 specific T cell lines respond, a T cell clone was generated by limiting dilution from an Amb a I.1 specific T cell line stimulated with AMB 2-10.1. Briefly, an Amb a I.1 specific T cell line from patient 776 was shown to respond to AMB 2-10.1 in a proliferation assay as described previously. To generate a AMB 2-10.1 specific T cell clone, Amb a I.1 specific T cells were plated at 0.3 cells/well in a V-bottom 96 well plate with 20,000 irradiated autologous EBV transformed antigen presenting cells, AMB 2-10.1 at 40 µg/ml, leukoagglutin at 1 mg/ml, and recombinant human IL-2 and IL-4 at 10 units/ml in a total volume of 100µl/well. Plating the T cells at 0.3 cells/well insures not more than 1 T cell/well can potentially proliferate to the peptide with the progeny of that T cell representing a clonal population. After five days, wells received an additional 15 units/ml of recombinant human IL-2 and IL-4 to expand peptide-specific T cells. This addition of IL-2 and IL-4 was repeated again every three days for the duration of the culture period. Twelve days after the initiation of culture, T cells in the wells were restimulated with 20,000 irradiated EBV antigen presenting cells and 40 µg/ml AMB 2-10.1. This was repeated again 20 days after the initial stimulation. T cells from wells which showed signs of growth were separated from cell debris by density centrifugation as described previously in the generation of T cell lines. The T cell clone was then expanded with additional IL-2 and IL-4 at 10 units/ml until there were significant numbers to assay for proliferation.

Assessment for T cell proliferation to selected peptides from those shown in Figure 25 was, performed as described previously for T cell lines. The results of a representative assay using this AMB 2-10.1 specific T cell clone are shown in Figure 29. The data indicates that the T cell epitope recognized by this clone is contained by all the peptides except AMB 2-34.1 and AMB 2-35.1. Thus, a substitution of the tryptophan at position 208 eliminates the ability of the T cell clone to respond to these peptides. This data suggests that substitutions on the N and C terminal ends of the truncated peptides does not effect the recognition of the peptides by the T cell clone and, thus, does not alter the residues which comprise the T cell epitope.

### Example XII Histamine Release Analysis With Purified Native Amb a I.1 and With Peptides Derived From Amb a I.1

The objective of the histamine release analysis was to compare the effects of Amb a I.1 or Amb a I.1-derived peptides in an in vitro allergic response system. The release of histamine via IgE recognition and IgE receptor crosslinking on viable cells directly assays the allergic potential of a protein antigen.

The histamine release assay used for these studies is based on the detection of an acylated derivative of histamine using a specific monoclonal antibody (Morel, A.M. and Delaage, M.A. (1988) J. Allergy Clin. Immunol. 82:646-654). The assay was performed in two steps: 1) the release of histamine from basophils present in heparinized whole blood in the presence of different concentrations of protein or peptide; and 2) the assay of histamine present in the supernatants of the release reactions following cell removal by centrifugation. The reagents for this second step are available commercially as a competitive radioimmunoassay from AMAC Inc. (Westbrook, ME).

Heparinized whole blood was drawn from ragweed allergic patients. The test protein Amb a I.1 and peptides AMB 1-2.1, AMB 2-9.1, AMB 4-9.1, and AMB 3-5.1 were each diluted to 2X the final release concentration in PACM buffer (PIPES 25mM, NaCl 110mM, KCl 5.0 mM, human serum albumin 0.003% (w/v), CaCl₂ 5mM, MgCl₂ 2mM, pH7.3) and 0.2 ml of each dilution was added to a 1.5 ml polypropylene tube. A 0.2ml aliquot of blood was then added to each tube and the release reactions started by inversion of the tubes. A negative control of 0.2 ml blood incubated with 0.2 ml PACM buffer was also included. The release reactions were performed for 30 minutes at 37°C. The tubes were then centrifuged in a microfuge at 1500 RPM for 3 minutes and the supernatants were carefully removed and analyzed or stored at -20°C for later analysis. To analyze the total histamine released, 0.1 ml blood was diluted with 0.9 ml PACM buffer and boiled 3 minutes. This tube was then centrifuged 2 minutes at 12,000 RPM and the supernatant was removed and save for analysis.

For the competition radioimmunoassay, a 50 µl aliquot of each release supernatant was mixed with 150 µl of histamine release buffer (supplied with the kit from AMAC). The diluted supernatant (100 µl) was added to a kit tube containing an acylation reagent. A 50 µl aliquot of acylation buffer was then immediately added and the tube mixed by vortexing. The acylation reactions were incubated at least 30 minutes at room temperature. A set of histamine standards supplied with the kit were acylated at the same time. 50 µl of each acylation reaction was then placed in a tube coated with a monoclonal antibody which specifically recognizes the acylated form of histamine. A 0.5 ml aliquot of ¹²⁵I-labelled tracer was added and the tubes were incubated at least 18 hours at 4°C. The solutions were aspirated from the tubes and the radioactivity bound to the tube was measured on a gamma counter (Cobra 5005, Beckman, Inc.) for two minutes per tube.

A standard curve was generated from the histamine standard counts and graphed on a semi-log plot. Since this is a competitive assay (the ¹²⁵-labelled trace competes with acylated histamine in the samples for binding to the antibody-coated tubes), the lower the number of radioactive counts measured, the greater the amount of histamine in the sample. The amount of histamine in each sample data point was extrapolated from the standard curve using a computer statistical program (StatView for the MacIntosh). The assay is sensitive to 0.2 nM histamine.

To study whether peptides derived from the Amb a I.1 sequence could induce histamine release, blood samples from 8 ragweed-allergic patients who exhibited histamine release to native Amb a I.1 were analyzed with AMB 1-2.1, AMB 2-9.1, AMB 4-9.1 and AMB 3-5.1. Six or more 10-fold dilutions of Amb a I.1 starting at 10 µg/ml (approximately 0.66 µM) were analyzed. Five five-fold dilutions of each peptide were also analyzed (ranging from 50 µg/ml to 0.08 µg/ml, this is approximately 15 µM to 24 nM in concentration for each peptide). The peptide concentrations were selected to encompass the higher end of the concentration curve. Figure 30 shows the representative results from 1 of the patients (#1273). The graph shows the concentration of each antigen in µg/ml versus the percent of total histamine released. The results are presented as the percent of the total histamine released for that patient, since patients vary greatly in their overall releasable histamine levels. This variability is likely to result from variation in the number of basophils per ml between patients and the histamine content of each basophil. As Figure 30 shows, Amb a I.1, at all the concentrations tested, yields a high level of histamine release in this patient. Similar experiments using blood samples from other patients have shown that for some patients the Amb a I.1 concentration does not become limiting until 10⁻⁵-10⁻⁶ µg/ml. By contrast, there is no discernible histamine release to any of the four Amb a I.1-derived peptides which were used. Similar results were obtained for seven other patients similarly tested.

Although the invention has been described with reference to its preferred embodiments, other embodiments can achieve the same results. Variations and modifications to the present invention will be obvious to those skilled in the art and it is intended to cover in the appended claims all such modifications and equivalents that follow in the true spirit and scope of this invention.

SEQUENCE LISTING

(1) GENERAL INFORMATION:

(i) APPLICANT: Kuo, Mei-chang
Garman, Richard
Greenstein, Julia

(ii) TITLE OF INVENTION: T CELL EPITOPES OF THE MAJOR ALLERGENS
FROM AMBROSIA ARTEMISIIFOLIA

(iii) NUMBER OF SEQUENCES: 150

(iv) CORRESPONDENCE ADDRESS:
(A) ADDRESSEE: LAHIVE & COCKFIELD
(B) STREET: 60 State Street  Suite 510
(C) CITY: Boston
(D) STATE: MA
(E) COUNTRY: USA
(F) ZIP: 02109

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: ASCII Text

(vi) CURRENT APPLICATION DATA:
(A) APPLICATION NUMBER:
(B) FILING DATE:
(C) CLASSIFICATION:

(vi) PRIOR APPLICATION DATA:
(A) APPLICATION NUMBER: US07/866,679
(B) FILING DATE: 09-APR-1992

(viii) ATTORNEY/AGENT INFORMATION:
(A) NAME: Mandragouras, Amy E.
(B) REGISTRATION NUMBER: 36,207
(C) REFERENCE/DOCKET NUMBER: IPC-001CC/IMI-003

(ix) TELECOMMUNICATION INFORMATION:
(A) TELEPHONE: (617) 227-7400
(B) TELEFAX: (617) 227-5941

(2) INFORMATION FOR SEQ ID NO:1:

(i) SEQUENCE CHARACTERISTICS:
(A) LENGTH: 1223 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..1188

(xi) SEQUENCE DESCRIPTION: SEQ ID NO:1:

ATG GGG ATC AAA CAC TGT TGT TAC ATC TTG TAT TTT ACC TTA GCC CTT 48
Met Gly Ile Lys His Cys Cys Tyr Ile Leu Tyr Phe Thr Leu Ala Leu
1               5                   10                  15

GTC ACT TTG CTG CAA CCT GTT CGT TCT GCC GAA GAT CTC CAG GAA ATC 96
Val Thr Leu Leu Gln Pro Val Arg Ser Ala Glu Asp Leu Gln Glu Ile
            20                  25                  30

TTA CCA GTT AAC GAA ACA AGG AGG CTG ACA ACA AGT GGA GCA TAC AAC 144
Leu Pro Val Asn Glu Thr Arg Arg Leu Thr Thr Ser Gly Ala Tyr Asn
        35                  40                  45

ATT ATA GAC GGG TGC TGG AGG GGC AAA GCC GAT TGG GCG GAA AAC CGA 192
Ile Ile Asp Gly Cys Trp Arg Gly Lys Ala Asp Trp Ala Glu Asn Arg
    50                  55                  60

AAA GCG TTA GCC GAT TGT GCC CAA GGT TTT GGG AAG GGA ACA GTG GGC 240
Lys Ala Leu Ala Asp Cys Ala Gln Gly Phe Gly Lys Gly Thr Val Gly
65                  70                  75                  80

GGA AAA GAT GGT GAT ATA TAC ACG GTC ACC AGT GAG CTA GAT GAT GAT 288
Gly Lys Asp Gly Asp Ile Tyr Thr Val Thr Ser Glu Leu Asp Asp Asp
                85                  90                  95

GTT GCA AAT CCA AAA GAA GGC ACA CTC CGG TTT GGT GCC GCC CAA AAC 336
Val Ala Asn Pro Lys Glu Gly Thr Leu Arg Phe Gly Ala Ala Gln Asn
            100                 105                 110

AGG CCC TTG TGG ATC ATT TTT GAA AGA GAT ATG GTG ATT CGT TTG GAT 384
Arg Pro Leu Trp Ile Ile Phe Glu Arg Asp Met Val Ile Arg Leu Asp
        115                 120                 125

AAA GAG ATG GTG GTA AAC AGT GAC AAG ACC ATC GAT GGC CGA GGG GCG 432
Lys Glu Met Val Val Asn Ser Asp Lys Thr Ile Asp Gly Arg Gly Ala
    130                 135                 140

AAA GTT GAA ATC ATT AAC GCT GGT TTC ACC CTT AAT GGT GTC AAG AAT 480
Lys Val Glu Ile Ile Asn Ala Gly Phe Thr Leu Asn Gly Val Lys Asn
145                 150                 155                 160

GTA ATC ATT CAT AAC ATA AAT ATG CAT GAT GTT AAA GTG AAT CCA GGA 528
Val Ile Ile His Asn Ile Asn Met His Asp Val Lys Val Asn Pro Gly
                165                 170                 175

GGC CTG ATT AAG TCC AAC GAT GGT CCA GCA GCT CCA AGA GCT GGT AGT 576
Gly Leu Ile Lys Ser Asn Asp Gly Pro Ala Ala Pro Arg Ala Gly Ser
            180                 185                 190

GAT GGT GAT GCT ATA AGT ATT TCT GGT AGT TCA CAA ATA TGG ATC GAC 624
Asp Gly Asp Ala Ile Ser Ile Ser Gly Ser Ser Gln Ile Trp Ile Asp
        195                 200                 205

CAT TGT TCG CTC AGT AAG TCT GTT GAT GGG CTG GTA GAT GCC AAG CTC 672
His Cys Ser Leu Ser Lys Ser Val Asp Gly Leu Val Asp Ala Lys Leu
    210                 215                 220

GGC ACC ACA CGC TTA ACC GTT TCC AAC AGC TTA TTC ACC CAA CAC CAG 720
Gly Thr Thr Arg Leu Thr Val Ser Asn Ser Leu Phe Thr Gln His Gln
225                 230                 235                 240

TTT GTA CTA TTA TTC GGG GCT GGT GAC GAA AAT ATT GAA GAT AGA GGC 768
Phe Val Leu Leu Phe Gly Ala Gly Asp Glu Asn Ile Glu Asp Arg Gly
                245                 250                 255

ATG CTA GCA ACG GTC GCT TTC AAC ACG TTC ACT GAT AAC GTT GAC CAA 816
Met Leu Ala Thr Val Ala Phe Asn Thr Phe Thr Asp Asn Val Asp Gln
            260                 265                 270

AGA ATG CCT AGA TGT CGA CAT GGG TTT TTC CAA GTC GTT AAC AAC AAC 864
Arg Met Pro Arg Cys Arg His Gly Phe Phe Gln Val Val Asn Asn Asn
        275                 280                 285

TAT GAT AAA TGG GGA TCG TAT GCC ATC GGT GGT AGC GCG TCC CCA ACC 912
Tyr Asp Lys Trp Gly Ser Tyr Ala Ile Gly Gly Ser Ala Ser Pro Thr
    290                 295                 300

ATA CTC AGC CAA GGG AAC AGA TTC TGC GCC CCC GAT GAA CGC AGC AAG 960
Ile Leu Ser Gln Gly Asn Arg Phe Cys Ala Pro Asp Glu Arg Ser Lys
305                 310                 315                 320

AAA AAT GTC CTA GGA AGG CAT GGT GAA GCC GCC GCA GAG TCG ATG AAG 1008
Lys Asn Val Leu Gly Arg His Gly Glu Ala Ala Ala Glu Ser Met Lys
                325                 330                 335

TGG AAC TGG AGA ACG AAT AAA GAC GTG CTT GAA AAT GGT GCT ATT TTT 1056
Trp Asn Trp Arg Thr Asn Lys Asp Val Leu Glu Asn Gly Ala Ile Phe
            340                 345                 350

GTT GCA TCC GGG GTC GAT CCA GTG CTA ACC CCT GAG CAA AGC GCA GGG 1104
Val Ala Ser Gly Val Asp Pro Val Leu Thr Pro Glu Gln Ser Ala Gly
        355                 360                 365

ATG ATT CCA GCC GAA CCA GGA GAG TCC GCT CTA AGC CTC ACT AGT AGT 1152
Met Ile Pro Ala Glu Pro Gly Glu Ser Ala Leu Ser Leu Thr Ser Ser
    370                 375                 380

GCT GGT GTA CTC TCA TGC CAA CCC GGA GCA CCT TGC TAAGCACCCG 1198
Ala Gly Val Leu Ser Cys Gln Pro Gly Ala Pro Cys
385                 390                 395

ACCAATTACT AAGCACTTAT AATGA 1223

(2) INFORMATION FOR SEQ ID NO:2:

(i) SEQUENCE CHARACTERISTICS:
(A) LENGTH: 396 amino acids
(B) TYPE: amino acid
(D) TOPOLOGY: linear

(ii) MOLECULE TYPE: protein

(xi) SEQUENCE DESCRIPTION: SEQ ID NO:2:
   Met Gly Ile Lys His Cys Cys Tyr Ile Leu Tyr Phe Thr Leu Ala Leu
   1 5 10 15
   Val Thr Leu Leu Gln Pro Val Arg Ser Ala Glu Asp Leu Gln Glu Ile
   20 25 30
   Leu Pro Val Asn Glu Thr Arg Arg Leu Thr Thr Ser Gly Ala Tyr Asn
   35 40 45
   Ile Ile Asp Gly Cys Trp Arg Gly Lys Ala Asp Trp Ala Glu Asn Arg
   50 55 60
   Lys Ala Leu Ala Asp Cys Ala Gln Gly Phe Gly Lys Gly Thr Val Gly
   65 70 75 80
   Gly Lys Asp Gly Asp Ile Tyr Thr Val Thr Ser Glu Leu Asp Asp Asp
   85 90 95
   Val Ala Asn Pro Lys Glu Gly Thr Leu Arg Phe Gly Ala Ala Gln Asn
   100 105 110
   Arg Pro Leu Trp Ile Ile Phe Glu Arg Asp Met Val Ile Arg Leu Asp
   115 120 125
   Lys Glu Met Val Val Asn Ser Asp Lys Thr Ile Asp Gly Arg Gly Ala
   130 135 140
   Lys Val Glu Ile Ile Asn Ala Gly Phe Thr Leu Asn Gly Val Lys Asn
   145 150 155 160
   Val Ile Ile His Asn Ile Asn Met His Asp Val Lys Val Asn Pro Gly
   165 170 175
   Gly Leu Ile Lys Ser Asn Asp Gly Pro Ala Ala Pro Arg Ala Gly Ser
   180 185 190
   Asp Gly Asp Ala Ile Ser Ile Ser Gly Ser Ser Gln Ile Trp Ile Asp
   195 200 205
   His Cys Ser Leu Ser Lys Ser Val Asp Gly Leu Val Asp Ala Lys Leu
   210 215 220
   Gly Thr Thr Arg Leu Thr Val Ser Asn Ser Leu Phe Thr Gln His Gln
   225 230 235 240
   Phe Val Leu Leu Phe Gly Ala Gly Asp Glu Asn Ile Glu Asp Arg Gly
   245 250 255
   Met Leu Ala Thr Val Ala Phe Asn Thr Phe Thr Asp Asn Val Asp Gln
   260 265 270
   Arg Met Pro Arg Cys Arg His Gly Phe Phe Gln Val Val Asn Asn Asn
   275 280 285
   Tyr Asp Lys Trp Gly Ser Tyr Ala Ile Gly Gly Ser Ala Ser Pro Thr
   290 295 300
   Ile Leu Ser Gln Gly Asn Arg Phe Cys Ala Pro Asp Glu Arg Ser Lys
   305 310 315 320
   Lys Asn Val Leu Gly Arg His Gly Glu Ala Ala Ala Glu Ser Met Lys
   325 330 335
   Trp Asn Trp Arg Thr Asn Lys Asp Val Leu Glu Asn Gly Ala Ile Phe
   340 345 350
   Val Ala Ser Gly Val Asp Pro Val Leu Thr Pro Glu Gln Ser Ala Gly
   355 360 365
   Met Ile Pro Ala Glu Pro Gly Glu Ser Ala Leu Ser Leu Thr Ser Ser
       370                 375                 380
   Ala Gly Val Leu Ser Cys Gln Pro Gly Ala Pro Cys
   385                 390                 395

(2) INFORMATION FOR SEQ ID NO:3:

(i) SEQUENCE CHARACTERISTICS:
(A) LENGTH: 1349 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..1194

(xi) SEQUENCE DESCRIPTION: SEQ ID NO:3:

ATG GGG ATC AAA CAC TGT TGT TAC ATC TTG TAT TTT ACC TTA GCC CTT 48
Met Gly Ile Lys His Cys Cys Tyr Ile Leu Tyr Phe Thr Leu Ala Leu
1               5                   10                  15

GTC ACT TTG CTG CAA CCT GTT CGT TCT GCA GAA GAT GTT GAA GAA TTC 96
Val Thr Leu Leu Gln Pro Val Arg Ser Ala Glu Asp Val Glu Glu Phe
            20                  25                  30

TTA CCT TCA GCT AAC GAA ACA AGG AGG AGC CTG AAA GCA TGT GAA GCA 144
Leu Pro Ser Ala Asn Glu Thr Arg Arg Ser Leu Lys Ala Cys Glu Ala
        35                  40                  45

CAC AAC ATT ATA GAC AAG TGC TGG AGG TGC AAA GCC GAT TGG GCG AAT 192
His Asn Ile Ile Asp Lys Cys Trp Arg Cys Lys Ala Asp Trp Ala Asn
    50                  55                  60

AAC CGA CAA GCG TTA GCC GAT TGT GCC CAA GGT TTT GCA AAG GGA ACC 240
Asn Arg Gln Ala Leu Ala Asp Cys Ala Gln Gly Phe Ala Lys Gly Thr
65                  70                  75                  80

TAC GGT GGA AAA CAT GGT GAT GTC TAC ACG GTC ACC AGT GAT AAA GAT 288
Tyr Gly Gly Lys His Gly Asp Val Tyr Thr Val Thr Ser Asp Lys Asp
                85                  90                  95

GAT GAT GTT GCA AAT CCA AAA GAA GGC ACA CTC CGG TTT GCT GCT GCC 336
Asp Asp Val Ala Asn Pro Lys Glu Gly Thr Leu Arg Phe Ala Ala Ala
            100                 105                 110

CAA AAC AGG CCC TTG TGG ATC ATT TTT AAA AGA AAT ATG GTG ATT CAT 384
Gln Asn Arg Pro Leu Trp Ile Ile Phe Lys Arg Asn Met Val Ile His
        115                 120                 125

TTG AAT CAA GAG CTT GTC GTA AAC AGC GAC AAG ACC ATC GAT GGC CGA 432
Leu Asn Gln Glu Leu Val Val Asn Ser Asp Lys Thr Ile Asp Gly Arg
    130                 135                 140

GGG GTG AAA GTT AAC ATC GTT AAC GCC GGT CTC ACC CTC ATG AAT GTC 480
Gly Val Lys Val Asn Ile Val Asn Ala Gly Leu Thr Leu Met Asn Val
145                 150                 155                 160

AAG AAT ATA ATC ATT CAT AAC ATA AAT ATC CAT GAT ATT AAA GTT TGT 528
Lys Asn Ile Ile Ile His Asn Ile Asn Ile His Asp Ile Lys Val Cys
                165                 170                 175

CCA GGA GGC ATG ATT AAG TCC AAC GAT GGT CCA CCA ATT TTA AGA CAA 576
Pro Gly Gly Met Ile Lys Ser Asn Asp Gly Pro Pro Ile Leu Arg Gln
            180                 185                 190

CAA AGT GAT GGT GAT GCT ATA AAT GTT GCT GGT AGT TCA CAA ATA TGG 624
Gln Ser Asp Gly Asp Ala Ile Asn Val Ala Gly Ser Ser Gln Ile Trp
        195                 200                 205

ATC GAC CAT TGC TCG CTC AGT AAG GCT TCC GAT GGG CTG CTC GAT ATC 672
Ile Asp His Cys Ser Leu Ser Lys Ala Ser Asp Gly Leu Leu Asp Ile
    210                 215                 220

ACC CTC GGC AGC TCA CAC GTG ACC GTT TCC AAC TGC AAA TTC ACC CAA 720
Thr Leu Gly Ser Ser His Val Thr Val Ser Asn Cys Lys Phe Thr Gln
225                 230                 235                 240

CAC CAA TTT GTA TTA TTG CTC GGG GCT GAT GAC ACC CAT TAT CAA GAT 768
His Gln Phe Val Leu Leu Leu Gly Ala Asp Asp Thr His Tyr Gln Asp
                245                 250                 255

AAA GGC ATG CTA GCA ACG GTA GCA TTC AAC ATG TTC ACC GAT CAC GTT 816
Lys Gly Met Leu Ala Thr Val Ala Phe Asn Met Phe Thr Asp His Val
            260                 265                 270

GAC CAA AGA ATG CCT AGA TGT AGA TTT GGG TTT TTC CAA GTC GTT AAC 864
Asp Gln Arg Met Pro Arg Cys Arg Phe Gly Phe Phe Gln Val Val Asn
        275                 280                 285

AAC AAC TAC GAC AGA TGG GGA ACG TAC GCC ATC GGT GGT AGC TCG GCC 912
Asn Asn Tyr Asp Arg Trp Gly Thr Tyr Ala Ile Gly Gly Ser Ser Ala
    290                 295                 300

CCA ACT ATA CTC AGC CAA GGG AAC AGA TTC TTC GCC CCC GAT GAT ATC 960
Pro Thr Ile Leu Ser Gln Gly Asn Arg Phe Phe Ala Pro Asp Asp Ile
305                 310                 315                 320

ATC AAG AAA AAT GTC TTA GCG AGG ACT GGT ACT GGC AAC GCA GAG TCG 1008
Ile Lys Lys Asn Val Leu Ala Arg Thr Gly Thr Gly Asn Ala Glu Ser
                325                 330                 335

ATG TCG TGG AAC TGG AGA ACA GAT AGA GAC TTG CTT GAA AAT GGT GCT 1056
Met Ser Trp Asn Trp Arg Thr Asp Arg Asp Leu Leu Glu Asn Gly Ala
            340                 345                 350

ATT TTT CTC CCA TCC GGG TCT GAT CCA GTG CTA ACC CCT GAG CAA AAA 1104
Ile Phe Leu Pro Ser Gly Ser Asp Pro Val Leu Thr Pro Glu Gln Lys
        355                 360                 365

GCA GGG ATG ATT CCA GCT GAA CCA GGA GAA GCC GTT CTA AGA CTC ACT 1152
Ala Gly Met Ile Pro Ala Glu Pro Gly Glu Ala Val Leu Arg Leu Thr
    370                 375                 380

AGT AGT GCT GGT GTA CTC TCA TGC CAT CAA GGA GCA CCT TGC TAA 1197
Ser Ser Ala Gly Val Leu Ser Cys His Gln Gly Ala Pro Cys
385                 390                 395

GCACCTGGCC AATTCCTAAG CTTTTATAAT AATCATAAAT ACTTATTTTA TTTTATTTTT 1260

GATATTTTAT ATGAACCATT ACGTTCAAGT ACTCTATTAA CATGTTTTAA ATTCATAAGA 1320

GTTTATTGAT AAAAAAAAAA AAAACCGAAT TC 1349

(2) INFORMATION FOR SEQ ID NO:4:

(i) SEQUENCE CHARACTERISTICS:
(A) LENGTH: 398 amino acids
(B) TYPE: amino acid
(D) TOPOLOGY: linear

(ii) MOLECULE TYPE: protein

(xi) SEQUENCE DESCRIPTION: SEQ ID NO:4:

Met Gly Ile Lys His Cys Cys Tyr Ile Leu Tyr Phe Thr Leu Ala Leu
1               5                   10                  15

Val Thr Leu Leu Gln Pro Val Arg Ser Ala Glu Asp Val Glu Glu Phe
            20                  25                  30

Leu Pro Ser Ala Asn Glu Thr Arg Arg Ser Leu Lys Ala Cys Glu Ala
        35                  40                  45

His Asn Ile Ile Asp Lys Cys Trp Arg Cys Lys Ala Asp Trp Ala Asn
    50                  55                  60

Asn Arg Gln Ala Leu Ala Asp Cys Ala Gln Gly Phe Ala Lys Gly Thr
65                  70                  75                  80

Tyr Gly Gly Lys His Gly Asp Val Tyr Thr Val Thr Ser Asp Lys Asp
                85                  90                  95

Asp Asp Val Ala Asn Pro Lys Glu Gly Thr Leu Arg Phe Ala Ala Ala
            100                 105                 110

Gln Asn Arg Pro Leu Trp Ile Ile Phe Lys Arg Asn Met Val Ile His
        115                 120                 125

Leu Asn Gln Glu Leu Val Val Asn Ser Asp Lys Thr Ile Asp Gly Arg
    130                 135                 140

Gly Val Lys Val Asn Ile Val Asn Ala Gly Leu Thr Leu Met Asn Val
145                 150                 155                 160

Lys Asn Ile Ile Ile His Asn Ile Asn Ile His Asp Ile Lys Val Cys
                165                 170                 175

Pro Gly Gly Met Ile Lys Ser Asn Asp Gly Pro Pro Ile Leu Arg Gln
            180                 185                 190

Gln Ser Asp Gly Asp Ala Ile Asn Val Ala Gly Ser Ser Gln Ile Trp
        195                 200                 205

Ile Asp His Cys Ser Leu Ser Lys Ala Ser Asp Gly Leu Leu Asp Ile
    210                 215                 220

Thr Leu Gly Ser Ser His Val Thr Val Ser Asn Cys Lys Phe Thr Gln
225                 230                 235                 240

His Gln Phe Val Leu Leu Leu Gly Ala Asp Asp Thr His Tyr Gln Asp
                245                 250                 255

Lys Gly Met Leu Ala Thr Val Ala Phe Asn Met Phe Thr Asp His Val
            260                 265                 270

Asp Gln Arg Met Pro Arg Cys Arg Phe Gly Phe Phe Gln Val Val Asn
        275                 280                 285

Asn Asn Tyr Asp Arg Trp Gly Thr Tyr Ala Ile Gly Gly Ser Ser Ala
    290                 295                 300

Pro Thr Ile Leu Ser Gln Gly Asn Arg Phe Phe Ala Pro Asp Asp Ile
305                 310                 315                 320

Ile Lys Lys Asn Val Leu Ala Arg Thr Gly Thr Gly Asn Ala Glu Ser
                325                 330                 335

Met Ser Trp Asn Trp Arg Thr Asp Arg Asp Leu Leu Glu Asn Gly Ala
            340                 345                 350

Ile Phe Leu Pro Ser Gly Ser Asp Pro Val Leu Thr Pro Glu Gln Lys
        355                 360                 365

Ala Gly Met Ile Pro Ala Glu Pro Gly Glu Ala Val Leu Arg Leu Thr
    370                 375                 380

Ser Ser Ala Gly Val Leu Ser Cys His Gln Gly Ala Pro Cys
385                 390                 395

(2) INFORMATION FOR SEQ ID NO:5:

     (i) SEQUENCE CHARACTERISTICS:
          (A) LENGTH: 1320 base pairs
          (B) TYPE: nucleic acid
          (C) STRANDEDNESS: single
          (D) TOPOLOGY: linear

    (ii) MOLECULE TYPE: cDNA

    (ix) FEATURE:
          (A) NAME/KEY: CDS
          (B) LOCATION: 1..1191

    (xi) SEQUENCE DESCRIPTION: SEQ ID NO:5:

ATG GGG ATC AAA CAA TGT TGT TAC ATC TTG TAT TTT ACC TTA GCA CTT      48
Met Gly Ile Lys Gln Cys Cys Tyr Ile Leu Tyr Phe Thr Leu Ala Leu
  1               5                  10                  15

GTC GCT TTG CTG CAA CCT GTT CGT TCT GCC GAA GGT GTC GGG GAA ATC      96
Val Ala Leu Leu Gln Pro Val Arg Ser Ala Glu Gly Val Gly Glu Ile
             20                  25                  30

TTA CCT TCA GTT AAC GAA ACG AGG AGC CTG CAA GCA TGT GAA GCA CTC 144
Leu Pro Ser Val Asn Glu Thr Arg Ser Leu Gln Ala Cys Glu Ala Leu
        35                  40                  45

AAC ATT ATA GAC AAG TGC TGG AGG GGC AAA GCC GAT TGG GAG AAC AAC 192
Asn Ile Ile Asp Lys Cys Trp Arg Gly Lys Ala Asp Trp Glu Asn Asn
    50                  55                  60

CGA CAA GCG TTA GCC GAC TGT GCC CAA GGT TTT GCA AAG GGA ACC TAC 240
Arg Gln Ala Leu Ala Asp Cys Ala Gln Gly Phe Ala Lys Gly Thr Tyr
65                  70                  75                  80

GGC GGA AAA TGG GGT GAT GTC TAC ACG GTC ACC AGC AAT CTA GAT GAT 288
Gly Gly Lys Trp Gly Asp Val Tyr Thr Val Thr Ser Asn Leu Asp Asp
                85                  90                  95

GAT GTT GCA AAT CCA AAA GAA GGC ACA CTC CGG TTT GCT GCC GCC CAA 336
Asp Val Ala Asn Pro Lys Glu Gly Thr Leu Arg Phe Ala Ala Ala Gln
            100                 105                 110

AAC AGG CCC TTG TGG ATC ATT TTT AAA AAT GAT ATG GTG ATT AAT TTG 384
Asn Arg Pro Leu Trp Ile Ile Phe Lys Asn Asp Met Val Ile Asn Leu
        115                 120                 125

AAT CAA GAG CTT GTC GTA AAC AGC GAC AAG ACC ATC GAT GGC CGA GGG 432
Asn Gln Glu Leu Val Val Asn Ser Asp Lys Thr Ile Asp Gly Arg Gly
    130                 135                 140

GTG AAA GTT GAA ATC ATT AAC GGA GGT CTC ACC CTC ATG AAT GTC AAG 480
Val Lys Val Glu Ile Ile Asn Gly Gly Leu Thr Leu Met Asn Val Lys
145                 150                 155                 160

AAT ATA ATC ATT CAT AAC ATA AAT ATC CAT GAT GTT AAA GTG CTT CCA 528
Asn Ile Ile Ile His Asn Ile Asn Ile His Asp Val Lys Val Leu Pro
                165                 170                 175

GGA GGC ATG ATT AAG TCC AAC GAT GGT CCA CCA ATT TTA AGA CAA GCA 576
Gly Gly Met Ile Lys Ser Asn Asp Gly Pro Pro Ile Leu Arg Gln Ala
            180                 185                 190

AGT GAT GGG GAT ACT ATA AAT GTT GCT GGT AGT TCC CAA ATA TGG ATA 624
Ser Asp Gly Asp Thr Ile Asn Val Ala Gly Ser Ser Gln Ile Trp Ile
        195                 200                 205

GAC CAT TGC TCA CTC AGC AAG TCT TTC GAT GGG CTG GTC GAT GTC ACC 672
Asp His Cys Ser Leu Ser Lys Ser Phe Asp Gly Leu Val Asp Val Thr
    210                 215                 220

CTC GGT AGC ACA CAC GTG ACC ATT TCC AAC TGC AAA TTC ACC CAA CAG 720
Leu Gly Ser Thr His Val Thr Ile Ser Asn Cys Lys Phe Thr Gln Gln
225                 230                 235                 240

TCA AAA GCA ATA TTG TTG GGA GCA GAT GAC ACC CAT GTT CAA GAT AAA 768
Ser Lys Ala Ile Leu Leu Gly Ala Asp Asp Thr His Val Gln Asp Lys
                245                 250                 255

GGA ATG CTA GCA ACG GTC GCT TTC AAC ATG TTC ACC GAT AAC GTT GAC 816
Gly Met Leu Ala Thr Val Ala Phe Asn Met Phe Thr Asp Asn Val Asp
            260                 265                 270

CAA AGA ATG CCT AGA TGT CGA TTT GGG TTT TTC CAA GTT GTT AAC AAC 864
Gln Arg Met Pro Arg Cys Arg Phe Gly Phe Phe Gln Val Val Asn Asn
        275                 280                 285

AAC TAC GAC AGA TGG GGA ACG TAC GCC ATA GGT GGT AGC TCG GCC CCA 912
Asn Tyr Asp Arg Trp Gly Thr Tyr Ala Ile Gly Gly Ser Ser Ala Pro
    290                 295                 300

ACT ATA CTC TGC CAA GGG AAC AGA TTC TTG GCC CCT GAT GAT CAG ATC 960
Thr Ile Leu Cys Gln Gly Asn Arg Phe Leu Ala Pro Asp Asp Gln Ile
305                 310                 315                 320

AAG AAA AAT GTC CTA GCG AGG ACT GGT ACA GGC GCT GCT GAG TCG ATG 1008
Lys Lys Asn Val Leu Ala Arg Thr Gly Thr Gly Ala Ala Glu Ser Met
                325                 330                 335

GCG TGG AAC TGG AGA TCT GAT AAA GAC TTG CTT GAA AAT GGT GCT ATT 1056
Ala Trp Asn Trp Arg Ser Asp Lys Asp Leu Leu Glu Asn Gly Ala Ile
            340                 345                 350

TTT GTT ACA TCT GGG TCT GAT CCA GTG CTA ACC CCT GTT CAA AGC GCA 1104
Phe Val Thr Ser Gly Ser Asp Pro Val Leu Thr Pro Val Gln Ser Ala
        355                 360                 365

GGG ATG ATT CCA GCT GAA CCA GGA GAA GCC GCT ATA AAA CTC ACT AGT 1152
Gly Met Ile Pro Ala Glu Pro Gly Glu Ala Ala Ile Lys Leu Thr Ser
    370                 375                 380

AGT GCT GGT GTA TTC TCA TGC CGT CCT GGA GCA CCT TGC TAAGCACCCT 1201
Ser Ala Gly Val Phe Ser Cys Arg Pro Gly Ala Pro Cys
385                 390                 395

GCCAATTCTC CTAAGCTTTT GCAATGATCA AAAATACTTT TTTATTTTAT TTTTAATATT 1261

TTATATGTAC TGGAAATGAA CCATTACCTT CTAGTACTCT ATAACATGTT TTGCATTTA 1320

(2) INFORMATION FOR SEQ ID NO:6:

(i) SEQUENCE CHARACTERISTICS:
(A) LENGTH: 397 amino acids
(B) TYPE: amino acid
(D) TOPOLOGY: linear

(ii) MOLECULE TYPE: protein

(xi) SEQUENCE DESCRIPTION: SEQ ID NO:6:

Met Gly Ile Lys Gln Cys Cys Tyr Ile Leu Tyr Phe Thr Leu Ala Leu
1               5                   10                  15

Val Ala Leu Leu Gln Pro Val Arg Ser Ala Glu Gly Val Gly Glu Ile
            20                  25                  30

Leu Pro Ser Val Asn Glu Thr Arg Ser Leu Gln Ala Cys Glu Ala Leu
        35                  40                  45

Asn Ile Ile Asp Lys Cys Trp Arg Gly Lys Ala Asp Trp Glu Asn Asn
    50                  55                  60

Arg Gln Ala Leu Ala Asp Cys Ala Gln Gly Phe Ala Lys Gly Thr Tyr
65                  70                  75                  80

Gly Gly Lys Trp Gly Asp Val Tyr Thr Val Thr Ser Asn Leu Asp Asp
                85                  90                  95

Asp Val Ala Asn Pro Lys Glu Gly Thr Leu Arg Phe Ala Ala Ala Gln
            100                 105                 110

Asn Arg Pro Leu Trp Ile Ile Phe Lys Asn Asp Met Val Ile Asn Leu
        115                 120                 125

Asn Gln Glu Leu Val Val Asn Ser Asp Lys Thr Ile Asp Gly Arg Gly
    130                 135                 140

Val Lys Val Glu Ile Ile Asn Gly Gly Leu Thr Leu Met Asn Val Lys
145                 150                 155                 160

Asn Ile Ile Ile His Asn Ile Asn Ile His Asp Val Lys Val Leu Pro
                165                 170                 175

Gly Gly Met Ile Lys Ser Asn Asp Gly Pro Pro Ile Leu Arg Gln Ala
            180                 185                 190

Ser Asp Gly Asp Thr Ile Asn Val Ala Gly Ser Ser Gln Ile Trp Ile
        195                 200                 205

Asp His Cys Ser Leu Ser Lys Ser Phe Asp Gly Leu Val Asp Val Thr
    210                 215                 220

Leu Gly Ser Thr His Val Thr Ile Ser Asn Cys Lys Phe Thr Gln Gln
225                 230                 235                 240

Ser Lys Ala Ile Leu Leu Gly Ala Asp Asp Thr His Val Gln Asp Lys
                245                 250                 255

Gly Met Leu Ala Thr Val Ala Phe Asn Met Phe Thr Asp Asn Val Asp
            260                 265                 270

Gln Arg Met Pro Arg Cys Arg Phe Gly Phe Pne Gln Val Val Asn Asn
        275                 280                 285

Asn Tyr Asp Arg Trp Gly Thr Tyr Ala Ile Gly Gly Ser Ser Ala Pro
    290                 295                 300

Thr Ile Leu Cys Gln Gly Asn Arg Phe Leu Ala Pro Asp Asp Gln Ile
305                 310                 315                 320

Lys Lys Asn Val Leu Ala Arg Thr Gly Thr Gly Ala Ala Glu Ser Met
                325                 330                 335

Ala Trp Asn Trp Arg Ser Asp Lys Asp Leu Leu Glu Asn Gly Ala Ile
            340                 345                 350

Phe Val Thr Ser Gly Ser Asp Pro Val Leu Thr Pro Val Gln Ser Ala
        355                 360                 365

Gly Met Ile Pro Ala Glu Pro Gly Glu Ala Ala Ile Lys Leu Thr Ser
    370                 375                 380

Ser Ala Gly Val Phe Ser Cys Arg Pro Gly Ala Pro Cys
385                 390                 395

(2) INFORMATION FOR SEQ ID NO:7:

     (i) SEQUENCE CHARACTERISTICS:
          (A) LENGTH: 1187 base pairs
          (B) TYPE: nucleic acid
          (C) STRANDEDNESS: single
          (D) TOPOLOGY: linear

    (ii) MOLECULE TYPE: cDNA

(ix) FEATURE:
(A) NAME/KEY: CDS
(B) LOCATION: 1..1176

(xi) SEQUENCE DESCRIPTION: SEQ ID NO:7:

ATG GGG ATC AAA CAC TGT TGT TAC ATC TTG TAT TTT ACC TTA GCC CTT 48
Met Gly Ile Lys His Cys Cys Tyr Ile Leu Tyr Phe Thr Leu Ala Leu
1               5                   10                  15

GTC ACT TTG CTG CAA CCT GTT CGT TCT GCC GAA GAT CTC CAG GAA ATC 96
Val Thr Leu Leu Gln Pro Val Arg Ser Ala Glu Asp Leu Gln Glu Ile
            20                  25                  30

TTA CCT TCA GCT AAC GAA ACA AGG AGC CTG ACA ACA TGT GGA ACA TAC 144
Leu Pro Ser Ala Asn Glu Thr Arg Ser Leu Thr Thr Cys Gly Thr Tyr
        35                  40                  45

AAC ATT ATA GAC GGG TGC TGG AGG GGC AAA GCC GAT TGG GCG GAA AAC 192
Asn Ile Ile Asp Gly Cys Trp Arg Gly Lys Ala Asp Trp Ala Glu Asn
    50                  55                  60

CGA AAA GCG TTA GCC GAT TGT GCC CAA GGT TTT GCA AAG GGA ACA ATC 240
Arg Lys Ala Leu Ala Asp Cys Ala Gln Gly Phe Ala Lys Gly Thr Ile
65                  70                  75                  80

GGC GGA AAA GAT GGT GAT ATA TAC ACG GTC ACC AGT GAG CTA GAT GAT 288
Gly Gly Lys Asp Gly Asp Ile Tyr Thr Val Thr Ser Glu Leu Asp Asp
                85                  90                  95

GAT GTT GCA AAT CCA AAA GAA GGC ACA CTC CGG TTT GGT GCC GCC CAA 336
Asp Val Ala Asn Pro Lys Glu Gly Thr Leu Arg Phe Gly Ala Ala Gln
            100                 105                 110

AAC AGG CCC TTG TGG ATT ATT TTT GAA AGA GAT ATG GTG ATT CGT TTG 384
Asn Arg Pro Leu Trp Ile Ile Phe Glu Arg Asp Met Val Ile Arg Leu
        115                 120                 125

GAT AGA GAG TTG GCT ATA AAC AAC GAC AAG ACC ATC GAT GGC CGA GGG 432
Asp Arg Glu Leu Ala Ile Asn Asn Asp Lys Thr Ile Asp Gly Arg Gly
    130                 135                 140

GCG AAA GTT GAA ATC ATT AAC GCT GGT TTC GCC ATC TAT AAT GTC AAG 480
Ala Lys Val Glu Ile Ile Asn Ala Gly Phe Ala Ile Tyr Asn Val Lys
145                 150                 155                 160

AAT ATA ATC ATT CAT AAC ATA ATT ATG CAT GAT ATT GTA GTG AAT CCA 528
Asn Ile Ile Ile His Asn Ile Ile Met His Asp Ile Val Val Asn Pro
                165                 170                 175

GGA GGC CTG ATT AAG TCC CAC GAT GGT CCA CCA GTT CCA AGA AAG GGT 576
Gly Gly Leu Ile Lys Ser His Asp Gly Pro Pro Val Pro Arg Lys Gly
            180                 185                 190

AGT GAT GGT GAT GCT ATA GGT ATT TCT GGT GGT TCA CAA ATA TGG ATC 624
Ser Asp Gly Asp Ala Ile Gly Ile Ser Gly Gly Ser Gln Ile Trp Ile
        195                 200                 205

GAC CAT TGC TCC CTC AGT AAG GCT GTT GAT GGG CTA ATC GAT GCT AAA 672
Asp His Cys Ser Leu Ser Lys Ala Val Asp Gly Leu Ile Asp Ala Lys
    210                 215                 220

CAC GGC AGC ACA CAC TTC ACC GTT TCT AAC TGC TTA TTC ACC CAA CAC 720
His Gly Ser Thr His Phe Thr Val Ser Asn Cys Leu Phe Thr Gln His
225                 230                 235                 240

CAA TAT TTA TTA TTG TTC TGG GAT TTT GAC GAG CGA GGC ATG CTA TGT 768
Gln Tyr Leu Leu Leu Phe Trp Asp Phe Asp Glu Arg Gly Met Leu Cys
                245                 250                 255

ACG GTC GCA TTC AAC AAG TTC ACT GAT AAC GTT GAC CAA AGA ATG CCT 816
Thr Val Ala Phe Asn Lys Phe Thr Asp Asn Val Asp Gln Arg Met Pro
            260                 265                 270

AAC TTA CGA CAT GGG TTT GTC CAA GTC GTT AAC AAC AAC TAC GAA AGA 864
Asn Leu Arg His Gly Phe Val Gln Val Val Asn Asn Asn Tyr Glu Arg
        275                 280                 285

TGG GGA TCG TAC GCC CTC GGT GGT AGC GCA GGC CCA ACC ATA CTT AGC 912
Trp Gly Ser Tyr Ala Leu Gly Gly Ser Ala Gly Pro Thr Ile Leu Ser
    290                 295                 300

CAA GGG AAC AGA TTC TTA GCC TCC GAT ATC AAG AAA GAG GTC GTA GGG 960
Gln Gly Asn Arg Phe Leu Ala Ser Asp Ile Lys Lys Glu Val Val Gly
305                 310                 315                 320

AGG TAT GGT GAA TCC GCC ATG TCA GAG TCG ATT AAT TGG AAC TGG AGA 1008
Arg Tyr Gly Glu Ser Ala Met Ser Glu Ser Ile Asn Trp Asn Trp Arg
                325                 330                 335

TCG TAT ATG GAC GTA TTT GAA AAT GGT GCT ATT TTT GTT CCA TCC GGG 1056
Ser Tyr Met Asp Val Phe Glu Asn Gly Ala Ile Phe Val Pro Ser Gly
            340                 345                 350

GTT GAT CCA GTG CTA ACC CCT GAG CAA AAC GCA GGG ATG ATT CCA GCC 1104
Val Asp Pro Val Leu Thr Pro Glu Gln Asn Ala Gly Met Ile Pro Ala
        355                 360                 365

GAA CCA GGA GAA GCC GTT CTA AGA CTC ACT AGT AGT GCT GGT GTC CTC 1152
Glu Pro Gly Glu Ala Val Leu Arg Leu Thr Ser Ser Ala Gly Val Leu
    370                 375                 380

TCA TGC CAA CCT GGA GCA CCT TGC TAAGCACTGC A 1187
Ser Cys Gln Pro Gly Ala Pro Cys
385                 390

(2) INFORMATION FOR SEQ ID NO:8:

(i) SEQUENCE CHARACTERISTICS:
(A) LENGTH: 392 amino acids
(B) TYPE: amino acid
(D) TOPOLOGY: linear

(ii) MOLECULE TYPE: protein

(xi) SEQUENCE DESCRIPTION: SEQ ID NO:8:

Met Gly Ile Lys His Cys Cys Tyr Ile Leu Tyr Phe Thr Leu Ala Leu
1               5                   10                  15

Val Thr Leu Leu Gln Pro Val Arg Ser Ala Glu Asp Leu Gln Glu Ile
            20                  25                  30

Leu Pro Ser Ala Asn Glu Thr Arg Ser Leu Thr Thr Cys Gly Thr Tyr
        35                  40                  45

Asn Ile Ile Asp Gly Cys Trp Arg Gly Lys Ala Asp Trp Ala Glu Asn
    50                  55                  60

Arg Lys Ala Leu Ala Asp Cys Ala Gln Gly Phe Ala Lys Gly Thr Ile
65                  70                  75                  80

Gly Gly Lys Asp Gly Asp Ile Tyr Thr Val Thr Ser Glu Leu Asp Asp
                85                  90                  95

Asp Val Ala Asn Pro Lys Glu Gly Thr Leu Arg Phe Gly Ala Ala Gln
            100                 105                 110

Asn Arg Pro Leu Trp Ile Ile Phe Glu Arg Asp Met Val Ile Arg Leu
        115                 120                 125

Asp Arg Glu Leu Ala Ile Asn Asn Asp Lys Thr Ile Asp Gly Arg Gly
    130                 135                 140

Ala Lys Val Glu Ile Ile Asn Ala Gly Phe Ala Ile Tyr Asn Val Lys
145                 150                 155                 160

Asn Ile Ile Ile His Asn Ile Ile Met His Asp Ile Val Val Asn Pro
                165                 170                 175

Gly Gly Leu Ile Lys Ser His Asp Gly Pro Pro Val Pro Arg Lys Gly
            180                 185                 190

Ser Asp Gly Asp Ala Ile Gly Ile Ser Gly Gly Ser Gln Ile Trp Ile
        195                 200                 205

Asp His Cys Ser Leu Ser Lys Ala Val Asp Gly Leu Ile Asp Ala Lys
    210                 215                 220

His Gly Ser Thr His Phe Thr Val Ser Asn Cys Leu Phe Thr Gln His
225                 230                 235                 240

Gln Tyr Leu Leu Leu Phe Trp Asp Phe Asp Glu Arg Gly Met Leu Cys
                245                 250                 255

Thr Val Ala Phe Asn Lys Phe Thr Asp Asn Val Asp Gln Arg Met Pro
            260                 265                 270

Asn Leu Arg His Gly Phe Val Gln Val Val Asn Asn Asn Tyr Glu Arg
        275                 280                 285

Trp Gly Ser Tyr Ala Leu Gly Gly Ser Ala Gly Pro Thr Ile Leu Ser
    290                 295                 300

Gln Gly Asn Arg Phe Leu Ala Ser Asp Ile Lys Lys Glu Val Val Gly
305                 310                 315                 320

Arg Tyr Gly Glu Ser Ala Met Ser Glu Ser Ile Asn Trp Asn Trp Arg
                325                 330                 335

Ser Tyr Met Asp Val Phe Glu Asn Gly Ala Ile Phe Val Pro Ser Gly
            340                 345                 350

Val Asp Pro Val Leu Thr Pro Glu Gln Asn Ala Gly Met Ile Pro Ala
        355                 360                 365

Glu Pro Gly Glu Ala Val Leu Arg Leu Thr Ser Ser Ala Gly Val Leu
    370                 375                 380

Ser Cys Gln Pro Gly Ala Pro Cys
385                 390

(2) INFORMATION FOR SEQ ID NO:9:

(i) SEQUENCE CHARACTERISTICS:
(A) LENGTH: 1395 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..1191

(xi) SEQUENCE DESCRIPTION: SEQ ID NO:9:

ATG GGG ATC AAA CAT TGT TGT TAC ATC TTG TAT TTT ACC TTA GCA CTT 48
Met Gly Ile Lys His Cys Cys Tyr Ile Leu Tyr Phe Thr Leu Ala Leu
1               5                   10                  15

GTC ACT TTG GTG CAA GCT GGA CGT CTT GGC GAA GAG GTC GAC ATC TTA 96
Val Thr Leu Val Gln Ala Gly Arg Leu Gly Glu Glu Val Asp Ile Leu
            20                  25                  30

CCT TCA CCT AAC GAT ACA AGG AGG AGC CTG CAA GGA TGT GAA GCA CAC 144
Pro Ser Pro Asn Asp Thr Arg Arg Ser Leu Gln Gly Cys Glu Ala His
        35                  40                  45

AAC ATT ATA GAC AAG TGT TGG AGG TGC AAA CCC GAT TGG GCG GAG AAC 192
Asn Ile Ile Asp Lys Cys Trp Arg Cys Lys Pro Asp Trp Ala Glu Asn
    50                  55                  60

CGA CAA GCG TTA GGC GAT TGT GCG CAA GGT TTT GGA AAG GCA ACT CAC 240
Arg Gln Ala Leu Gly Asp Cys Ala Gln Gly Phe Gly Lys Ala Thr His
65                  70                  75                  80

GGC GGA AAA TGG GGT GAT ATC TAC ATG GTC ACA AGT GAT CAG GAT GAT 288
Gly Gly Lys Trp Gly Asp Ile Tyr Met Val Thr Ser Asp Gln Asp Asp
                85                  90                  95

GAT GTT GTA AAT CCA AAA GAA GGC ACA CTC CGG TTC GGT GCT ACC CAG 336
Asp Val Val Asn Pro Lys Glu Gly Thr Leu Arg Phe Gly Ala Thr Gln
            100                 105                 110

GAC AGG CCC TTG TGG ATC ATT TTT CAA AGA GAT ATG ATT ATT TAT TTG 384
Asp Arg Pro Leu Trp Ile Ile Phe Gln Arg Asp Met Ile Ile Tyr Leu
        115                 120                 125

CAA CAA GAG ATG GTC GTA ACC AGC GAC ACC ACC ATT GAT GGT CGA GGG 432
Gln Gln Glu Met Val Val Thr Ser Asp Thr Thr Ile Asp Gly Arg Gly
    130                 135                 140

GCG AAA GTT GAG CTC GTT TAT GGA GGT ATC ACC CTC ATG AAT GTC AAG 480
Ala Lys Val Glu Leu Val Tyr Gly Gly Ile Thr Leu Met Asn Val Lys
145                 150                 155                 160

AAT GTA ATC ATT CAC AAC ATA GAT ATC CAT GAT GTT AGA GTG CTT CCA 528
Asn Val Ile Ile His Asn Ile Asp Ile His Asp Val Arg Val Leu Pro
                165                 170                 175

GGA GGT AGG ATT AAG TCC AAT GGT GGT CCA GCC ATA CCA AGA CAT CAG 576
Gly Gly Arg Ile Lys Ser Asn Gly Gly Pro Ala Ile Pro Arg His Gln
            180                 185                 190

AGT GAT GGT GAT GCT ATC CAT GTT ACG GGT AGT TCA GAC ATA TGG ATC 624
Ser Asp Gly Asp Ala Ile His Val Thr Gly Ser Ser Asp Ile Trp Ile
        195                 200                 205

GAC CAT TGC ACG CTC AGT AAG TCA TTT GAT GGG CTC GTC GAT GTC AAC 672
Asp His Cys Thr Leu Ser Lys Ser Phe Asp Gly Leu Val Asp Val Asn
    210                 215                 220

TGG GGC AGC ACA GGA GTA ACC ATT TCC AAC TGC AAA TTC ACC CAC CAC 720
Trp Gly Ser Thr Gly Val Thr Ile Ser Asn Cys Lys Phe Thr His His
225                 230                 235                 240

GAA AAA GCT GTT TTG CTC GGG GCT AGT GAC ACG CAT TTT CAA GAT CTG 768
Glu Lys Ala Val Leu Leu Gly Ala Ser Asp Thr His Phe Gln Asp Leu
                245                 250                 255

AAA ATG CAT GTA ACG CTT GCA TAC AAC ATC TTC ACC AAT ACC GTT CAC 816
Lys Met His Val Thr Leu Ala Tyr Asn Ile Phe Thr Asn Thr Val His
            260                 265                 270

GAA AGA ATG CCC AGA TGC CGA TTT GGG TTT TTC CAA ATC GTT AAC AAC 864
Glu Arg Met Pro Arg Cys Arg Phe Gly Phe Phe Gln Ile Val Asn Asn
        275                 280                 285

TTC TAC GAC AGA TGG GAT AAG TAC GCC ATC GGT GGT AGC TCG AAC CCT 912
Phe Tyr Asp Arg Trp Asp Lys Tyr Ala Ile Gly Gly Ser Ser Asn Pro
    290                 295                 300

ACT ATT CTC AGC CAA GGG AAC AAA TTC GTG GCC CCC GAT TTC ATT TAC 960
Thr Ile Leu Ser Gln Gly Asn Lys Phe Val Ala Pro Asp Phe Ile Tyr
305                 310                 315                 320

AAG AAA AAC GTC TGT CTA AGG ACT GGT GCA CAG GAG CCA GAA TGG ATG 1008
Lys Lys Asn Val Cys Leu Arg Thr Gly Ala Gln Glu Pro Glu Trp Met
                325                 330                 335

ACT TGG AAC TGG AGA ACA CAA AAC GAC GTG CTT GAA AAT GGT GCT ATC 1056
Thr Trp Asn Trp Arg Thr Gln Asn Asp Val Leu Glu Asn Gly Ala Ile
            340                 345                 350

TTT GTG GCA TCT GGG TCT GAT CCA GTG CTA ACC GCT GAA CAA AAT GCA 1104
Phe Val Ala Ser Gly Ser Asp Pro Val Leu Thr Ala Glu Gln Asn Ala
        355                 360                 365

GGC ATG ATG CAA GCT GAA CCG GGA GAT ATG GTT CCA CAA CTC ACC ATG 1152
Gly Met Met Gln Ala Glu Pro Gly Asp Met Val Pro Gln Leu Thr Met
    370                 375                 380

AAT GCA GGT GTA CTC ACA TGC TCG CCT GGA GCA CCT TGC TAAGCACCTG 1201
Asn Ala Gly Val Leu Thr Cys Ser Pro Gly Ala Pro Cys
385                 390                 395

GCCAATTCCT ATGCAACGAT CATAAATACT TGCTCACCAT AAGTGTTCAT TTGATTAGAT 1261

TTGGACACGA ATGATGTAAC CGATTCGTCT GAATTATGAT TTGTTTTGAT TCTCAGTTTC 1321

ATAATATGGC TTCTTGAGAG CAAAATTAGA GAAGAGTGTC TTTGATCAAC TACATTTTAT 1381

GGTTTTTATA TTAA 1395

(2) INFORMATION FOR SEQ ID NO:10:

(i) SEQUENCE CHARACTERISTICS:
(A) LENGTH: 397 amino acids
(B) TYPE: amino acid
(D) TOPOLOGY: linear

(ii) MOLECULE TYPE: protein

(xi) SEQUENCE DESCRIPTION: SEQ ID NO:10:

Met Gly Ile Lys His Cys Cys Tyr Ile Leu Tyr Phe Thr Leu Ala Leu
1               5                   10                  15

Val Thr Leu Val Gln Ala Gly Arg Leu Gly Glu Glu Val Asp Ile Leu
            20                  25                  30

Pro Ser Pro Asn Asp Thr Arg Arg Ser Leu Gln Gly Cys Glu Ala His
        35                  40                  45

Asn Ile Ile Asp Lys Cys Trp Arg Cys Lys Pro Asp Trp Ala Glu Asn
    50                  55                  60

Arg Gln Ala Leu Gly Asp Cys Ala Gln Gly Phe Gly Lys Ala Thr His
65                  70                  75                  80

Gly Gly Lys Trp Gly Asp Ile Tyr Met Val Thr Ser Asp Gln Asp Asp
                85                  90                  95

Asp Val Val Asn Pro Lys Glu Gly Thr Leu Arg Phe Gly Ala Thr Gln
            100                 105                 110

Asp Arg Pro Leu Trp Ile Ile Phe Gln Arg Asp Met Ile Ile Tyr Leu
        115                 120                 125

Gln Gln Glu Met Val Val Thr Ser Asp Thr Thr Ile Asp Gly Arg Gly
    130                 135                 140

Ala Lys Val Glu Leu Val Tyr Gly Gly Ile Thr Leu Met Asn Val Lys
145                 150                 155                 160

Asn Val Ile Ile His Asn Ile Asp Ile His Asp Val Arg Val Leu Pro
                165                 170                 175

Gly Gly Arg Ile Lys Ser Asn Gly Gly Pro Ala Ile Pro Arg His Gln
            180                 185                 190

Ser Asp Gly Asp Ala Ile His Val Thr Gly Ser Ser Asp Ile Trp Ile
        195                 200                 205

Asp His Cys Thr Leu Ser Lys Ser Phe Asp Gly Leu Val Asp Val Asn
    210                 215                 220

Trp Gly Ser Thr Gly Val Thr Ile Ser Asn Cys Lys Phe Thr His His
225                 230                 235                 240

Glu Lys Ala Val Leu Leu Gly Ala Ser Asp Thr His Phe Gln Asp Leu
                245                 250                 255

Lys Met His Val Thr Leu Ala Tyr Asn Ile Phe Thr Asn Thr Val His
            260                 265                 270

Glu Arg Met Pro Arg Cys Arg Phe Gly Phe Phe Gln Ile Val Asn Asn
        275                 280                 285

Phe Tyr Asp Arg Trp Asp Lys Tyr Ala Ile Gly Gly Ser Ser Asn Pro
    290                 295                 300

Thr Ile Leu Ser Gln Gly Asn Lys Phe Val Ala Pro Asp Phe Ile Tyr
305                 310                 315                 320

Lys Lys Asn Val Cys Leu Arg Thr Gly Ala Gln Glu Pro Glu Trp Met
                325                 330                 335

Thr Trp Asn Trp Arg Thr Gln Asn Asp Val Leu Glu Asn Gly Ala Ile
            340                 345                 350

Phe Val Ala Ser Gly Ser Asp Pro Val Leu Thr Ala Glu Gln Asn Ala
        355                 360                 365

Gly Met Met Gln Ala Glu Pro Gly Asp Met Val Pro Gln Leu Thr Met
    370                 375                 380

Asn Ala Gly Val Leu Thr Cys Ser Pro Gly Ala Pro Cys
385                 390                 395

(2) INFORMATION FOR SEQ ID NO:11:

(i) SEQUENCE CHARACTERISTICS:
(A) LENGTH: 312 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..276

(xi) SEQUENCE DESCRIPTION: SEQ ID NO:11:

ATG TCG ATT CTT GGA GGA ATT ACC GAA GTT AAA GAC AAT GAT AAC AGC 48
Met Ser Ile Leu Gly Gly Ile Thr Glu Val Lys Asp Asn Asp Asn Ser
1               5                   10                  15

GTC GAT TTC GAC GAG CTT GCT AAA TTC GCC ATC GCT GAA CAC AAC AAG 96
Val Asp Phe Asp Glu Leu Ala Lys Phe Ala Ile Ala Glu His Asn Lys
            20                  25                  30

AAG GAG AAT GCT GCT CTG GAG TTT GGA AAA GTA ATA GAA AAA AAG CAG 144
Lys Glu Asn Ala Ala Leu Glu Phe Gly Lys Val Ile Glu Lys Lys Gln
        35                  40                  45

CAG GCG GTA CAG GGC ACC ATG TAT TAT ATA AAA GTG GAA GCA AAT GAT 192
Gln Ala Val Gln Gly Thr Met Tyr Tyr Ile Lys Val Glu Ala Asn Asp
    50                  55                  60

GGT GGT GAG AAG AAA ACT TAT GAA GCC AAG GTG TGG GTT AAG CTA TGG 240
Gly Gly Glu Lys Lys Thr Tyr Glu Ala Lys Val Trp Val Lys Leu Trp
65                  70                  75                  80

GAA AAT TTC AAG GAA TTG CAG GAA CTC AAA CTT GTT TGATGTTGCC 286
Glu Asn Phe Lys Glu Leu Gln Glu Leu Lys Leu Val
                85                  90

ACCTCACCTTA ACTCCATATG GACGG 312

(2) INFORMATION FOR SEQ ID NO:12:

(i) SEQUENCE CHARACTERISTICS:
(A) LENGTH: 92 amino acids
(B) TYPE: amino acid
(D) TOPOLOGY: linear

(ii) MOLECULE TYPE: protein

(xi) SEQUENCE DESCRIPTION: SEQ ID NO:12:

Met Ser Ile Leu Gly Gly Ile Thr Glu Val Lys Asp Asn Asp Asn Ser
1               5                   10                  15

Val Asp Phe Asp Glu Leu Ala Lys Phe Ala Ile Ala Glu His Asn Lys
            20                  25                  30

Lys Glu Asn Ala Ala Leu Glu Phe Gly Lys Val Ile Glu Lys Lys Gln
        35                  40                  45

Gln Ala Val Gln Gly Thr Met Tyr Tyr Ile Lys Val Glu Ala Asn Asp
    50                  55                  60

Gly Gly Glu Lys Lys Thr Tyr Glu Ala Lys Val Trp Val Lys Leu Trp
65                  70                  75                  80

Glu Asn Phe Lys Glu Leu Gln Glu Leu Lys Leu Val
                85                  90

(2) INFORMATION FOR SEQ ID NO:13:

(i) SEQUENCE CHARACTERISTICS:
(A) LENGTH: 27 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:13:

Tyr Ile Leu Tyr Phe Thr Leu Ala Leu Val Thr Leu Leu Gln Pro Val
1               5                   10                  15

Arg Ser Ala Glu Asp Leu Gln Glu Ile Leu Pro
            20                  25

(2) INFORMATION FOR SEQ ID NO:14:

(i) SEQUENCE CHARACTERISTICS:
(A) LENGTH: 28 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:14:

Pro Val Arg Ser Ala Glu Asp Leu Gln Glu Ile Leu Pro Val Asn Glu
1               5                   10                  15

Thr Arg Arg Leu Thr Thr Ser Gly Ala Tyr Asn Ile
            20                  25

(2) INFORMATION FOR SEQ ID NO:15:

(i) SEQUENCE CHARACTERISTICS:
(A) LENGTH: 24 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:15:

Glu Thr Arg Arg Ser Leu Lys Thr Ser Gly Ala Tyr Asn Ile Ile Asp
1               5                   10                  15

Gly Cys Trp Arg Gly Lys Ala Asp
            20

(2) INFORMATION FOR SEQ ID NO:16:

    (i) SEQUENCE CHARACTERISTICS:
        (A) LENGTH: 23 amino acids
        (B) TYPE: amino acid
        (D) TOPOLOGY: linear

    (ii) MOLECULE TYPE: peptide

    (v) FRAGMENT TYPE: internal

    (xi) SEQUENCE DESCRIPTION: SEQ ID NO:16:

    Glu Thr Arg Arg Leu Thr Thr Ser Gly Ala Tyr Asn Ile Ile Asp Gly
    1               5                   10                  15

    Cys Trp Arg Gly Lys Ala Asp
                20

(2) INFORMATION FOR SEQ ID NO:17:

    (i) SEQUENCE CHARACTERISTICS:
        (A) LENGTH: 28 amino acids
        (B) TYPE: amino acid
        (D) TOPOLOGY: linear

    (ii) MOLECULE TYPE: peptide

    (v) FRAGMENT TYPE: internal

    (xi) SEQUENCE DESCRIPTION: SEQ ID NO:17:

    Ala Tyr Asn Ile Ile Asp Gly Cys Trp Arg Gly Lys Ala Asp Trp Ala
    1               5                   10                  15

    Glu Asn Arg Lys Ala Leu Ala Asp Cys Ala Gln Gly
                20                  25

(2) INFORMATION FOR SEQ ID NO:18:

(i) SEQUENCE CHARACTERISTICS:
(A) LENGTH: 19 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:18:

Arg Gly Lys Ala Asp Trp Ala Glu Asn Arg Lys Ala Leu Ala Asp Cys
1               5                   10                  15

Ala Gln Gly

(2) INFORMATION FOR SEQ ID NO:19:

(i) SEQUENCE CHARACTERISTICS:
(A) LENGTH: 10 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:19:

Gly Lys Ala Asp Trp Ala Glu Asn Arg Cys
1               5                   10

(2) INFORMATION FOR SEQ ID NO:20:

(i) SEQUENCE CHARACTERISTICS:
(A) LENGTH: 10 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:20:

Gly Lys Ala Asp Trp Ala Glu Asn Arg Lys
1               5                   10

(2) INFORMATION FOR SEQ ID NO:21:

(i) SEQUENCE CHARACTERISTICS:
(A) LENGTH: 13 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:21:

Ala Glu Asn Arg Lys Ala Leu Ala Asp Cys Ala Gln Gly
1               5                   10

(2) INFORMATION FOR SEQ ID NO:22:

(i) SEQUENCE CHARACTERISTICS:
(A) LENGTH: 17 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:22:

Lys Ala Leu Ala Asp Cys Ala Gln Gly Phe Gly Lys Gly Thr Val Gly
1               5                   10                  15

Gly

(2) INFORMATION FOR SEQ ID NO:23:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:23:

Gly Phe Gly Lys Gly Thr Val Gly Gly Lys Asp Gly Asp Ile Tyr Ser
1               5                   10                  15

Val Thr

(2) INFORMATION FOR SEQ ID NO:24:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:24:

Gly Phe Gly Lys Gly Thr Val Gly Gly Lys Asp Gly Asp Ile Tyr Thr
1               5                   10                  15

Val Thr

(2) INFORMATION FOR SEQ ID NO:25:

(i) SEQUENCE CHARACTERISTICS:
(A) LENGTH: 17 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:25:

Lys Asp Gly Asp Ile Tyr Ser Val Thr Ser Glu Leu Asp Asp Asp Val
1               5                   10                  15

Ala

(2) INFORMATION FOR SEQ ID NO:26:

(i) SEQUENCE CHARACTERISTICS:
(A) LENGTH: 17 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:26:

Lys Asp Gly Asp Ile Tyr Thr Val Thr Ser Glu Leu Asp Asp Asp Val
1               5                   10                  15

Ala

([?]) INFORMATION FOR SEQ ID NO:27:

(i) SEQUENCE CHARACTERISTICS:
(A) LENGTH: 11 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:27:

Ile Tyr Thr Val Thr Ser Glu Leu Asp Asp Asp
1               5                   10

(2) INFORMATION FOR SEQ ID NO:28:

(i) SEQUENCE CHARACTERISTICS:
(A) LENGTH: 14 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:28:

Ile Tyr Thr Val Thr Ser Glu Leu Asp Asp Asp Val Ala Asn
1               5                   10

(2) INFORMATION FOR SEQ ID NO:29:

(i) SEQUENCE CHARACTERISTICS:
(A) LENGTH: 17 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:29:

Ile Tyr Thr Val Thr Ser Glu Leu Asp Asp Asp Val Ala Asn Pro Lys
1               5                   10                  15

Glu

(2) INFORMATION FOR SEQ ID NO:30:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:30:

Ile Tyr Ser Val Thr Ser Glu Leu Asp Asp Asp Val Ala Asn Pro Lys
1               5                   10                  15

Glu Gly Thr Leu
            20

(2) INFORMATION FOR SEQ ID NO:31:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:31:

Ile Tyr Thr Val Thr Ser Glu Leu Asp Asp Asp Val Ala Asn Pro Lys
1               5                   10                  15

Glu Gly Thr Leu
            20

(2) INFORMATION FOR SEQ ID NO:32:

(i) SEQUENCE CHARACTERISTICS:
(A) LENGTH: 29 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:32:

Asn Pro Lys Glu Gly Thr Leu Arg Phe Gly Ala Ala Gln Asn Arg Pro
1               5                   10                  15

Leu Trp Ile Ile Phe Glu Arg Asp Met Val Ile Arg Leu
            20                  25

(2) INFORMATION FOR SEQ ID NO:33:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:33:

Trp Ile Ile Phe Glu Arg Asp Met Val Ile Arg Leu Asp Lys Glu Met
1               5                   10                  15

Val Val Asn Ser
            20

(2) INFORMATION FOR SEQ ID NO:34:

(i) SEQUENCE CHARACTERISTICS:
(A) LENGTH: 19 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:34:

Leu Asp Lys Glu Met Val Val Asn Ser Asp Lys Thr Ile Asp Gly Arg
1               5                   10                  15

Gly Ala Lys

(2) INFORMATION FOR SEQ ID NO:35:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:35:

Asp Lys Thr Ile Asp Gly Arg Gly Ala Lys Val Glu Ile Ile Asn Ala
1               5                   10                  15

Gly Phe Thr Leu Asn Gly Val Lys Asn Val
            20                  25

(2) INFORMATION FOR SEQ ID NO:36:

(i) SEQUENCE CHARACTERISTICS:
(A) LENGTH: 27 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:36:

Gly Phe Thr Leu Asn Gly Val Lys Asn Val Ile Ile His Asn Ile Asn
1               5                   10                  15

Met His Asp Val Lys Val Asn Pro Gly Gly Leu
            20                  25

(2) INFORMATION FOR SEQ ID NO:37:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:37:

His Asp Val Lys Val Asn Pro Gly Gly Leu Ile Lys Ser Asn Asp Gly
1               5                   10                  15

Pro Ala Ala Pro Arg Ala Gly Ser Asp Gly Asp Ala
            20                  25

(2) INFORMATION FOR SEQ ID NO:38:

(i) SEQUENCE CHARACTERISTICS:
(A) LENGTH: 23 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:38:

Asn Pro Gly Gly Leu Ile Lys Ser Asn Asp Gly Pro Ala Ala Pro Arg
1               5                   10                  15

Ala Gly Ser Asp Gly Asp Ala
            20

(2) INFORMATION FOR SEQ ID NO:39:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:30:

Ile Lys Ser Asn Asp Gly Pro Ala Ala Pro Arg Ala Gly Ser Asp Gly
1               5                   10                  15

Asp Ala

(2) INFORMATION FOR SEQ ID NO:40:

(i) SEQUENCE CHARACTERISTICS:
(A) LENGTH: 13 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:40:

Gly Pro Ala Ala Pro Arg Ala Gly Ser Asp Gly Asp Ala
1               5                   10

(2) INFORMATION FOR SEQ ID NO:41:

(i) SEQUENCE CHARACTERISTICS:
(A) LENGTH: 13 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:41:

Ala Pro Arg Ala Gly Ser Asp Gly Asp Ala Ile Ser Ile
1               5                   10

(2) INFORMATION FOR SEQ ID NO:42:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:42:

Ala Pro Arg Ala Gly Ser Asp Gly Asp Ala Ile Ser Ile Ser Gly Ser
1               5                   10                  15

Ser Gln

(2) INFORMATION FOR SEQ ID NO:43:

(i) SEQUENCE CHARACTERISTICS:
(A) LENGTH: 23 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:43:

Ala Pro Arg Ala Gly Ser Asp Gly Asp Ala Ile Ser Ile Ser Gly Ser
1               5                   10                  15

Ser Gln Ile Trp Ile Asp His
            20

(2) INFORMATION FOR SEQ ID NO:44:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:44:

Ala Pro Arg Ala Gly Ser Asp Gly Asp Ala Ile Ser Ile Ser Gly Ser
1               5                   10                  15

Ser Gln Ile Trp Ile Asp His Cys Ser Leu Ser Lys
            20                  25

(2) INFORMATION FOR SEQ ID NO:45:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:45:

Trp Ile Asp His Cys Ser Leu Ser Lys Ser Val Asp Gly Leu Val Asp
1               5                   10                  15

Ala Lys Leu Gly Thr Thr Arg Leu Thr Val Ser Asn
            20                  25

(2) INFORMATION FOR SEQ ID NO:46:

(i) SEQUENCE CHARACTERISTICS:
(A) LENGTH: 27 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:46:

Leu Gly Thr Thr Arg Leu Thr Val Ser Asn Ser Leu Phe Thr Gln His
1               5                   10                  15

Gln Phe Val Leu Leu Phe Gly Ala Gly Asp Glu
            20                  25

(2) INFORMATION FOR SEQ ID NO:47:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:47:

Phe Val Leu Leu Phe Gly Ala Gly Asp Glu Asn Ile Glu Asp Arg Gly
1               5                   10                  15

Met Leu Ala Thr Val Ala Phe Asn Thr Phe Thr Asp
            20                  25

(2) INFORMATION FOR SEQ ID NO:48:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:48:

Leu Ala Thr Val Ala Phe Asn Thr Phe Thr Asp Asn Val Asp Gln Arg
1               5                   10                  15

Met Pro

(2) INFORMATION FOR SEQ ID NO:49:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:49:

Phe Thr Asp Asn Val Asp Gln Arg Met Pro Arg Cys Arg His Gly Phe
1               5                   10                  15

Phe Gln Val Val
            20

(2) INFORMATION FOR SEQ ID NO:50:

    (i) SEQUENCE CHARACTERISTICS:
        (A) LENGTH: 28 amino acids
        (B) TYPE: amino acid
        (D) TOPOLOGY: linear

    (ii) MOLECULE TYPE: peptide

    (v) FRAGMENT TYPE: internal

    (xi) SEQUENCE DESCRIPTION: SEQ ID NO:50:

Arg Cys Arg His Gly Phe Phe Gln Val Val Asn Asn Asn Tyr Asp Lys
1               5                   10                  15

Trp Gly Ser Tyr Ala Ile Gly Gly Ser Ala Ser Pro
            20                  25

(2) INFORMATION FOR SEQ ID NO:51:

    (i) SEQUENCE CHARACTERISTICS:
        (A) LENGTH: 25 amino acids
        (B) TYPE: amino acid
        (D) TOPOLOGY: linear

    (ii) MOLECULE TYPE: peptide

    (v) FRAGMENT TYPE: internal

    (xi) SEQUENCE DESCRIPTION: SEQ ID NO:51:

His Gly Phe Phe Gln Val Val Asn Asn Asn Tyr Asp Lys Trp Gly Ser
1               5                   10                  15

Tyr Ala Ile Gly Gly Ser Ala Ser Pro
            20                  25

(2) INFORMATION FOR SEQ ID NO:52:

(i) SEQUENCE CHARACTERISTICS:
(A) LENGTH: 22 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:52:

Phe Gln Val Val Asn Asn Asn Tyr Asp Lys Trp Gly Ser Tyr Ala Ile
1               5                   10                  15

Gly Gly Ser Ala Ser Pro
            20

(2) INFORMATION FOR SEQ ID NO:53:

(i) SEQUENCE CHARACTERISTICS:
(A) LENGTH: 19 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:53:

Val Asn Asn Asn Tyr Asp Lys Trp Gly Ser Tyr Ala Ile Gly Gly Ser
1               5                   10                  15

Ala Ser Pro

(2) INFORMATION FOR SEQ ID NO:54:

(i) SEQUENCE CHARACTERISTICS:
(A) LENGTH: 12 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:54:

Gly Ser Tyr Ala Ile Gly Gly Ser Ala Ser Pro Thr
1               5                   10

(2) INFORMATION FOR SEQ ID NO:55:

(i) SEQUENCE CHARACTERISTICS:
(A) LENGTH: 16 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:55:

Gly Ser Tyr Ala Ile Gly Gly Ser Ala Ser Pro Thr Ile Leu Ser Gln
1               5                   10                  15

(2) INFORMATION FOR SEQ ID NO:56:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:56:

Gly Ser Tyr Ala Ile Gly Gly Ser Ala Ser Pro Thr Ile Leu Ser Gln
1               5                   10                  15

Gly Asn Arg Phe
            20

(2) INFORMATION FOR SEQ ID NO:57:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:57:

Gly Ser Tyr Ala Ile Gly Gly Ser Ala Ser Pro Thr Ile Leu Ser Gln
1               5                   10                  15

Gly Asn Arg Phe Cys Ala Pro Asp Glu Arg Ser Lys
            20                  25

(2) INFORMATION FOR SEQ ID NO:58:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:58:

Arg Phe Cys Ala Pro Asp Glu Arg Ser Lys Lys Asn Val Leu Gly Arg
1               5                   10                  15

His Gly Glu Ala Ala Ala Glu Ser Met Lys Trp Asn
            20                  25

(2) INFORMATION FOR SEQ ID NO:59:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:59:

Glu Ala Ala Ala Glu Ser Met Lys Trp Asn Trp Arg Thr Asn Lys Asp
1               5                   10                  15

Val Leu Glu Asn Gly Ala Ile Phe Val Ala
            20                  25

(2) INFORMATION FOR SEQ ID NO:60:

(i) SEQUENCE CHARACTERISTICS:
(A) LENGTH: 23 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:60:

Glu Ser Met Lys Trp Asn Trp Arg Thr Asn Lys Asp Val Leu Glu Asn
1               5                   10                  15

Gly Ala Ile Phe Val Ala Ser
            20

(2) INFORMATION FOR SEQ ID NO:61:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:61:

Glu Phe Gly Trp Arg Thr Asn Lys Asp Val Leu Glu Asn Gly Ala Ile
1               5                   10                  15

Phe Val Ala Ser
            20

(2) INFORMATION FOR SEQ ID NO:62:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:62:

Lys Trp Asn Trp Arg Thr Asn Lys Asp Val Leu Glu Asn Gly Ala Ile
1               5                   10                  15

Phe Val Ala Ser
            20

(2) INFORMATION FOR SEQ ID NO:63:

(i) SEQUENCE CHARACTERISTICS:
(A) LENGTH: 30 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:63:

Lys Trp Asn Trp Arg Thr Asn Lys Asp Val Leu Glu Asn Gly Ala Ile
1               5                   10                  15

Phe Val Ala Ser Gly Val Asp Pro Val Leu Thr Pro Glu Gln
            20                  25                  30

(2) INFORMATION FOR SEQ ID NO:64:

(i) SEQUENCE CHARACTERISTICS:
(A) LENGTH: 17 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:64:

Trp Arg Thr Asn Lys Asp Val Leu Glu Asn Gly Ala Ile Phe Val Ala
1               5                   10                  15

Ser

(2) INFORMATION FOR SEQ ID NO:65:

(i) SEQUENCE CHARACTERISTICS:
(A) LENGTH: 14 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:65:

Asn Lys Asp Val Leu Glu Asn Gly Ala Ile Phe Val Ala Ser
1               5                   10

(2) INFORMATION FOR SEQ ID NO:66:

(i) SEQUENCE CHARACTERISTICS:
(A) LENGTH: 11 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:66:

Val Leu Glu Asn Gly Ala Ile Phe Val Ala Ser
1               5                   10

(2) INFORMATION FOR SEQ ID NO:67:

(i) SEQUENCE CHARACTERISTICS:
(A) LENGTH: 11 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:67:

Leu Glu Asn Gly Ala Ile Phe Val Ala Ser Gly
1               5                   10

(2) INFORMATION FOR SEQ ID NO:68:

(i) SEQUENCE CHARACTERISTICS:
(A) LENGTH: 14 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:68:

Leu Glu Asn Gly Ala Ile Phe Val Ala Ser Gly Val Asp Pro
1               5                   10

(2) INFORMATION FOR SEQ ID NO:69:

(i) SEQUENCE CHARACTERISTICS:
(A) LENGTH: 17 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:69:

Leu Glu Asn Gly Ala Ile Phe Val Ala Ser Gly Val Asp Pro Val Leu
1               5                   10                  15

Thr

(2) INFORMATION FOR SEQ ID NO:70:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:70:

Leu Glu Asn Gly Ala Ile Phe Val Ala Ser Gly Val Asp Pro Val Leu
1               5                   10                  15

Thr Pro Glu Gln
            20

(2) INFORMATION FOR SEQ ID NO:71:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:71:

Gly Val Asp Pro Val Leu Thr Pro Glu Gln Ser Ala Gly Met Ile Pro
1               5                   10                  15

Ala Glu Pro Gly
            20

(2) INFORMATION FOR SEQ ID NO:72:

(i) SEQUENCE CHARACTERISTICS:
(A) LENGTH: 9 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:72:

Gly Val Asp Pro Val Leu Thr Pro Glu
1               5

(2) INFORMATION FOR SEQ ID NO:73:

(i) SEQUENCE CHARACTERISTICS:
(A) LENGTH: 12 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:73:

Gly Val Asp Pro Val Leu Thr Pro Glu Gln Ser Ala
1               5                   10

(2) INFORMATION FOR SEQ ID NO:74:

(i) SEQUENCE CHARACTERISTICS:
(A) LENGTH: 16 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:74:

Gly Val Asp Pro Val Leu Thr Pro Glu Gln Ser Ala Gly Met Ile Pro
1               5                   10                  15

(2) INFORMATION FOR SEQ ID NO:75:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:75:

Ser Ala Gly Met Ile Pro Ala Glu Pro Gly Glu Ser Ala Leu Ser Leu
1               5                   10                  15

Thr Ser Ser Ala
            20

(2) INFORMATION FOR SEQ ID NO:76:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:76:

Glu Pro Gly Glu Ser Ala Leu Ser Leu Thr Ser Ser Ala Gly Val Leu
1               5                   10                  15

Ser Cys

(2) INFORMATION FOR SEQ ID NO:77:

(i) SEQUENCE CHARACTERISTICS:
(A) LENGTH: 15 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:77:

Thr Ser Ser Ala Gly Val Leu Ser Cys Gln Pro Gly Ala Pro Cys
1               5                   10                  15

(2) INFORMATION FOR SEQ ID NO:78:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:78:

Leu Ala Thr Val Ala Phe Asn Met Phe Thr Asp His Val Asp Gln Arg
1               5                   10                  15

Met Pro

(2) INFORMATION FOR SEQ ID NO:79:

(i) SEQUENCE CHARACTERISTICS:
(A) LENGTH: 27 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:79:

Tyr Ile Leu Tyr Phe Thr Leu Ala Leu Val Ala Leu Leu Gln Pro Val
1               5                   10                  15

Arg Ser Ala Glu Gly Val Gly Glu Ile Leu Pro
            20                  25

(2) INFORMATION FOR SEQ ID NO:80:

(i) SEQUENCE CHARACTERISTICS:
(A) LENGTH: 19 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:80:

Leu Asn Gln Glu Leu Val Val Asn Ser Asp Lys Thr Ile Asp Gly Arg
1               5                   10                  15

Gly Val Lys

(2) INFORMATION FOR SEQ ID NO:81:

(i) SEQUENCE CHARACTERISTICS:
(A) LENGTH: 21 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:81:

Gly Met Leu Ala Thr Val Ala Phe Asn Met Phe Thr Asp Asn Val Asp
1               5                   10                  15

Gln Arg Met Pro Arg
            20

(2) INFORMATION FOR SEQ ID NO:82:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:82:

Arg Gly Lys Ala Asp Trp Ala Glu Asn Arg Lys Ala Leu Ala Asp Cys
1               5                   10                  15

Ala Gln Gly Phe Gly Lys Gly Thr Val Gly Gly Lys
            20                  25

(2) INFORMATION FOR SEQ ID NO:83:

(i) SEQUENCE CHARACTERISTICS:
(A) LENGTH: 22 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:83:

Ala Glu Asn Arg Lys Ala Leu Ala Asp Cys Ala Gln Gly Phe Gly Lys
1               5                   10                  15

Gly Thr Val Gly Gly Lys
            20

(2) INFORMATION FOR SEQ ID NO:84:

(i) SEQUENCE CHARACTERISTICS:
(A) LENGTH: 25 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:84:

Ala Glu Asn Arg Lys Ala Leu Ala Asp Cys Ala Gln Gly Phe Gly Lys
1               5                   10                  15

Gly Thr Val Gly Gly Lys Asp Gly Asp
            20                  25

(2) INFORMATION FOR SEQ ID NO:85:

(i) SEQUENCE CHARACTERISTICS:
(A) LENGTH: 27 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:85:

Gly Phe Gly Lys Gly Thr Val Gly Gly Lys Asp Gly Asp Ile Tyr Thr
1               5                   10                  15

Val Thr Ser Glu Leu Asp Asp Asp Val Ala Asn
            20                  25

(2) INFORMATION FOR SEQ ID NO:86:

(i) SEQUENCE CHARACTERISTICS:
(A) LENGTH: 24 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:86:

Lys Gly Thr Val Gly Gly Lys Asp Gly Asp Ile Tyr Thr Val Thr Ser
1               5                   10                  15

Glu Leu Asp Asp Asp Val Ala Asn
            20

(2) INFORMATION FOR SEQ ID NO:87:

(i) SEQUENCE CHARACTERISTICS:
(A) LENGTH: 19 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:87:

Gly Lys Asp Gly Asp Ile Tyr Thr Val Thr Ser Glu Leu Asp Asp Asp
1               5                   10                  15

Val Ala Asn

(2) INFORMATION FOR SEQ ID NO:88:

(i) SEQUENCE CHARACTERISTICS:
(A) LENGTH: 25 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:88:

Glu Asn Asn Arg Gln Ala Leu Ala Asp Cys Ala Gln Gly Phe Ala Lys
1               5                   10                  15

Gly Thr Tyr Gly Gly Lys Trp Gly Asp
            20                  25

(2) INFORMATION FOR SEQ ID NO:89:

(i) SEQUENCE CHARACTERISTICS:
(A) LENGTH: 24 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:89:

Lys Gly Thr Tyr Gly Gly Lys Trp Gly Asp Val Tyr Thr Val Thr Ser
1               5                   10                  15

Asn Leu Asp Asp Asp Val Ala Asn
            20

(2) INFORMATION FOR SEQ ID NO:90:

(i) SEQUENCE CHARACTERISTICS:
(A) LENGTH: 22 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:90:

Lys Ser Asn Asp Gly Pro Ala Ala Pro Arg Ala Gly Ser Asp Gly Asp
1               5                   10                  15

Ala Ile Ser Ile Ser Gly
            20

(2) INFORMATION FOR SEQ ID NO:91:

(i) SEQUENCE CHARACTERISTICS:
(A) LENGTH: 25 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:91:

Lys Ser Asn Asp Gly Pro Ala Ala Pro Arg Ala Gly Ser Asp Gly Asp
1               5                   10                  15

Ala Ile Ser Ile Ser Gly Ser Ser Gln
            20                  25

(2) INFORMATION FOR SEQ ID NO:92:

(i) SEQUENCE CHARACTERISTICS:
(A) LENGTH: 30 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:92:

Lys Ser Asn Asp Gly Pro Ala Ala Pro Arg Ala Gly Ser Asp Gly Asp
1               5                   10                  15

Ala Ile Ser Ile Ser Gly Ser Ser Gln Ile Trp Ile Asp His
            20                  25                  30

(2) INFORMATION FOR SEQ ID NO:93:

(i) SEQUENCE CHARACTERISTICS:
(A) LENGTH: 35 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:93:

Lys Ser Asn Asp Gly Pro Ala Ala Pro Arg Ala Gly Ser Asp Gly Asp
1               5                   10                  15

Ala Ile Ser Ile Ser Gly Ser Ser Gln Ile Trp Ile Asp His Cys Ser
            20                  25                  30

Leu Ser Lys
        35

(2) INFORMATION FOR SEQ ID NO:94:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:94:

Gly Pro Ala Ala Pro Arg Ala Gly Ser Asp Gly Asp Ala Ile Ser Ile
1               5                   10                  15

Ser Gly

(2) INFORMATION FOR SEQ ID NO:95:

(i) SEQUENCE CHARACTERISTICS:
(A) LENGTH: 21 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:95:

Gly Pro Ala Ala Pro Arg Ala Gly Ser Asp Gly Asp Ala Ile Ser Ile
1               5                   10                  15

Ser Gly Ser Ser Gln
            20

(2) INFORMATION FOR SEQ ID NO:96:

    (i) SEQUENCE CHARACTERISTICS:
        (A) LENGTH: 27 amino acids
        (B) TYPE: amino acid
        (D) TOPOLOGY: linear

    (ii) MOLECULE TYPE: peptide

    (v) FRAGMENT TYPE: internal

    (xi) SEQUENCE DESCRIPTION: SEQ ID NO:96:

    Lys Lys Ala Pro Arg Ala Gly Ser Asp Gly Asp Ala Ile Ser Ile Ser
    1               5                   10                  15

    Gly Ser Ser Gln Ile Trp Ile Asp His Lys Lys
                20                  25

(2) INFORMATION FOR SEQ ID NO:97:

    (i) SEQUENCE CHARACTERISTICS:
        (A) LENGTH: 31 amino acids
        (B) TYPE: amino acid
        (D) TOPOLOGY: linear

    (ii) MOLECULE TYPE: peptide

    (v) FRAGMENT TYPE: internal

    (xi) SEQUENCE DESCRIPTION: SEQ ID NO:97:

    Lys Lys Ala Pro Arg Ala Gly Ser Asp Gly Asp Ala Ile Ser Ile Ser
    1               5                   10                  15

    Gly Ser Ser Gln Ile Trp Ile Asp His Cys Ser Leu Ser Lys Lys
                20                  25                  30

(2) INFORMATION FOR SEQ ID NO:98:

    (i) SEQUENCE CHARACTERISTICS:
        (A) LENGTH: 31 amino acids
        (B) TYPE: amino acid
        (D) TOPOLOGY: linear

(ii) MOLECULE TYPE: peptide

(v) FRAGMENT TYPE: internal

(xi) SEQUENCE DESCRIPTION: SEQ ID NO:98:

Lys Lys Ala Pro Arg Ala Gly Ser Asp Gly Asp Ala Ile Ser Ile Ser
1               5                   10                  15

Gly Ser Ser Gln Ile Trp Ile Asp His Ser Ser Leu Ser Lys Lys
            20                  25                  30

(2) INFORMATION FOR SEQ ID NO:99:

(i) SEQUENCE CHARACTERISTICS:
(A) LENGTH: 31 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:99:

Lys Lys Ala Pro Arg Ala Gly Ser Asp Gly Asp Ala Ile Ser Ile Ser
1               5                   10                  15

Gly Ser Ser Gln Ile Trp Ile Asp His Leu Ser Leu Ser Lys Lys
            20                  25                  30

(2) INFORMATION FOR SEQ ID NO:100:

(i) SEQUENCE CHARACTERISTICS:
(A) LENGTH: 31 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:100:

Lys Lys Ala Pro Arg Ala Gly Ser Asp Gly Asp Ala Ile Ser Ile Ser
1               5                   10                  15

Gly Ser Ser Gln Ile Trp Ile Asp His Glu Ser Leu Ser Lys Lys .
            20                  25                  30

(2) INFORMATION FOR SEQ ID NO:101:

(i) SEQUENCE CHARACTERISTICS:
(A) LENGTH: 21 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:101:

Gly Pro Pro Ile Leu Arg Gln Ala Ser Asp Gly Asp Thr Ile Asn Val
1               5                   10                  15

Ala Gly Ser Ser Gln
            20

(2) INFORMATION FOR SEQ ID NO:102:

(i) SEQUENCE CHARACTERISTICS:
(A) LENGTH: 27 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:102:

Arg His Gly Phe Phe Gln Val Val Asn Asn Asn Tyr Asp Lys Trp Gly
1               5                   10                  15

Ser Tyr Ala Ile Gly Gly Ser Ala Ser Pro Thr
            20                  25

(2) INFORMATION FOR SEQ ID NO:103:

(i) SEQUENCE CHARACTERISTICS:
(A) LENGTH: 24 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:103:

Phe Phe Gln Val Val Asn Asn Asn Tyr Asp Lys Trp Gly
1               5                   10

Ile Gly Gly Ser Ala Ser Pro Thr
            20

(2) INFORMATION FOR SEQ ID NO:104:

(i) SEQUENCE CHARACTERISTICS:
(A) LENGTH: 24 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:104:

Gly Ser Tyr Ala Ile Gly Gly Ser Ala Ser Pro Thr Ile
1               5                   10

Gly Asn Arg Phe Cys Ala Pro Asp
            20

(2) INFORMATION FOR SEQ ID NO:105:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:105:

Gly Ser Tyr Ala Ile Gly Gly Ser Ala Ser Pro Thr Ile Leu Ser Gln
1               5                   10                  15

Gly Asn Arg Phe Cys Ala Pro Asp Glu Arg
            20                  25

(2) INFORMATION FOR SEQ ID NO:106:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:106:

Gly Ser Tyr Ala Ile Gly Gly Ser Ala Ser Pro Thr Ile Leu Ser Gln
1               5                   10                  15

Gly Asn Arg Phe Cys Ala Pro Asp Glu Arg Ser Lys
            20                  25

(2) INFORMATION FOR SEQ ID NO:107:

(i) SEQUENCE CHARACTERISTICS:
(A) LENGTH: 24 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:107:

Phe Phe Gln Val Val Asn Asn Asn Tyr Asp Arg Trp Gly Thr Tyr Ala
1               5                   10                  15

Ile Gly Gly Ser Ser Ala Pro Thr
            20

(2) INFORMATION FOR SEQ ID NO:108:

(i) SEQUENCE CHARACTERISTICS:
(A) LENGTH: 28 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:108:

Gly Thr Tyr Ala Ile Gly Gly Ser Ser Ala Pro Thr Ile Leu Cys Gln
1               5                   10                  15

Gly Asn Arg Phe Leu Ala Pro Asp Asp Gln Ile Lys
            20                  25

(2) INFORMATION FOR SEQ ID NO:109:

(i) SEQUENCE CHARACTERISTICS:
(A) LENGTH: 29 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:109:

Lys Asp Val Leu Glu Asn Gly Ala Ile Phe Val Ala Ser Gly Val Asp
1               5                   10                  15

Pro Val Leu Thr Pro Glu Gln Ser Ala Gly Met Ile Pro
            20                  25

(2) INFORMATION FOR SEQ ID NO:110:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:110:

Lys Asp Val Leu Glu Asn Gly Ala Ile Phe Val Ala Ser Gly Val Asp
1               5                   10                  15

Pro Val Leu Thr Pro Glu Gln Ser Ala Gly
            20                  25

(2) INFORMATION FOR SEQ ID NO:111:

(i) SEQUENCE CHARACTERISTICS:
(A) LENGTH: 31 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:111:

Val Leu Glu Asn Gly Ala Ile Phe Val Ala Ser Gly Val Asp Pro Val
1               5                   10                  15

Leu Thr Pro Glu Gln Ser Ala Gly Met Ile Pro Ala Glu Pro Gly
            20                  25                  30

(2) INFORMATION FOR SEQ ID NO:112:

(i) SEQUENCE CHARACTERISTICS:
(A) LENGTH: 29 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:112:

Val Leu Glu Asn Gly Ala Ile Phe Val Ala Ser Gly Val Asp Pro Val
1               5                   10                  15

Leu Thr Pro Glu Gln Ser Ala Gly Met Ile Pro Ala Glu
            20                  25

(2) INFORMATION FOR SEQ ID NO:113:

(i) SEQUENCE CHARACTERISTICS:
(A) LENGTH: 27 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:113:

Val Leu Glu Asn Gly Ala Ile Phe Val Ala Ser Gly Val Asp Pro Val
1               5                   10                  15

Leu Thr Pro Glu Gln Ser Ala Gly Met Ile Pro
            20                  25

(2) INFORMATION FOR SEQ ID NO:114:

(i) SEQUENCE CHARACTERISTICS:
(A) LENGTH: 24 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:114:

Val Leu Glu Asn Gly Ala Ile Phe Val Ala Ser Gly Val Asp Pro Val
1               5                   10                  15

Leu Thr Pro Glu Gln Ser Ala Gly
            20

(2) INFORMATION FOR SEQ ID NO:115:

(i) SEQUENCE CHARACTERISTICS:
(A) LENGTH: 21 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:115:

Val Leu Glu Asn Gly Ala Ile Phe Val Ala Ser Gly Val Asp Pro Val
1               5                   10                  15

Leu Thr Pro Glu Gln
            20

(2) INFORMATION FOR SEQ ID NO:116:

(i) SEQUENCE CHARACTERISTICS:
(A) LENGTH: 27 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:116:

Leu Leu Glu Asn Gly Ala Ile Phe Val Thr Ser Gly Ser Asp Pro Val
1               5                   10                  15

Leu Thr Pro Val Gln Ser Ala Gly Met Ile Pro
            20                  25

(2) INFORMATION FOR SEQ ID NO:117:

(i) SEQUENCE CHARACTERISTICS:
(A) LENGTH: 60 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:117:

Ala Tyr Asn Ile Ile Asp Gly Cys Trp Arg Gly Lys Ala Asp Trp Ala
1               5                   10                  15

Glu Asn Arg Lys Ala Leu Ala Asp Cys Ala Gln Gly Phe Gly Lys Gly
            20                  25                  30

Thr Val Gly Gly Lys Asp Gly Asp Ile Tyr Thr Val Thr Ser Glu Leu
        35                  40                  45

Asp Asp Asp Val Ala Asn Pro Lys Glu Gly Thr Leu
    50                  55                  60

(2) INFORMATION FOR SEQ ID NO:118:

(i) SEQUENCE CHARACTERISTICS:
(A) LENGTH: 46 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:118:

His Asp Val Lys Val Asn Pro Gly Gly Leu Ile Lys Ser Asn Asp Gly
1               5                   10                  15

Pro Ala Ala Pro Arg Ala Gly Ser Asp Gly Asp Ala Ile Ser Ile Ser
            20                  25                  30

Gly Ser Ser Gln Ile Trp Ile Asp His Cys Ser Leu Ser Lys
        35                  40                  45

(2) INFORMATION FOR SEQ ID NO:119:

(i) SEQUENCE CHARACTERISTICS:
(A) LENGTH: 45 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:119:

Arg Cys Arg His Gly Phe Phe Gln Val Val Asn Asn Asn Tyr Asp Lys
1               5                   10                  15

Trp Gly Ser Tyr Ala Ile Gly Gly Ser Ala Ser Pro Thr Ile Leu Ser
            20                  25                  30

Gln Gly Asn Arg Phe Cys Ala Pro Asp Glu Arg Ser Lys
        35                  40                  45

(2) INFORMATION FOR SEQ ID NO:120:

(i) SEQUENCE CHARACTERISTICS:
(A) LENGTH: 47 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:120:

Glu Ala Ala Ala Glu Ser Met Lys Trp Asn Trp Arg Thr Asn Lys Asp
1               5                   10                  15

Val Leu Glu Asn Gly Ala Ile Phe Val Ala Ser Gly Val Asp Pro Val
            20                  25                  30

Leu Thr Pro Glu Gln Ser Ala Gly Met Ile Pro Ala Glu Pro Gly
        35                  40                  45

(2) INFORMATION FOR SEQ ID NO:121:

(i) SEQUENCE CHARACTERISTICS:
(A) LENGTH: 45 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:121:

Arg Gly Lys Ala Asp Trp Ala Glu Asn Arg Lys Ala Leu Ala Asp Cys
1               5                   10                  15

Ala Gln Gly Phe Gly Lys Gly Thr Val Gly Gly Lys Asp Gly Asp Ile
            20                  25                  30

Tyr Thr Val Thr Ser Glu Leu Asp Asp Asp Val Ala Asn
        35                  40                  45

(2) INFORMATION FOR SEQ ID NO:122:

(i) SEQUENCE CHARACTERISTICS:
(A) LENGTH: 35 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:122:

Lys Ser Asn Asp Gly Pro Ala Ala Pro Arg Ala Gly Ser Asp Gly Asp
1               5                   10                  15

Ala Ile Ser Ile Ser Gly Ser Ser Gln Ile Trp Ile Asp His Cys Ser
            20                  25                  30

Leu Ser Lys
        35

(2) INFORMATION FOR SEQ ID NO:123:

(i) SEQUENCE CHARACTERISTICS:
(A) LENGTH: 43 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:123:

Arg His Gly Phe Phe Gln Val Val Asn Asn Asn Tyr Asp Lys Trp Gly
1               5                   10                  15

Ser Tyr Ala Ile Gly Gly Ser Ala Ser Pro Thr Ile Leu Ser Gln Gly
            20                  25                  30

Asn Arg Phe Cys Ala Pro Asp Glu Arg Ser Lys
        35                  40

(2) INFORMATION FOR SEQ ID NO:124:

(i) SEQUENCE CHARACTERISTICS:
(A) LENGTH: 36 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:124:

Arg Thr Asn Lys Asp Val Leu Glu Asn Gly Ala Ile Phe Val Ala Ser
1               5                   10                  15

Gly Val Asp Pro Val Leu Thr Pro Glu Gln Ser Ala Gly Met Ile Pro
            20                  25                  30

Ala Glu Pro Gly
        35

(2) INFORMATION FOR SEQ ID NO:125:

(i) SEQUENCE CHARACTERS:
(A) LENGHT 24 amino acids
(B) TYPE: amino acid
(D) TOPOLOGY: linear

(ii) MOLECULAR TYPE: peptide

(v) FRAGMENT TYPE: internal

(xi) SEQUENCE DESCRIPTION: SEQ ID NO:125:

Ser Asp Gly Asp Ala Ile Ser Ile Ser Gly Ser Ser Gln Ile Trp Ile
1               5                   10                  15

Asp His Cys Ser Leu Ser Lys Ser
            20

(2) INFORMATION FOR SEQ ID NO:126:

(i) SEQUENCE CHARACTERISTICS:
(A) LENGTH: 16 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:126:

Ser Gly Ser Ser Gln Ile Trp Ile Asp His Ser Ser Leu Ser Lys Ser
1               5                   10                  15

(2) INFORMATION FOR SEQ ID NO:127:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:127:

Ser Ile Ser Gly Ser Ser Gln Ile Trp Ile Asp His Ser Ser Leu Ser
1               5                   10                  15

Lys Ser

(2) INFORMATION FOR SEQ ID NO:128:

(i) SEQUENCE CHARACTERISTICS:
(A) LENGTH: 21 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:128:

Asp Ala Ile Ser Ile Ser Gly Ser Ser Gln Ile Trp Ile Asp His Ser
1               5                   10                  15

Ser Leu Ser Lys Ser
            20

(2) INFORMATION FOR SEQ ID NO:129:

(i) SEQUENCE CHARACTERISTICS:
(A) LENGTH: 24 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:129:

Ser Asp Gly Asp Ala Ile Ser Ile Ser Gly Ser Ser Gln Ile Trp Ile
1               5                   10                  15

Asp His Ser Ser Leu Ser Lys Ser
            20

(2) INFORMATION FOR SEQ ID NO:130:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:130:

Ser Glu Ser Gly Ser Ser Gln Ile Trp Ile Asp His Ser Ser Leu Ser
1               5                   10                  15

Lys Ser

(2) INFORMATION FOR SEQ ID NO:131:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:131:

Ser Lys Ser Gly Ser Ser Gln Ile Trp Ile Asp His Ser Ser Leu Ser
1               5                   10                  15

Lys Ser

(2) INFORMATION FOR SEQ ID NO:132:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:132:

Asp Lys Ser Gly Ser Ser Gln Ile Trp Ile Asp His Ser Ser Leu Ser
1               5                   10                  15

Lys Glu

(2) INFORMATION FOR SEQ ID NO:133:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:133:

Asp Lys Ser Ile Ser Gly Ser Ser Gln Ile Trp Ile Asp His Ser Ser
1               5                   10                  15

Leu Ser Lys Glu
            20

(2) INFORMATION FOR SEQ ID NO:134:

(i) SEQUENCE CHARACTERISTICS:
(A) LENGTH: 19 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:134:

Asp Lys Glu Ser Gly Ser Ser Gln Ile Trp Ile Asp His Ser Ser Leu
1               5                   10                  15

Ser Lys Glu

(2) INFORMATION FOR SEQ ID NO:135:

(i) SEQUENCE CHARACTERISTICS:
(A) LENGTH: 18 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:135:

Asp Lys Ser Gly Ser Ser Gln Ile Trp Ile Asp His Ser Ser Leu Ser
1               5                   10                  15

Lys Lys

(2) INFORMATION FOR SEQ ID NO:136:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:136:

Asp Lys Glu Ser Gly Ser Ser Gln Ile Trp Ile Asp His Ser Ser Leu
1               5                   10                  15

Ser Lys Glu Lys
            20

(2) INFORMATION FOR SEQ ID NO:137:

(i) SEQUENCE CHARACTERISTICS:
(A) LENGTH: 16 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:137:

Ser Gly Ser Ser Gln Ile Ala Ile Asp His Ser Ser Leu Ser Lys Ser
1               5                   10                  15

(2) INFORMATION FOR SEQ ID NO:138:

(i) SEQUENCE CHARACTERISTICS:
(A) LENGTH: 20 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:138:

Asp Lys Glu Ser Gly Ser Ser Gln Ile Ala Ile Asp His Ser Ser Leu
1               5                   10                  15

Ser Lys Ser Glu
            20

(2) INFORMATION FOR SEQ ID NO:139:

(i) SEQUENCE CHARACTERISTICS:
(A) LENGTH: 21 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:139:

Asp Lys Glu Ser Gly Ser Ser Gln Ile Trp Ile Asp His Ser Ser Leu
1               5                   10                  15

Ser Lys Glu Lys Asp
            20

(2) INFORMATION FOR SEQ ID NO:140:

(i) SEQUENCE CHARACTERISTICS:
(A) LENGTH: 13 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:140:

Ser Gln Ile Trp Ile Asp His Ser Ser Leu Ser Lys Ser
1               5                   10

(2) INFORMATION FOR SEQ ID NO:141:

(i) SEQUENCE CHARACTERISTICS:
(A) LENGTH: 23 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:141:

Asp Lys Glu Ser Ile Ser Gly Ser Ser Gln Ile Trp Ile Asp His Ser
1               5                   10                  15

Ser Leu Ser Lys Glu Lys Asp
            20

(2) INFORMATION FOR SEQ ID NO:142:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:142:

Lys Asp Val Leu His Asn Gly Ala Ile Phe Val Ala Ser Gly Val Glu
1               5                   10                  15

Pro Val Leu Thr Pro His Gln Ser Ala Gly
            20                  25

(2) INFORMATION FOR SEQ ID NO:143:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:143:

Lys Asp Val Leu His Asn Gly Ala Ile Phe Val Ala Ser Gly Val Asn
1               5                   10                  15

Pro Val Leu Thr Pro His Gln Ser Ala Gly
            20                  25

(2) INFORMATION FOR SEQ ID NO:144:

(i) SEQUENCE CHARACTERISTICS:
(A) LENGHT: 26 amino acids
(B) TYPE: amino acid
(D) TOPOLOGY: linear

(ii) MOLECULAR TYPE: peptide

(v) FRAGMENT TYPE: internal

(xi) SEQUENCE DESCRIPTION SEQ ID NO:144:

Lys Asp Val Leu His Asn Gly Ala Ile Phe Val Ala Ser Gly Val Ala
1               5                   10                  15

Pro Val Leu Thr Pro His Gln Ser Ala Gly
            20                  25

(2) INFORMATION FOR SEQ ID NO:145:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:145:

Lys Asp Val Leu His Asn Gly Ala Ile Phe Val Ala Ser Gly Val Ser
1               5                   10                  15

Pro Val Leu Thr Pro His Gln Ser Ala Gly
            20                  25

(2) INFORMATION FOR SEQ ID NO:146:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:146:

Lys Asp Val Leu His Asn Gly Ala Ile Phe Val Ala Ser Gly Val Gln
1               5                   10                  15

Pro Val Leu Thr Pro His Gln Ser Ala Gly
            20                  25

(2) INFORMATION FOR SEQ ID NO:147:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:147:

Lys Asp Val Leu His Asn Gly Ala Ile Phe Val Ala Ser Gly Val Asp
1               5                   10                  15

Ala Val Leu Thr Pro His Gln Ser Ala Gly
            20                  25

(2) INFORMATION FOR SEQ ID NO:148:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:148:

Lys Asp Val Leu His Asn Gly Ala Ile Phe Val Ala Ser Gly Val Asp
1               5                   10                  15

Ser Val Leu Thr Pro His Gln Ser Ala Gly
            20                  25

(2) INFORMATION FOR SEQ ID NO:149:

(i) SEQUENCE CHARACTERISTICS:
(A) LENGTH: 26 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:149:

Lys Asp Val Leu His Asn Gly Ala Ile Phe Val Ala Ser Gly Val Asp
1               5                   10                  15

Gly Val Leu Thr Pro His Gln Ser Ala Gly
            20                  25

(2) INFORMATION FOR SEQ ID NO:150:

(i) SEQUENCE CHARACTERISTICS:
(A) LENGTH: 35 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:150:

Lys Ser Asn Asp Gly Pro Ala Ala Pro Arg Ala Gly Ser Asp Gly Asp
1               5                   10                  15

Ala Ile Ser Ile Ser Gly Ser Ser Gln Ile Trp Ile Asp His Ser Ser
            20                  25                  30

Leu Ser Lys
        35

## Claims

1. All or a portion of an isolated peptide of Amb a I.1, optionally said portion comprising at least fifteen amino acid residues, said peptide or portion thereof comprising at least one T cell epitope of Amb a I.1, optionally at least two such epitopes, said peptide comprising an amino acid sequence selected from any of the following, or said peptide being a modified peptide thereof:
a) AMB 2-4.1 (SEQ ID NO:90);
b) AMB 2-3.1 (SEQ ID NO:91);
c) AMB 2-5.1 (SEQ ID NO:92);
d) AMB 2-6.1 (SEQ ID NO:93);
e) AMB 2-2.1 (SEQ ID NO:94);
f) AMB 2-1.1 (SEQ ID NO:95);
g) RAE 70.1-1 (SEQ ID NO:43);
h) AMB 2-7.1 (SEQ ID NO:96);
i) AMB 2-8.1 (SEQ ID No:97);
j) AMB 2-9.1 (SEQ ID NO:98);
k) AMB 2-10.1 (SEQ ID NO:99);
l) AMB 2-11.1 (SEQ ID NO:100);
m) RAE 70.1 (SEQ ID NO:44);
n) AMB 3-4.1 (SEQ ID NO:103);
o) AMB 3-5.1 (SEQ ID NO:102);
p) AMB 3-3.1 (SEQ ID NO:104);
q) AMB 3-2.1 (SEQ ID NO:105);
r) AMB 3-1.1 (SEQ ID NO:106);
s) AMB 4-8.1 (SEQ ID NO:109);
t) AMB 4-9.1 (SEQ ID NO:110);
u) AMB 4-6.1 (SEQ ID NO:111);
v) AMB 4-5.1 (SEQ ID NO:112);
w) AMB 4-3.1 (SEQ ID NO:113);
x) AMB 4-2.1 (SEQ ID NO:114);
y) AMB 4-1.1 (SEQ ID NO:115);
z) AMB 2-38.1 (SEQ ID NO:141);
a') RA-02.1 (SEQ ID NO:150); and
b') AMB 2-36.1 (SEQ ID NO:139).

2. All or a portion of an isolated or modified peptide of claim 1 which has a stimulation index of at least 2.0.

3. All or a portion of an isolated or modified peptide of claim 1 which has minimal immunoglobulin E stimulating activity.

4. All or a portion of an isolated or modified peptide of claim 1 which either:-
(a) does not bind immunoglobulin E specific for Amb a I.1 in a substantial percentage of individuals sensitive to Amb a I.1, or if binding of the peptide to said immunoglobulin E occurs, such binding does not result in release of mediators from mast cells or basophils in a substantial percentage of individuals sensitive to Amb a I.1; or
(b) binds immunoglobulin E to a substantially lesser extent than Amb a I.1 binds said immunoglobulin E.

5. All or a portion of an isolated or modified peptide of any one of claims 1 to 4 which modifies, in a ragweed pollen-sensitive individual to whom it is administered, the allergic response of the individual to ragweed pollen.

6. An isolated peptide of Amb a I.1 comprising an amino acid sequence selected from any of the following, or a modified peptide thereof:
a) AMB 2-4.1 (SEQ ID NO:90);
b) AMB 2-3.1 (SEQ ID NO:91);
c) AMB 2-5.1 (SEQ ID NO:92);
d) AMB 2-6.1 (SEQ ID NO:93);
e) AMB 2-2.1 (SEQ ID NO:94);
f) AMB 2-1.1 (SEQ ID NO:95);
g) RAE 70.1-1 (SEQ ID NO:43);
h) AMB 2-7.1 (SEQ ID NO:96);
i) AMB 2-8.1 (SEQ ID NO:97);
j) AMB 2-9.1 (SEQ ID NO:98);
k) AMB 2-10.1 (SEQ ID NO:99);
l) AMB 2-11.1 (SEQ ID NO:100);
m) RAE 70.1 (SEQ ID NO:44);
n) AMB 3-4.1 (SEQ ID NO:103);
o) AMB 3-5.1 (SEQ ID NO:102);
p) AMB 3-3.1 (SEQ ID NO:104);
q) AMB 3-2.1 (SEQ ID NO:105);
r) AMB 3-1.1 (SEQ ID NO:106);
s) AMB 4-8.1 (SEQ ID NO:109);
t) AMB 4-9.1 (SEQ ID NO:110);
u ) AMB 4-6.1 (SEQ ID NO:111);
v ) AMB 4-5.1 (SEQ ID NO:112);
w ) AMB 4-3.1 (SEQ ID NO:113);
x ) AMB 4-2.1 (SEQ ID NO:114);
y ) AMB 4-1.1 (SEQ ID NO:115);
z ) AMB 2-38.1 (SEQ ID NO:141);
a' ) RA-02.1 (SEQ ID NO:150); and
b' ) AMB 2-36.1 (SEQ ID NO:139).
optionally in which either:-
(a) at least one amino acid residue not essential for T cell receptor interaction has been substituted with another amino acid residue, optionally selected from a methyl amino acid, alanine and glutamic acid; or
(b) at least one amino acid residue essential for T cell receptor interaction has been substituted with a similar amino acid residue.

7. An isolated peptide which is immunologically cross-reactive with antibodies specific for all or a portion of a peptide of any one of claims 1 to 5 or which is immunologically cross-reactive with T cells reactive with all or a portion of a peptide of any one of claims 1 to 5.

8. An isolated nucleic acid having a sequence encoding all or a portion of a peptide of any one of claims 1 to 5 or the functional equivalent of said nucleic acid sequence.

9. All or a portion of an isolated peptide of Ambrosia artemisiifolia, said peptide or portion thereof comprising at least one T cell epitope of Ambrosia artemisiifolia, said peptide comprising an amino acid sequence selected from:
a) RAE 67.1 (SEQ ID NO:13);
b) RAE 57.1 (SEQ ID NO:14);
c) RAE 24.1 (SEQ ID NO:15);
d) RAE 24.1 (SEQ ID NO:16);
e ) RAE 61.1 (SEQ ID NO:32);
f ) RAE 80.1 (SEQ ID NO:33);
g ) RAE 45.1 (SEQ ID No:34);
h ) RAE 75.1 (SEQ ID NO:35);
i ) RAE 62.1 (SEQ ID NO:36);
j ) RAE 69.1 (SEQ ID NO:37);
k ) RAE 69.1-1 (SEQ ID NO:38);
l ) RAE 69.1-2 (SEQ ID NO:39);
m) RAE 69.1-3 (SEQ ID NO:40);
n) RAE 70.1-3 (SEQ ID NO:41);
o) RAE 70.1-2 (SEQ ID NO:42);
p) RAE 71.1 (SEQ ID NO:45);
q) RAE 65.1 (SEQ ID NO:46);
r) RAE 63.1 (SEQ ID NO:47);
s) RAE 76.1 (SEQ ID NO:48);
t) RAE 27.1 (SEQ ID NO:49);
u) RAE 66.1 (SEQ ID NO:50);
v) RAE 66.1-1 (SEQ ID NO:51);
w) RAE 66.1-2 (SEQ ID NO:52);
x) RAE 66.1-3 (SEQ ID NO:53);
y) RAE 64.1-3 (SEQ ID NO:54);
z) RAE 64.1-2 (SEQ ID NO:55);
a') RAE 64.1-1 (SEQ ID NO:56);
b') RAE 64.1 (SEQ ID NO:57);
c') RAE 73.1 (SEQ ID NO:58);
d') RAE 74.1 (SEQ ID NO:59);
e') RAE 74.1-1 (SEQ ID NO:60);
f') RAE 29.1 (SEQ ID NO:61);
g') RAE 29.1-1 (SEQ ID NO:62);
h') RAE 28+29 (SEQ ID NO:63);
i') RAE 29.1-2 (SEQ ID NO:64);
j') RAE 29.1-3 (SEQ ID NO:65);
k') RAE 29.1-4 (SEQ ID NO:66);
l') RAE 28.1-3 (SEQ ID NO:67);
m') RAE 28.1-2 (SEQ ID NO:68);
n') RAE 28.1-1 (SEQ ID NO:69);
o') RAE 28.1 (SEQ ID NO:70);
p') RAE 20.1 (SEQ ID NO:71);
q') RAE 20.1-3 (SEQ ID NO:72);
r') RAE 20.1-2 (SEQ ID NO:73);
s') RAE 20.1-1 (SEQ ID NO:74);
t') RAE 21.1 (SEQ ID NO:75);
u') RAE 17.1 (SEQ ID NO:76);
v') RAE 55.1 (SEQ ID NO:77);
w') RAE 76.6 (SEQ ID NO:78);
x') RAE 67.15 (SEQ ID NO:79);
y') RAE 45.15 (SEQ ID NO:80); and
z') RAE 27.15 (SEQ ID NO:81).

10. An isolated peptide comprising at least two regions, each region comprising at least one T cell epitope of a protein allergen of Ambrosia artemisiifolia, said regions derived from the same or from different protein allergens of Ambrosia artemisiifolia, said regions each comprising all or a portion of an amino acid sequence selected from:
a) AMB 1-1.1 (SEQ ID NO:85);
b) AMB 1-2.1 (SEQ ID NO:86);
c) AMB 1-3.1 (SEQ ID NO:87);
d) AMB 1-4.1 (SEQ ID NO:84);
e) AMB 1-5.1 (SEQ ID NO:83);
f) AMB 1-6.1 (SEQ ID NO:82);
g) AMB 2-4.1 (SEQ ID NO:90);
h) AMB 2-3.1 (SEQ ID NO:91);
i) AMB 2-5.1 (SEQ ID NO:92);
j) AMB 2-6.1 (SEQ ID NO:93);
k) AMB 2-2.1 (SEQ ID NO:94);
l) AMB 2-1.1 (SEQ ID NO:95);
m) RAE 70.1-1 (SEQ ID NO:43);
n) AMB 2-7.1 (SEQ ID NO:96);
o) AMB 2-8.1 (SEQ ID NO:97);
p) AMB 2-9.1 (SEQ ID NO:98);
q) AMB 2-10.1 (SEQ ID NO:99);
r) AMB 2-11.1 (SEQ ID NO:100);
s) RAE 70.1 (SEQ ID NO:44);
t) AMB 3-4.1 (SEQ ID NO:103);
u) AMB 3-5.1 (SEQ ID NO:102);
v) AMB 3-3.1 (SEQ ID NO:104);
w) AMB 3-2.1 (SEQ ID NO:105);
x) AMB 3-1.1 (SEQ ID NO:106);
y) AMB 4-8.1 (SEQ ID NO:109);
z) AMB 4-9.1 (SEQ ID NO:110);
a') AMB 4-6.1 (SEQ ID NO:111);
b') AMB 4-5.1 (SEQ ID NO:112);
c') AMB 4-3.1 (SEQ ID NO:113);
d') AMB 4-2.1 (SEQ ID NO:114);
e') AMB 4-1.1 (SEQ ID NO:115);
f') AMB 2-38.1 (SEQ ID NO:141);
g') RA-02.1 (SEQ ID NO:150); and
h') AMB 2-36.1 (SEQ ID NO:139).

11. An isolated peptide of claim 10 wherein said peptide comprises various combinations of regions, said combinations of regions selected from:
a) AMB 4-6.1 and RAE 70.1 (SEQ ID NO:111 and SEQ ID NO:44);
b) AMB 4-6.1 and AMB 2-5.1 (SEQ ID NO:111 and SEQ ID NO:92);
c) AMB 4-9.1 and AMB 2-5.1 (SEQ ID NO:110 and SEQ ID NO:92);
d) AMB 4-9.1 and RAE 70.1 (SEQ. ID NO:110 and SEQ ID NO:44);
e) AMB 4-6.1, AMB 2-5.1 and AMB 1-2.1 (SEQ ID NO:111, SEQ ID NO:92 and SEQ ID NO:86);
f) AMB 4-9.1, AMB 2-5.1 and AMB 1-2.1 (SEQ ID NO:110, SEQ ID NO:92 and SEC ID NO:86);
g) AMB 4-6.1, RAE 70.1 and AMB 1-2.1 (SEQ ID NO:111, SEQ ID NO:44 and SEQ ID NO:86);
h) AMB 4-9.1, RAE 70.1 and AMB 1-2.1 (SEQ ID NO:110, SEQ ID NO:44 and SEQ ID NO:86);
i) AMB 4-6.1, RAE 70.1, AMB 1-2.1 and AMB 3-5.1 (SEQ ID NO:111, SEQ ID NO:44, SEC ID NO:86 and SEQ ID NO:102);
j) AMB 4-9.1, RAE 70.1, AMB 1-2.1 and AMB 3-5.1 (SEQ ID NO:110, SEQ ID NO:44, SEQ ID NO:86 and SEQ ID NO:102);
k) AMB 4-6.1, AMB 2-5.1, AMB 1-2.1 and AMB 3-5.1 (SEQ ID NO:111, SEQ ID NO:92, SEQ ID NO:86 and SEQ ID NO:102);
l) AMB 4-9.1, AMB 2-5.1, AMB 1-2.1 and AMB 3-5.1 (SEQ ID NO:110, SEQ ID NO:92, SEQ ID NO:86 and SEQ ID NO:102);
m) AMB 4-6.1, RAE 70.1, AMB 1-2.1 and AMB 3-4.1 (SEQ ID NO:111, SEQ ID NO:44, SEQ ID NO:86 and SEQ ID NO:103);
n) AMB 4-9.1, RAE 70.1, AMB 1-2.1 and AMB 3-4.1 (SEQ ID NO:110, SEQ ID NO:44, SEQ ID NO:86 and SEQ ID NO:103);
o) AMB 4-6.1, AMB 2-5.1, AMB 1-2.1 and AMB 3-4.1 (SEQ ID NO:111, SEQ ID NO:92, SEQ ID NO:86 and SEQ ID NO:103);
p) AMB 4-9.1, AMB 2-5.1, AMB 1-2.1 and AMB 3-4.1 (SEQ ID NO:110, SEQ ID NO:92, SEQ ID NO:86 and SEQ ID NO:103);
q') AMB 2-1.15 and AMB 4-3.15 (SEQ ID NO:101, and SEQ ID NO:116);
r') AMB 1-2.15, AMB 2-1.15 and AMB 4-3.15 (SEQ ID NO:89, SEQ ID NO:101, and SEQ ID NO:116); and
s') AMB 1-2.15, AMB 2-1.15, AMB 4-3.15 and AMB 3-4.15 (SEQ ID NO:89, SEQ ID NO:101, SEQ ID NO:116 and SEQ ID NO:107).

12. An isolated peptide of claim 10 wherein the regions each comprise an amino acid sequence selected from:
a) AMB 1-1.1 (SEQ ID NO:85);
b) AMB 1-2.1 (SEQ ID NO:86);
c) AMB 1-3.1 (SEQ ID NO:87);
d) AMB 1-4.1 (SEQ ID NO:84);
e) AMB 1-5.1 (SEQ ID NO:83);
f) AMB 1-6.1 (SEQ ID NO:82);
g) AMB 2-4.1 (SEQ ID NO:90);
h) AMB 2-3.1 (SEQ ID NO:91);
i) AMB 2-5.1 (SEQ ID NO:92);
j) AMB 2-6.1 (SEQ ID NO:93);
k) AMB 2-2.1 (SEQ ID NO:94);
L) AMB 2-1.1 (SEQ ID NO:95);
m) RAE 70.1-1 (SEQ ID NO:43);
n) AMB 2-7.1 (SEQ ID NO:96);
o) AMB 2-8.1 (SEQ ID NO:97);
p) AMB 2-9.1 (SEQ ID NO:98);
q) AMB 2-10.1 (SEQ ID NO:99);
r) AMB 2-11.1 (SEQ ID NO:100);
s) RAE 70.1 (SEQ ID NO:44);
t) AMB 3-4.1 (SEQ ID NO:103);
u) AMB 3-5.1 (SEQ ID NO:102);
v) AMB 3-3.1 (SEQ ID NO:104);
w) AMB 3-2.1 (SEQ ID NO:105);
x) AMB 3-1.1 (SEQ ID NO:106);
y) AMB 4-8.1 (SEQ ID NO:109);
z) AMB 4-9.1 (SEQ ID NO:110);
a') AMB 4-6.1 (SEQ ID NO:111);
b') AMB 4-5.1 (SEQ ID NO:112);
c') AMB 4-3.1 (SEQ ID NO:113);
d') AMB 4-2.1 (SEQ ID NO:114); and
e') AMB 4-1.1 (SEQ ID NO:115).

13. An isolated nucleic acid having a sequence encoding all or a portion of a peptide of any one of claims 10 to 12, or the functional equivalent of said nucleic acid sequence.

14. An isolated peptide produced in a host cell transformed with the nucleic acid of claim 13.

15. All or a portion of an isolated peptide of Amb a I.3, said peptide or portion thereof comprising at least one T cell epitope of Amb a I.3, said peptide comprising an amino acid sequence selected from the group consisting of:
a) AMB 1-4.15 (SEQ ID NO:88);
b) AMB 1-2.15 (SEQ ID NO:89);
c) AMB 2-1.15 (SEQ ID NO:101);
d) AMB 3-4.15 (SEQ ID NO:107);
e) AMB 3-1.15 (SEQ ID NO:108); and
f) AMB 4-3.15 (SEQ ID NO:116).

16. A therapeutic composition comprising all or a portion of an isolated peptide of any one of claims 1 to 6 or 9 to 12 or 15 and a pharmaceutically acceptable carrier or diluent.

17. A method of detecting sensitivity to ragweed pollen in an individual, comprising combining a blood sample obtained from the individual with all or a portion of peptide of any one of claims 1 to 6 or 9 to 12 or 15, under conditions appropriate for binding of blood components with the peptide, and determining the extent to which such binding occurs as indicative of sensitivity in the individual to ragweed pollen, optionally the extent to which binding occurs being determined by assessing T cell function, T cell proliferation or a combination thereof.

18. A composition comprising at least two peptides, said peptides each comprising at least one T cell epitope of a protein allergen of ambrosia artemisiifolia, said peptides derived from the same of from different protein allergens of ambrosia artemisiifolia, said composition selected from:
a) AMB 4-6.1 and RAE 70.1 (SEQ ID NO:111 and SEQ ID NO:44);
b) AMB 4-6.1 and AMB 2-5.1 (SEQ ID NO:111 and SEQ ID NO:92);
c) AMB 4-9.1 and AMB 2-5.1 (SEQ ID NO:110 and SEQ ID NO:92);
d) AMB 4-9.1 and RAE 70.1 (SEQ ID NO:110 and SEQ ID NO:44);
e) AMB 4-6.1, AMB 2-5.1 and AMB 1-2.1 (SEQ ID NO:111, SEQ ID NO:92 and SEQ ID NO:86);
f) AMB 4-9.1, AMB 2-5.1 and AMB 1-2.1 (SEQ ID NO:110, SEQ ID NO:92 and SEQ ID NO:86);
g) AMB 4-6.1, RAE 70.1 and AMB 1-2.1 (SEQ ID NO:111, SEQ ID NO:44 and SEQ ID NO:86);
h) AMB 4-9.1, RAE 70.1 and AMB 1-2.1 (SEQ ID NO:110, SEQ ID NO:44 and SEQ ID NO:86);
i) AMB 4-6.1, RAE 70.1, AMB 1-2.1 and AMB 3-5.1 (SEQ ID NO:111, SEQ ID NO:44, SEQ ID NO:86 and SEQ ID NO:102);
j) AMB 4-9.1, RAE 70.1, AMB 1-2.1 and AMB 3-5.1 (SEQ ID NO:110, SEQ ID NO:44, SEQ ID NO:86 and SEQ ID NO:102);
k) AMB 4-6.1, AMB 2-5.1, AMB 1-2.1 and AMB 3-5.1 (SEQ ID NO:111, SEQ ID NO:92, SEQ ID NO:86 and SEQ ID NO:102);
l) AMB 4-9.1, AMB 2-5.1, AMB 1-2.1 and AMB 3-5.1 (SEQ ID NO:110, SEQ ID NO:92, SEQ ID NO:86 and SEQ ID NO:102);
m) AMB 4-6.1, RAE 70.1, AMB 1-2.1 and AMB 3-4.1 (SEQ ID NO:111, SEQ ID NO:44, SEQ ID NO:86 and SEQ ID NO:103);
n) AMB 4-9.1, RAE 70.1, AMB 1-2.1 and AMB 3-4.1 (SEQ ID NO:110, SEQ ID NO:44, SEQ ID NO:86 and SEQ ID NO:103);
o) AMB 4-6.1, AMB 2-5.1, AMB 1-2.1 and AMB 3-4.1 (SEQ ID NO:111, SEQ ID NO:92, SEQ ID NO:86 and SEQ ID NO:103);
p) AMB 4-9.1, AMB 2-5.1, AMB- 1-2.1 and AMB 3-4.1 (SEQ ID NO:110, SEQ ID NO:92, SEQ ID NO:86 and SEQ ID NO:103);
q') AMB 2-1.15 and AMB 4-3.15 (SEQ ID NO:101, and SEQ ID NO:116);
r') AMB 1-2.15, AMB 2-1.15 and AMB 4-3.15 (SEQ ID NO:89, SEQ ID NO:101, and SEQ ID NO:116); and
s') AMB 1-2.15, AMB 2-1.15, AMB 4-3.15 and AMB 3-4.15 (SEQ ID NO:89, SEQ ID NO:101, SEQ ID NO:116 and SEQ ID NO:107).

19. A therapeutic composition comprising a pharmaceutically acceptable carrier or diluent and at least two peptides, said peptides each comprising at least one T cell epitope of a protein allergen of Ambrosia artemisiifolia, said peptides derived from the same or from different protein allergens of Ambrosia artemisiifolia; optionally said peptides being selected from those combinations of peptides referred to in claim 18.

20. An isolated peptide comprising at least two different regions, each region comprising at least one T cell epitope of Amb a I.1, said regions each comprising all or a portion of an amino acid sequence selected from amino acid residues 48-107 (SEQ ID NO 117); amino acid residues 171-216 (SEQ ID NO 118); amino acid residues 278-322 (SEQ ID NO 119); amino acid residues 331-377 (SEQ ID NO 120); amino acid residues 57-101 (SEQ ID NO 121); amino acid residues 182-216 (SEQ ID NO 122); amino acid residues 280-322 (SEQ ID NO 123); and amino acid residues 342-377 (SEQ ID NO 124); optionally wherein the regions comprise amino acid residues 171-216 (SEQ ID NO 118) of Amb a I.1 and amino acid residues 331-377 (SEQ ID NO 120) of Amb a I.1.

21. An isolated peptide of Amb a I.1 comprising at least one T cell epitope of Amb a I.1 said peptide comprising all or a portion of an amino acid sequence selected from the group consisting of: amino acid residues 48-107 (SEQ ID NO 117); amino acid residues 171-216 (SEQ ID NO 118); amino acid residues 278-322 (SEQ ID NO 119); and amino acid residues 331-377 (SEQ ID NO 120).

22. A therapeutic composition comprising at least one peptide of Amb a I.1 and a pharmaceutically acceptable carrier or diluent, said at least one peptide comprising a sufficient percentage of the T cell epitopes of Amb a I.1 such that upon administration of the peptide to an individual sensitive to ragweed pollen, T cells of the individual are tolerized to Amb a I.1.

23. The use of all or a portion of an isolated peptide of any one of claims 1 to 6, 9 to 12, or 15, or of peptides as defined in claims 18 or 19, or of a peptide as defined in claim 20 or 21, or of a peptide as referred to in claim 22, in the manufacture of a medicament for treating or preventing sensitivity to ragweed pollen optionally said medicament providing for administration of at least two peptides sequentially.

24. The use of a protein allergen selected from recombinant Amb a I.1, recombinant Amb a I.2, recombinant Amb a I.3, recombinant Amb a I.4 and recombinant Amb a II, said protein allergen binding immunoglobulin E to a substantially lesser extent that the corresponding native protein allergen, in the manufacture of a medicament for treating sensitivity to ragweed pollen.

25. A composition comprising at least two peptides, said peptides each comprising at least one T cell epitope of Amb a I.1, said composition selected from the group consisting of:
a) AMB 1-2.1 and AMB 4-9.1 (SEQ ID NO:86 and SEQ ID NO:110);
b) AMB 1-2.1, AMB 2-38.1 and AMB 4-9.1 (SEQ ID NO:86, SEQ ID NO:141, and SEQ ID NO:110);
c) AMB 1-2.1, AMB 2-38.1, AMB 4-9.1 and AMB 2-4.1 (SEQ ID NO:86, SEQ ID NO:141, SEQ ID NO:110, and SEQ ID NO:90);
d) AMB 1-2.1, AMB 2-38.1, AMB 4-9.1, AMB 2-4.1, and AMB 3-5.1 (SEQ ID NO:86, SEQ ID NO:141, SEQ ID NO:110, SEQ ID NO:90, and SEQ ID NO:102);
e) AMB 1-2.1, AMB 2-36.1 and AMB 4-9.1 (SEQ ID NO:86, SEQ ID NO:139, and SEQ ID NO:110);
f) AMB 1-2.1, AMB 2-36.1, AMB 4-9.1 and AMB 2-4.1 (SEQ ID NO:86, SEQ ID NO:139, SEQ ID NO:110, and SEQ ID NO:90); and
g) AMB 1-2.1, AMB 2-36.1, AMB 4-9.1, AMB 2-4.1, and AMB 3-5.1 (SEQ ID NO:86, SEQ ID NO:139, SEQ ID NO:110, SEQ ID NO:90, and SEQ ID NO:102).

26. A process for preparing a modified peptide of claim 6, comprising the steps of either:
(i)
a) determining the amino acid residues of said peptide essential to T cell receptor recognition;
b) substituting at least one amino acid residue not essential to T cell receptor recognition with an amino acid residue, selected from the group consisting of a methyl amino acid, alanine and glutamic acid; and optionally
c) substituting at least one amino acid residue essential for T cell interaction with a similar amino acid residue; or
(ii)
a) determining the amino acid residues of said peptide which are essential to T cell receptor recognition;
b) substituting at least one amino acid residue essential for T cell interaction with a similar amino acid residue; and optionally
c) substituting at least one amino acid residue not essential to T cell receptor interaction with an amino acid residue selected from the group consisting of methyl amino acid, alanine and glutamic acid.
